# EUROPEAN PATENT SPECIFICATION

(11) **EP 2 104 535 B1**
(45) Date of publication and mention of the grant of the patent: **29.12.2010**
(21) Application number: 07854747.8
(22) Date of filing: 21.11.2007
(51) Int. Cl.: A61P 11/00, A61K 31/425, A61P 11/06, A61K 31/444, A61P 11/08, C07K 5/06, A61K 31/401, A61P 29/00

(54) **COMPOUNDS AND COMPOSITIONS AS CHANNEL ACTIVATING PROTEASE INHIBITORS**
VERBINDUNGEN UND ZUSAMMENSETZUNGEN ALS KANAL-AKTIVIERENDE PROTEASEHEMMER
COMPOSÉS ET COMPOSITIONS EN TANT QU'INHIBITEURS DE PROTÉASES ACTIVATRICES DE CANAUX

(30) Priority: 10.01.2007 US 884334 P; 23.02.2007 US 891474 P
(43) Date of publication of application: 30.09.2009
(60) Divisional application: 10013675.3
(73) Proprietor: IRM LLC, Hamilton HM LX (BM)
(72) Inventor: TULLY, David C., San Diego, California 92130 (US); CHATTERJEE, Arnab K., San Diego, California 92103 (US); VIDAL, Agnes, San Diego, California 92122 (US); BURSULAYA, Badry, San Diego, California 92122 (US); SPRAGGON, Glen, San Diego, California 92117 (US)
(74) Representative: Kiddle, Simon John
(86) International application number: PCT/US2007/085366
(87) International publication number: WO 2008/085608

(56) References cited:
- WO-A-2006/108643
- US-A1- 2004 186 060

## Description

### Technical Field

The invention generally relates to channel activating protease (CAP) inhibitors.

### Background Art

Prostasin is a trypsin-like serine protease that is present in a variety of mammalian tissues. It is a membrane anchored protease that is expressed on the extracellular membrane of cells but that may also be secreted into body fluids such as semen, urine and airway surface liquid. Prostasin (PRSS8), together with proteases such as matriptase, CAP2, CAP3, trypsin, PRSS22, TMPRSS11, cathepsin A, and neutrophil elastase, may stimulate the activity of the amiloride-sensitive epithelial sodium channel (ENaC). Inhibiting these enzymes may induce changes in epithelial ion transport and therefore fluid homeostasis across epithelial membranes. For example, CAP inhibition in the kidney is thought to promote diuresis, whilst CAP inhibition in the airways promotes the clearance of mucus and sputum in lung. CAP inhibition in the kidney may therefore be used therapeutically to treat hypertension. CAP inhibition in the airways prevents the stagnation of respiratory secretions that otherwise tends to make sufferers vulnerable to secondary bacterial infections. WO2006/108643 and US2004/0186060 disclose exemplary CAP inhibitors.

### Disclosure of the Invention

The invention provides compounds, pharmaceutical compositions and compounds for modulating channel activating proteases (CAP). For example, the compounds and compositions of the invention may be used for modulating prostasin, PRSS22, TMPRSS11 (*e.g*., TMPRSS11B, TMPRSS 11 E), TMPRSS2, TMPRSS3, TMPRSS4 (MTSP-2), matriptase (MTSP-1), CAP2, CAP3, trypsin, cathepsin A, and neutrophil elastase.

In one aspect, the present invention provides compounds of Formula (1): 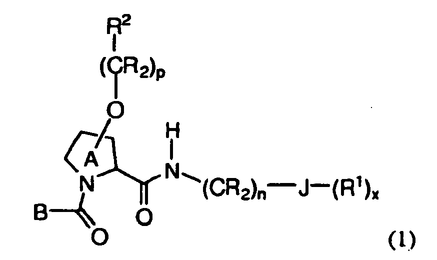 or pharmaceutically acceptable salts thereof, wherein
O-(CR₂)ₚ-R² is a substituent at any position on ring A;
J is a 5-13 membered monocyclic or fused carbocyclic ring, heterocyclic ring comprising N, O and/or S; aryl or heteroaryl ring, provided J is not triazolyl;
B is 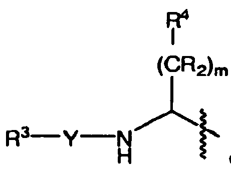 or (CR₂)ₖ-_{R}⁵_{;}
Y is a bond, -SO₂-, -NHCO- or -O-(CO)-;
R¹ is halo, -(CR₂)₁-NR⁶R⁷, -(CR₂)₁-NRC(=NR)-NR⁶R⁷, -(CR₂)1-C(=NR)-NR⁶R⁷, -C(O)-(CR₂)₁⁻NR⁶R⁷, -(CR₂)₁-NR-SO₂R⁶, -(CR₂)₁-NR-C(O)-R⁶, -(CR₂)₁-SO₂NR⁶R⁷, or -(CR₂)₁-OR⁶, or an optionally substituted C₁₋₆alkoxy, C₁₋₆ alkyl, C₂₋₆ alkenyl or C₂₋₆-alkynyl; or an optionally substituted carbocyclic ring, heterocyclic ring, aryl or heteroaryl;
R³ is C₁₋₆ alkyl, C₂₋₆ alkenyl, C₂₋₆ alkynyl or -(CR₂)₁-R⁵;
alternatively, NH-Y-R³ together form NH₂;
R², R⁴ and R⁵ are independently an optionally substituted 5-12 membered carbocyclic ring, heterocyclic ring, aryl or heteroaryl; or R⁴ is H, C₁₋₆ alkyl, C₂₋₆ alkenyl, C₂₋₆
alkynyl, or 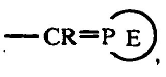 wherein P is C or N and ring E together with P form an optionally substituted 5-12 membered monocyclic or fused ring;
R⁶ and R⁷ are independently H, C₁₋₆alkyl, C₂-₆alkenyl, C₂₋₆alkynyl or -(CR₂)₁-R⁵; each R is H, or C₁₋₆alkyl, C₂₋₆alkenyl, or C₂₋₆alkynyl;
1 is 0-6;
k, m, n and p are independently 1-6;
x is 0-4;
provided R⁴ is piperidinyl when NH-Y-R³ together form NH₂; and further provided that R⁵ is piperidinyl when B is (CR₂)ₖ-R⁵.

In the above Formula (1), J may be thiophenyl, thiazolyl, phenyl, pyridyl, indazolyl, piperidinyl or pyrrolidinyl. In other examples, R² may be phenyl or cyclohexyl, each of which may be optionally substituted with halo, SO₂(C₁₋₆ alkyl), or an optionally substituted C₁₋₆alkyl or C₁₋₆alkoxy, such as an optionally halogenated C₁₋₆ alkyl or C₁₋₆ alkoxy.

In one embodiment, the invention provides compounds of Formula (2): 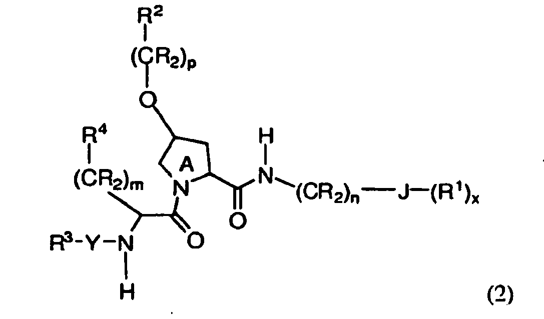 wherein R² and J are independently an optionally substituted 6-membered aryl;
R³ is C₁₋₆alkyl, C₂₋₆alkenyl, C₂₋₆alkynyl or -(CR₂)₁-R⁵; or NH-Y-R³ together form NH₂;
each R in (CR₂) is H or C₁₋₆ alkyl; and
m, n and p are independently 1-2.

In the above Formula (1) and (2); Y may be a bond, SO₂ or -O-(CO)-. In other example, R¹ is halo, C₁₋₆ alkyl, CF₃, OCF₃, phenyl, -(CR₂)ₗ-NR⁶R⁷, -(CR₂)ₗ-C(=NR)-NR⁶R⁷, -C(O)-(CR₂)ₗ-NR⁶R⁷, -(CR₂)ₗ-NR-SO₂R⁶, -(CR₂)ₗ-NR-C(O)-R⁶, -(CR₂)ₗ-SO₂NR⁶R⁷, or -(CR₂)ₗ-OR⁶, wherein each 1 is 0-1; and R, R⁶ and R⁷ are independently H or C₁₋₆alkyl.

In the above Formula (1) and (2), R⁴ may be an optionally substituted 5-6 membered carbocyclic ring, heterocyclic ring, aryl, heteroaryl, or 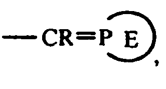 wherein P is C or N and ring E together with P form an optionally substituted 5-6 membered
monocyclic ring. For example, R⁴ may be an optionally substituted piperidinyl, cyclohexyl, phenyl,

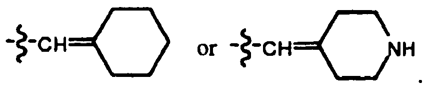

In particular example, R³ in Formula (2) is C₁₋₆alkyl or an optionally substituted benzyl. In some examples, Y is SO₂. In other examples, R⁴ is an optionally substituted piperidinyl. In yet other examples, J and R² are independently optionally substituted phenyl. For example, J may be substituted with 1-3 R¹ (i.e., wherein x is 1-3), and R² may optionally be substituted with halo.

In another aspect, the present invention provides pharmaceutical compositions comprising a compound of Formula (1) or (2), and a pharmaceutically acceptable excipient.

The invention also provides compounds for modulating a channel activating protease, which can be administered to a system or a mammal, a therapeutically effective amount of a compound having Formula (1) or (2), or pharmaceutically acceptable salts or pharmaceutical compositions thereof, thereby modulating said channel activating protease.

In one embodiment, the invention provides compounds for inhibiting a channel activating protease, which can be administered to a cell or tissue system or to a mammal, a therapeutically effective amount of a compound having Formula (1) or (2), or pharmaceutically acceptable salts or pharmaceutical compositions thereof; wherein said channel activating protease is prostasin, PRSS22, TMPRSS11 (*e.g*., TMPRSS 11B, TMPRSS11E), TMPRSS2, TMPRSS3, TMPRSS4 (MTSP-2), matriptase (MTSP-1), CAP2, CAP3, trypsin, cathepsin A, or neutrophil elastase, thereby inhibiting said channel activating protease. In particular examples, the invention provides compounds for inhibiting prostasin.

In another aspect, the invention provides compounds for ameliorating or treating a condition mediated by a channel activating protease, which can be administered to a cell or tissue system or to a mammal, an effective amount of a compound having Formula (1) or (2), or pharmaceutically acceptable salts or pharmaceutical compositions thereof, and optionally in combination with a second therapeutic agent; wherein said channel activating protease is prostasin, PRSS22, TMPRSS11 (*e.g*., TMPRSS11B, TMPRSS11E), TMPRSS2, TMPRSS3, TMPRSS4 (MTSP-2), matriptase (MTSP-1), CAP2, CAP3, trypsin, cathepsin A, or neutrophil elastase, thereby treating said condition.

Furthermore, the present invention provides compounds of Formula (1) or (2), for use in a method for treating a condition mediated by a channel activating protease. The present invention also provides the use of a compound of Formula (1) or (2), and optionally in combination with a second therapeutic agent, in the manufacture of a medicament for treating a condition mediated by a channel activating protease.

In particular examples, the compounds of the invention may be used for treating a prostasin-mediated condition. In one embodiment, the second therapeutic agent may be an anti-inflammatory, bronchodilatory, antihistamine, anti-tussive, antibiotic or DNase, and is administered prior to, simultaneously with, or after the compound of Formula (1) or (2). In some example, the compounds of the invention are administered to bronchial epithelial cells, particularly human bronchial epithelial cells.

Examples of conditions which may be ameliorated or treated using the compounds of the invention include a condition associated with the movement of fluid across ion transporting epithelia or the accumulation of mucus and sputum in respiratory tissues, or a combination thereof. In some examples, the condition which may be mediated using the compounds of the invention is cystic fibrosis, primary ciliary dyskinesia, lung carcinoma, chronic bronchitis, chronic obstructive pulmonary disease, asthma or a respiratory tract infection.

### Definitions

"Alkyl" refers to a moiety and as a structural element of other groups, for example halo-substituted-alkyl and alkoxy, and may be straight-chained or branched. An optionally substituted alkyl, alkenyl or alkynyl as used herein may be optionally halogenated (*e.g*., CF₃), or may have one or more carbons that is substituted or replaced with a heteroatom, such as NR, O or S (e.g., -OCH₂CH₂O-, alkylthiols, thioalkoxy, alkylamines, etc).

"Aryl" refers to a monocyclic or fused bicyclic aromatic ring containing carbon atoms. For example, aryl may be phenyl or naphthyl. "Arylene" means a divalent radical derived from an aryl group.

"Heteroaryl" as used herein is as defined for aryl above, where one or more of the ring members is a heteroatom. Examples of heteroaryls include pyridyl, indolyl, indazolyl, quinoxalinyl, quinolinyl, benzofuranyl, benzopyranyl, benzothiopyranyl, benzo[1,3]dioxole, imidazolyl, benzo-imidazolyl, pyrimidinyl, furanyl, oxazolyl, isoxazolyl, triazolyl, tetrazolyl, pyrazolyl, thienyl, etc.

A "carbocyclic ring" as used herein refers to a saturated or partially unsaturated, monocyclic, fused bicyclic or bridged polycyclic ring containing carbon atoms, which may optionally be substituted, for example, with =O. Examples of carbocyclic rings include cyclopropyl, cyclobutyl, cyclopentyl, cyclohexyl, cyclopropylene, cyclohexanone, etc.

A "heterocyclic ring" as used herein is as defined for a carbocyclic ring above, wherein one or more ring carbons is a heteroatom. For example, a heterocyclic ring may contain N, O, S, -N=, -S-, -S(O), -S(O)₂-, or -NR- wherein R may be hydrogen, C₁₋₄alkyl or a protecting group. Examples of heterocyclic rings include morpholino, pyrrolidinyl, pyrrolidinyl-2-one, piperazinyl, piperidinyl, piperidinylone, 1,4-dioxa-8-aza-spiro[4.5]dec-8-yl, etc.

Unless otherwise indicated, when a substituent is deemed to be "optionally substituted," it is meant that the substituent is a group that may be substituted with one or more group(s) individually and independently selected from, for example, an optionally halogenated alkyl, alkenyl, alkynyl, alkoxy, alkylamine, alkylthio, alkynyl, amide, amino, including mono- and di-substituted amino groups, aryl, aryloxy, arylthio, carbonyl, carbocyclic, cyano, cycloalkyl, halogen, heteroalkyl, heteroalkenyl, heteroalkynyl, heteroaryl, heterocyclic, hydroxy, isocyanato, isothiocyanato, mercapto, nitro, O-carbamyl, N-carbamyl, O-thiocarbamyl, N-thiocarbamyl, C-amido, N-amido, S-sulfonamido, N-sulfonamido, C-carboxy, O-carboxy, perhaloalkyl, perfluoroalkyl, silyl, sulfonyl, thiocarbonyl, thiocyanato, trihalomethanesulfonyl, and the protected compounds thereof. The protecting groups that may form the protected compounds of the above substituents are known to those of skill in the art and may be found in references such as Greene and Wuts, Protective Groups in Organic Synthesis, 3rd Ed., John Wiley & Sons, New York, NY, 1999, and Kocienski, Protective Groups, Thieme Verlag, New York, NY, 1994

The terms "co-administration" or "combined administration" as used herein are meant to encompass administration of the selected therapeutic agents to a single patient, and are intended to include treatment regimens in which the agents are not necessarily administered by the same route of administration or at the same time.

The term "pharmaceutical combination" as used herein refers to a product obtained from mixing or combining active ingredients, and includes both fixed and non-fixed combinations of the active ingredients. The term "fixed combination" means that the active ingredients, e.g. a compound of Formula (1) and a co-agent, are both administered to a patient simultaneously in the form of a single entity or dosage. The term "non-fixed combination" means that the active ingredients, e.g. a compound of Formula (1) and a co-agent, are both administered to a patient as separate entities either simultaneously, concurrently or sequentially with no specific time limits, wherein such administration provides therapeutically effective levels of the active ingredients in the body of the patient. The latter also applies to cocktail therapy, e.g. the administration of three or more active ingredients.

The term "therapeutically effective amount" means the amount of the subject compound that will elicit a biological or medical response in a cell, tissue, organ, system, animal or human that is being sought by the researcher, veterinarian, medical doctor or other clinician.

The term "administration" and or "administering" of the subject compound should be understood to mean as providing a compound of the invention including a prodrug of a compound of the invention to the individual in need of treatment.

As used herein, the terms "treat", "treating" and "treatment" refer to a method of alleviating or abating a disease and/or its attendant symptoms.

The term "prostasin" may also be referred to as: human channel-activating protease (hCAP); channel-activating protease-1; and PRSS8, MERPOPS ID S01.159.

### Modes of Carrying Out the Invention

The invention provides compounds, pharmaceutical compositions and of compounds for modulating channel activating proteases (CAP).

In one aspect, the present invention provides compounds of Formula (1): 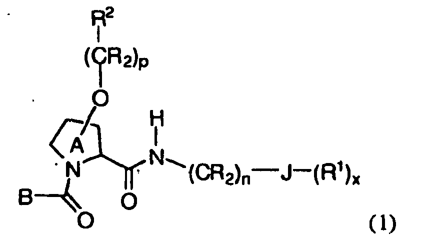 or pharmaceutically acceptable salts thereof, wherein
O-(CR₂)_{P}-R² is a substituent at any position on ring A;
J is a 5-12 membered monocyclic or fused carbocyclic ring, heterocyclic ring comprising N, O and/or S; aryl or heteroaryl ring, provided J is not triazolyl;
B is 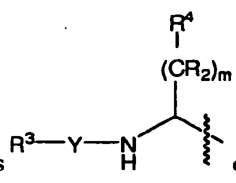 or (CR₂)ₖ-R⁵;
Y is a bond, -SO₂-, -NHCO- or -O-(CO)-;
R¹ is halo, -(CR₂)ₗ-NR⁶R⁷, -(CR₂)ₗ-NRC(=NR)-NR⁶R⁷, -(CR₂)ₗ-C(=NR)-NR⁶R⁷, -C(O)-(CR₂)ₗ-NR⁶R⁷, -(CR₂)ₗ-NR-SO₂R⁶, -(CR₂)ₗ-NR-C(O)-R⁶, -(CR₂)ₗ-SO₂NR⁶R⁷, or -(CR₂)₁-OR⁶, or an optionally substituted C₁₋₆alkoxy, C₁₋₆alkyl, C₂₋₆alkenyl or C₂₋₆alkynyl; or an optionally substituted carbocyclic ring, heterocyclic ring, aryl or heteroaryl;
R³ is C₁₋₆alkyl, C₂₋₆alkenyl, C₂₋₆alkynyl or -(CR₂)₁-R⁵;
alternatively, NH-Y-R³ together form NH₂;
R², R⁴ and R⁵ are independently an optionally substituted 5-12 membered carbocyclic ring, heterocyclic ring, aryl or heteroaryl; or R⁴ is H, C₁₋₆alkyl, C₂₋₆ alkenyl, C₂₋₆
alkynyl, or 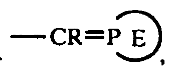 wherein P is C or N and ring E together with P form an optionally substituted 5-12 membered monocyclic or fused ring;
R⁶ and R⁷ are independently H, C₁₋₆alkyl, C₂₋₆alkenyl, C₂-₆alkynyl or -(CR₂)ₗ-R⁵;
each R is H, or C₁₋₆alkyl, C₂₋₆ alkenyl, or C₂₋₆ alkynyl;
1 is 0-6;
k, m, n and p are independently 1-6;
x is 0-4;
provided R⁴ is piperidinyl when NH-Y-R³ together form NH₂; and further provided that R⁵ is piperidinyl when B is (CR₂)ₖ-R⁵.

In other embodiments, the invention provides a compound of Formula (2): 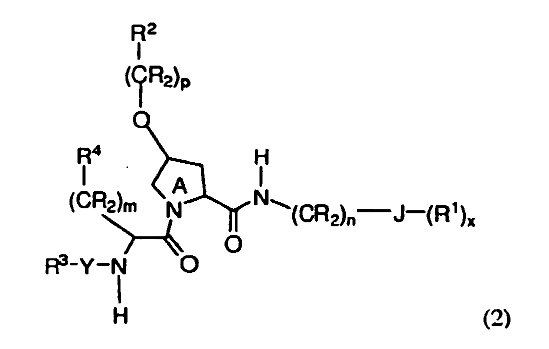 wherein R² and J are independently an optionally substituted 6-membered aryl;
R³ is C₁₋₆alkyl, C₂₋₆alkenyl, C₂₋₆alkynyl or -(CR₂)ₗ-R⁵; or NH-Y-R³ together form NH₂;
each R in (CR₂) is H or C₁₋₆ alkyl; and
m, n and p are independently 1-2.

Alternatively, k, m, n and p in the above Formula (1) and (2) may independently be 0-6. In particular examples, k in Formula (1) is 2-3 and J is a heteroaryl, such as thiophenyl. In other alternative embodiments, Y in Formula (1) and (2) may be -CO-.

In each of the above formula, J may also be selected from the group consisting of 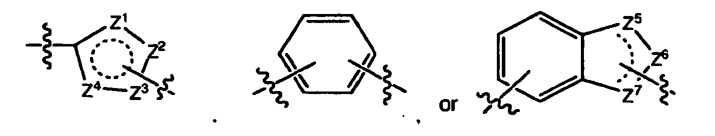 wherein one or more Z¹, Z², Z³, Z⁴, Z⁵, Z⁶ and Z⁷ is a heteroatom selected from N, NR, O or S wherein R is H or C₁₋₆ alkyl, and the other Z¹-Z⁷ atoms are CH.

In some example, at least two of Z¹, Z², Z³, Z⁴, Z⁵, Z⁶ and Z⁷ are a heteroatom selected from N, NR, O or S wherein R is H or C₁₋₆alkyl, and the other Z¹-Z⁷ atoms are CH.

In the above Formula (1) and (2), wherein each optionally substituted moiety may be substituted with halo, =O, amino, guanidinyl, amidino, an optionally substituted C₁₋₆ alkoxy; C₁₋₆ alkyl, C₂₋₆ alkenyl or C₂₋₆ alkynyl, each of which may optionally be halogenated or may optionally have a carbon that may be replaced or substituted with N, O or S; CO₂R⁸, -O-(CR₂)ₗC(O) -R⁸; -(CR₂)ₗ-R⁸, -(CR₂)ₗ-C(O)-R⁸, or -(CR₂)ₗSO₂-R⁸; or a combination thereof, wherein each R⁸ is H, C₁₋₆ alkyl, or an optionally substituted carbocyclic ring, heterocyclic ring, aryl or heteroaryl.

The present invention also includes all suitable isotopic variations of the compounds of the invention, or pharmaceutically acceptable salts thereof. An isotopic variation of a compound of the invention or a pharmaceutically acceptable salt thereof is defined as one in which at least one atom is replaced by an atom having the same atomic number but an atomic mass different from the atomic mass usually found in nature. Examples of isotopes that may be incorporated into the compounds of the invention and pharmaceutically acceptable salts thereof include but are not limited to isotopes of hydrogen, carbon, nitrogen and oxygen such as ²H, ³H, ¹³C, ¹⁴C, ¹⁵N, ¹⁷O, ¹⁸O, ³⁵S, ¹⁸F, and ³⁶Cl_{.} Certain isotopic variations of the compounds of the invention and pharmaceutically acceptable salts thereof, for example, those in which a radioactive isotope such as ³H or ¹⁴C is incorporated, are useful in drug and/or substrate tissue distribution studies. In particular examples , ³H and ¹⁴C isotopes may be used for their ease of preparation and detectability. In other examples, substitution with isotopes such as ²H may afford certain therapeutic advantages resulting from greater metabolic stability, such as increased in vivo half-life or reduced dosage requirements. Isotopic variations of the compounds of the invention or pharmaceutically acceptable salts thereof can generally be prepared by conventional procedures using appropriate isotopic variations of suitable reagents.

The compounds and compositions of the invention may be useful for modulating a channel activating protease. Examples of channel activating proteases which may be modulated using the compounds and compositions of the invention include prostasin, PRSS22, TMPRSS11 (*e.g*., TMPRSS11B, TMPRSS11E), TMPRSS2, TMPRSS3, TMPRSS4 (MTSP-2), matriptase (MTSP-1), CAP2, CAP3, trypsin, cathepsin A, or neutrophil elastase. The compounds of this invention may also inhibit the activity of proteases that stimulate the activity of ion channels, such as the epithelial sodium channel, and may be useful in the treatment of CAP-associated diseases.

### Pharmacology and Utility

Compounds of the invention modulate the activity of channel activating protease, particularly trypsin-like serine proteases such as prostasin, and as such, are useful for treating diseases or disorders in which prostasin, for example, contributes to the pathology and/or symptomology of the disease.

Diseases mediated by inhibition of a channel activating protease, particularly by a trypsin-like serine protease such as prostasin, include diseases associated with the regulation of fluid volumes across epithelial membranes. For example, the volume of airway surface liquid is a key regulator of mucociliary clearance and the maintenance of lung health. The inhibition of a channel activating protease will promote fluid accumulation on the mucosal side of the airway epithelium thereby promoting mucus clearance and preventing the accumulation of mucus and sputum in respiratory tissues (including lung airways). Such diseases include respiratory diseases such as cystic fibrosis, primary ciliary dyskinesia, chronic bronchitis, chronic obstructive pulmonary disease (COPD), asthma, respiratory tract infections (acute and chronic; viral and bacterial) and lung carcinoma. Diseases mediated by inhibition of channel activating proteases also include diseases other than respiratory diseases that are associated with abnormal fluid regulation across an epithelium, perhaps involving abnormal physiology of the protective surface liquids on their surface, for example xerostomia (dry mouth) or keratoconjunctivitis sire (dry eye). Furthermore, CAP regulation of ENaC in the kidney could be used to promote diuresis and thereby induce a hypotensive effect.

Chronic obstructive pulmonary disease includes chronic bronchitis or dyspnoea associated therewith, emphysema, as well as exacerbation of airways hyper reactivity consequent to other drug therapy, in particular other inhaled drug therapy. The invention is also applicable to the treatment of bronchitis of whatever type or genesis including, for example, acute, arachidic, catarrhal, croupus, chronic or phthinoid bronchitis.

Asthma includes intrinsic (non-allergic) asthma and extrinsic (allergic) asthma, mild asthma, moderate asthma, severe asthma, bronchitic asthma, exercise-induced asthma, occupational asthma and asthma induced following bacterial infection. Asthma also encompasses a condition referred to as "wheezy-infant syndrome," which involves subjects less than 4 or 5 years of age who exhibit wheezing symptoms and diagnosed or diagnosable as "wheezy infants," an established patient category of major medical concern and often identified as incipient or early-phase asthmatics.

The suitability of a channel activating protease inhibitor such as a prostasin inhibitor for the treatment of a disease mediated by inhibition of a channel activating protease, may be tested by determining the inhibitory effect of the channel activating protease inhibitor according to the assays described below and following methods known in the art.

In accordance with the foregoing, the present invention further provides compounds for preventing or treating any of the diseases or disorders described above in a subject in need of such treatment, which compounds are administered to said subject in a therapeutically effective amount of a compound of Formula (1) or (2), or a pharmaceutically acceptable salt thereof. For any of the above uses, the required dosage will vary depending on the mode of administration, the particular condition to be treated and the effect desired. (See, "Administration and Pharmaceutical Compositions", infra).

### Administration and Pharmaceutical Compositions

In general, compounds of the invention will be administered in therapeutically effective amounts via any of the usual and acceptable modes known in the art, either singly or in combination with one or more therapeutic agents.

Channel activating protease inhibitors of the invention are also useful as co-therapeutic agents for use in combination with another therapeutic agent. For example, a channel activating protease inhibitor may be used in combination with an anti-inflammatory, bronchodilatory, antihistamine or anti-tussive, antibiotic or DNase therapeutic agent. The channel activating protease inhibitor and other therapeutic agent may be in the same or different pharmaceutical composition. The channel activating protease inhibitor may be mixed with the other therapeutic agent in a fixed pharmaceutical composition, or it may be administered separately, before, simultaneously with or after the other therapeutic agent. The combination may be useful particularly in the treatment of cystic fibrosis or obstructive or inflammatory airways diseases such as those mentioned hereinbefore, for example as potentiators of therapeutic activity of such drugs or as a means of reducing required dosaging or potential side effects of such drugs.

Suitable anti-inflammatory therapeutic agents include steroids, in particular glucocorticosteroids such as budesonide, beclamethasone dipropionate, fluticasone propionate, ciclesonide or mometasone furoate, or steroids described in international patent application WO 02/88167, WO 02/12266, WO 02/100879, WO 02/00679 (for example, Examples 3, 11, 14, 17, 19, 26, 34, 37, 39, 51, 60, 67, 73, 73, 90, 99 and 101), WO 03/35668, WO 03/48181, WO 03/62259, WO 03/64445, WO 03/72592, WO 04/39827 and WO 04/66920; non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935 and WO 04/26248; LTD4 antagonists such as montelukast and zafirlukast; PDE4 inhibitors such cilomilast (ARIFLO® GlaxoSmithKline), ROFLUMILAST® (Byk Gulden), V-11294A (Napp), BAY19-8004 (Bayer), SCH-351591 (Schering-Plough), AROFYLLINE® (Almirall Prodesfarma), PD189659 /PD168787 (Parke-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839, WO 04/005258, WO 04/018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607 and WO 04/037805; and adenosine A_{2B} receptor antagonists such as those described in WO 02/42298 .

Suitable bronchodilatory therapeutic agents include beta-2 adrenoceptor agonists such as albuterol (salbutamol), metaproterenol, terbutaline, salmeterol fenoterol, procaterol, formoterol, carmoterol, or pharmaceutically acceptable salts thereof; and compounds (in free or salt or solvate form) of Formula (1) as described in WO 00/75114, a compound of formula: 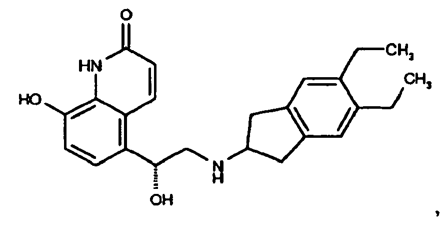 compounds of Formula (1) of WO 04/16601 (in free or salt or solvate form), and compounds of EP 1440966, JP 05025045, WO 93/18007, WO 99/64035, US 2002/0055651, WO 01/42193, WO 01/83462, WO 02/66422, WO 02/70490, WO 02/76933, WO 03/24439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618 WO 04/46083 and WO 04/80964 or pharmaceutically acceptable salts thereof,

Suitable bronchodilatory therapeutic agents also include anticholinergic or antimuscarinic agents, in particular ipratropium bromide, oxitropium bromide, tiotropium salts and CHF 4226 (Chiesi), and glycopyrrolate, but also those described in EP 42402 1, US 3714357, US 5171744, WO 01/04118, WO 02/00652, WO 02/51841, WO 02/53564, WO 03/00840, WO 03/33495, WO 03/53966, WO 03/87094, WO 04/018422 and WO 04/05285.

Suitable dual anti-inflammatory and bronchodilatory therapeutic agents include dual beta-2 adrenoceptor agonist / muscarinic antagonists such as those disclosed in US 2004/0167167, WO 04/74246 and WO 04/74812.

Suitable antihistamine therapeutic agents include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine as well as those disclosed in JP 2004107299, WO 03/099807 and WO 04/026841,

Suitable antibiotics include macrolide antibiotics, for example tobramycin (TOBI™)_{.}

Suitable DNase therapeutic agents include dornase alfa (PULMOZYME™), a highly purified solution of recombinant human deoxyribonuclease I (rhDNase), which selectively cleaves DNA. Dornase alfa is used to treat cystic fibrosis.

Other useful combinations of channel activating protease inhibitors with anti-inflammatory therapeutic agents are those with antagonists of chemokine receptors, e.g. CCR-1, CCR-2, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methyl-phenyl)-5H-benzo-cyclohepten-8-yl]carbonyl]amino]phenyl]-methyl]tetrahydro-N,N-dimethyl-2H-pyran-4-amin-ium chloride (TAK-770), and CCR-5 antagonists described in US 6166037, WO 00/66558, WO 00/66559, WO 04/018425 and WO 04/026873 entirety.

In the treatment of a disease mediated by inhibition of prostasin , a channel activating protease inhibitor of the invention, in free form or in pharmaceutically acceptable salt form, may be administered by any appropriate route, for example orally, e.g. in tablet, capsule or liquid form, parenterally, for example in the form of an injectable solution or suspension, or intranasally, for example in the form of an aerosol or other atomisable formulation using an appropriate intranasal delivery device, e.g. a nasal spray such as those known in the art, or by inhalation, particularly for use with a nebulizer.

The channel activating protease inhibitor may be administered in a pharmaceutical composition together with a pharmaceutically acceptable diluent or carrier. Such compositions may be dry powders, tablets, capsules and liquids, but also injection solutions, infusion solutions or inhalation suspensions, which may be prepared using other formulating ingredients and techniques known in the art.

The dosage of the channel activating protease inhibitor in free form or in pharmaceutically acceptable salt form can depend on various factors, such as the activity and duration of action of the active ingredient, the severity of the condition to be treated, the mode of administration, the species, sex, ethnic origin, age and weight of the subject and/or its individual condition. A typical daily dose for administration, for example oral administration to a warm-blooded animal, particularly a human being weighing about 75 kg, is estimated to be from approximately 0.7 mg to approximately 1400 mg, more particularly from approximately 5 mg to approximately 200 mg. That dose may be administered, for example, in a single dose or in several part doses of, for example, from 5 to 200 mg.

When the composition comprises an aerosol formulation, it may contain, for example, a hydro-fluoro-alkane (HFA) propellant such as HFA134a or HFA227 or a mixture of these, and may contain one or more co-solvents known in the art such as ethanol (up to 20% by weight), and/or one or more surfactants such as oleic acid or sorbitan trioleate, and/or one or more bulking agents such as lactose. When the composition comprises a dry powder formulation, it may contain, for example, the channel activating protease inhibitor having a particle diameter up to 10 microns, optionally together with a diluent or carrier, such as lactose, of the desired particle size distribution and a compound that helps to protect against product performance deterioration due to moisture e.g. magnesium stearate. When the composition comprises a nebulised formulation, it may contain, for example, the channel activating protease inhibitor either dissolved, or suspended, in a vehicle containing water, a co-solvent such as ethanol or propylene glycol and a stabilizer, which may be a surfactant.

In particular embodiments, the invention provides compounds of Formula (1) or (2) in inhalable form, e.g. in an aerosol or other atomisable composition or in inhalable particulate, e.g. micronised, fonn. The invention also provides an inhalable medicament comprising compounds of the invention in inhalable form; a pharmaceutical product comprising compounds of the invention in inhalable form in association with an inhalation device; and an inhalation device comprising compounds of the invention in inhalable form.

### Processes for Making Compounds of the Invention

The compounds of the invention may be prepared, following procedures exemplified in the Examples.

In the reactions described, reactive functional groups, where desired in the final product (e.g., hydroxy, amino, imino, thio or carboxy groups), may be protected using protecting groups known in the art, to avoid their unwanted participation in the reactions. Conventional protecting groups may be used in accordance with standard practice, for example, see T.W. Greene and P. G. M. Wuts in "Protective Groups in Organic Chemistry", John Wiley and Sons, 1991.

Compounds of the invention may also be prepared as a pharmaceutically acceptable acid addition salt by reacting the free base form of the compound with a pharmaceutically acceptable inorganic or organic acid. Alternatively, a pharmaceutically acceptable base addition salt of a compound of the invention may be prepared by reacting the free acid form of the compound with a pharmaceutically acceptable inorganic or organic base. Alternatively, salt forms of the compounds of the invention may be prepared using salts of the starting materials or intermediates.

The free acid or free base forms of the compounds of the invention may be prepared from the corresponding base addition salt or acid addition salt from, respectively. For example, a compound of the invention in an acid addition salt form may be converted to the corresponding free base by treating with a suitable base (e.g., ammonium hydroxide solution, sodium hydroxide ). A compound of the invention in a base addition salt form may be converted to the corresponding free acid by treating with a suitable acid (e.g., hydrochloric acid).

Compounds of the invention in unoxidized form may be prepared from N-oxides of compounds of the invention by treating with a reducing agent (e.g., sulfur, sulfur dioxide, triphenyl phosphine, lithium borohydride, sodium borohydride, phosphorus trichloride, tribromide, or the like) in a suitable inert organic solvent (e.g. acetonitrile, ethanol, aqueous dioxane, or the like) at 0 to 80°C.

Prodrug derivatives of the compounds of the invention may be prepared by methods known to those of ordinary skill in the art (e.g., for further details see Saulnier et al., (1994), Bioorganic and Medicinal Chemistry Letters, Vol. 4, p. 1985). For example, appropriate prodrugs may be prepared by reacting a non-derivatized compound of the invention with a suitable carbamylating agent (e.g., 1,1-acyloxyalkylcarbanochloridate, paranitrophenyl carbonate, or the like).

Protected derivatives of the compounds of the invention may be made by means known to those of ordinary skill in the art. A detailed description of techniques applicable to the creation of protecting groups and their removal may be found in T. W. Greene, "Protecting Groups in Organic Chemistry", 3rd edition, John Wiley and Sons, Inc., 1999.

Compounds of the present invention may be conveniently prepared, or formed during the process of the invention, as solvates (e.g., hydrates). Hydrates of compounds of the present invention may be conveniently prepared by recrystallization from an aqueous/organic solvent mixture, using organic solvents such as dioxin, tetrahydrofuran or methanol.

Compounds of the invention may be prepared as their individual stereoisomers by reacting a racemic mixture of the compound with an optically active resolving agent to form a pair of diastereoisomeric compounds, separating the diastereomers and recovering the optically pure enantiomers. While resolution of enantiomers may be carried out using covalent diastereomeric derivatives of the compounds of the invention, dissociable complexes are preferred (e.g., crystalline diastereomeric salts). Diastereomers have distinct physical properties (e.g., melting points, boiling points, solubilities, reactivity, etc.) and may be readily separated by taking advantage of these dissimilarities. The diastereomers may be separated by chromatography, or by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixture may be found in Jean Jacques, Andre Collet, Samuel H. Wilen, "Enantiomers, Racemates and Resolutions", John Wiley And Sons, Inc., 1981.

In summary, the compounds of the invention may be prepared as exemplified in the Examples, and Formula (1) and (2) may be made by a process, which involves:
(a) optionally converting a compound of the invention into a pharmaceutically acceptable salt;
(b) optionally converting a salt form of a compound of the invention to a non-salt form;
(c) optionally converting an unoxidized form of a compound of the invention into a pharmaceutically acceptable N-oxide;
(d) optionally converting an N-oxide form of a compound of the invention to its unoxidized form;
(e) optionally resolving an individual isomer of a compound of the invention from a mixture of isomers;
(f) optionally converting a non-derivatized compound of the invention into a pharmaceutically acceptable prodrug derivative; and
(g) optionally converting a prodrug derivative of a compound of the invention to its non-derivatized form.

Insofar as the production of the starting materials is not particularly described, the compounds are known or may be prepared analogously to methods known in the art or as disclosed in the Examples hereinafter. One of skill in the art will appreciate that the above transformations are only representative of methods for preparation of the compounds of the present invention, and that other well-known methods may similarly be used. The present invention is further exemplified, by the following intermediates (Reference compounds) and Examples that illustrate the preparation of the compounds of the invention.

### Reference compound 1

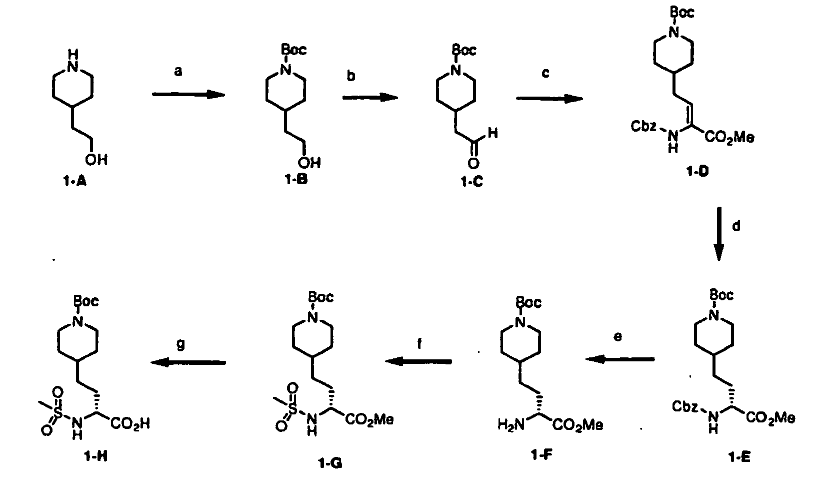

**1-B:** 4-piperidine ethanol (**1-A**) (5 g, 39.7 mmol) is dissolved in THF (120 mL). Triethylamine (5.6 mL, 40 mmol) is added and the solution is cooled to 0 °C. Boc₂O (9.59 g, 44 mmol) is added, and the reaction is stirred overnight at room temperature. Solvent is removed *in vacuo,* and the crude residue is dissolved in ethyl acetate (120 mL). The solution is washed with 0.1 N HCl (3x100 mL) and brine (1x100 mL), dried with MgSO₄, filtered and solvent evaporated *in vacuo* to give **1-B** as a clear oil.
**1-C:** Trichloroisocyanuric acid (2.66 g, 11.46 mmol) is added to a solution of the alcohol 1-B (2.39 g, 10.42 mmol) in DCM, and the solution is stirred and maintained at 0 °C, followed by addition of a catalytic amount of TEMPO. After the addition, the mixture is warmed to room temperature and stirred for an hour and then filtered on Celite®. The organic phase is washed with saturated aqueous Na₂CO₃, followed by 1N HCl and brine. The organic layer is dried (MgSO₄) and the solvent is evaporated to give **1-C.** ¹H NMR (CDCl₃, 400 MHz) δ 89.72 (1H, s), 4.07-4.01 (2H, m), 2.70-2.57 (2H, m), 3.35-3.31 (2H, m), 2.05-1.94 (1H, m), 1.64-1.46 (2H, m), 1.39 (9H, s), 1.30-1.02 (2H, m).
**1-D:** To a solution of Cbz-α-phosphonoglycine trimethyl ester (2.8 g, 8.45 mmol) in THF at -78 °C is added 1,1,3,3-tetramethyl-guanidine (1.022 ml, 8.14 mmol). After 10 minutes, the aldehyde **1-C** (1.76 g, 7.76 mmol) is added. The solution is then placed in an ice bath at 0 °C for 1 hour, and then allowed to warm to room temperature and stirred for one more hour. The solution is diluted with EtOAc, washed with 1M NaHSO₄, dried (MgSO₃) and concentrated *in vacuo.* The residue is purified by silica gel flash chromatography with Ethyl acetate/Hexane 0 to 100% to afford **1-D** as a white solid. MS *m*/*z* 333.2 (M + 1), ¹H NMR (CDCl3, 400 MHz) δ. 7.35-7.33 (5H, m), 6.63 (1H, t, J = 8 Hz), 6.30 (1H, bs), 5.12 (2H, s), 4.10-4.04 (2H, m), 3.73 (3H, s), 2.67-2.62 (2H, m), 2.14 (2H, t, J = 6.8 Hz), 1.63-1.46 (3H, m), 1.43 (9H, s), 1.14-1.06 (2H, m).
**1-E:** A Parr vessel is charged with **1-D** (1 g, 2.31 mmol) and MeOH (100 mL) under nitrogen. The solution is subjected to 3 cycles of vacuum and nitrogen bubbling, and the catalyst (R,R)-Ethyl-DuPHOS-Rh(COD) triflate is added (30 mg, 0.04 mmol). The mixture is placed under 60 psi of hydrogen gas at room temperature for 24 h. The conversion to 1-E is complete after 24 h with >99% e.e., the solvent is removed in vacuo, and the crude product is purified by silica gel chromatography (hexanes/EtOAc).
**1-F:** Intermediate 1-E is dissolved in MeOH. The solution is flushed with nitrogen, and Pd/Carbon (5% wt, Degussa) is added. The mixture is placed under 50 psi of hydrogen gas at room temperature and shaken for 24h. The mixture is flushed with nitrogen and filtered through Celite®. The cake is washed with MeOH, and the combined organic solution is concentrated under vacuum. Hexanes is added and then evaporated to azeotrope the remaining methanol to afford 1-F as an oil, which is then used in the next step without further purification. MS *m*/*z* 201.4 (M + 1 - Boc), ¹H NMR (CDCl₃, 400 MHz) δ. 4.06-3.97 (2H, m), 3.63 (3H, s), 3.36-3.31 (1H, m), 2.63-2.50 (2H, m), 1.70-1.61 (1H, m), 1.61-1.43 (3H, m), 1.36 (3H, s), 1.55 (6H, s), 1.34-1.15 (3H, m), 1.02-1.97 (2H, m).
**1-G:** Crude 1-F (0.6 g, 1.99 mmol) is dissolved in THF (10 mL), and 2,4,6-collidine (315 mg, 2.38 mmol) and methanesulfonyl chloride (0.170 ml, 2.19 mmol) are added to the solution and stirred for 2 hours. The reaction is diluted with EtOAc (50 mL), and solution is washed with 1M NaHSO₄ (2 × 25 mL), brine (25 mL), and dried (MgSO₄). The solvent is removed *in vacuo* and the crude residue is purified by flash chromatography using a gradient of hexanes and EtOAc to afford **1-G.** MS *m*/*z* 279.4 (M + 1 - Boc), ¹H NMR (CDCl₃, 400 MHz) δ. 5.60-5.42 (1H, m), 3.99-3.96 (3H, m), 3.68 (3H, s), 2.86 (3H, m), 2.60-2.54 (2H, m), 1.79-1.77 (1H, m), 1.60-1.45 (2H, m), 1.35 (9H, s), 1.35-1.26 (3H, m), 1.16-0.95 (2H, m).
**1-H:** Compound **1-G** (0.70 g, 1.84 mmol) is dissolved in dioxane (7 mL), and LiOH · H₂O (232 mg, 5.55 mmol) dissolved in water (4 mL) is added. The reaction mixture is stirred for 1 h. The solvent is evaporated; and the residue is diluted with EtOAc (25 mL) and washed with 1N NaHSO₄ (25 mL) and brine (25 mL), and dried (MgSO₄). The solvent is removed in vacuo and the crude residue is purified by silica gel chromatography (Hexanes/EtOAc gradient) to afford Reference Compound I as a white solid. MS *m*/*z* 265.4 (M + 1 - Boc), ¹H NMR (CDCl3, 400 MHz) δ. 8.97 (1H, broad s), 5.44 (1H, d, *J* = 8.8 Hz), 4.15-3.90 (3H, m), 2.94 (3H, s), 2.77-2.55 (2H, m), 1.88-1.87 (1H, m), 1.78-1.58 (3H), 1.42-1.37 (12H, m), 1.16-0.94 (2H, m).

### Reference compound 2

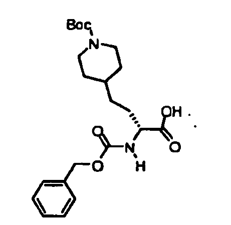

Intermediate **1-E** is saponified with LiOH·H₂O following the same procedure used to obtain the compound **1-H.** MS *m*/*z* 421.5 (M + 1), ¹H NMR (CDCl₃, 400 MHz) δ. 9.75 (1H, broad s), 7.26-7.41 (5H, m), 5.39 (1H, s), 5.10 (2H, s), 4.41-4.34 (1H, m), 4.46-4.03 (2H, m), 2.68-2.61 (2H, m), 1.94 -1.82 (1H, m), 1.78-1.53 (3H, m), 1.44 (9H, s), 1.44-1.19 (3H, m), 1.09-1.03 (2H, m).

### Reference compound 3

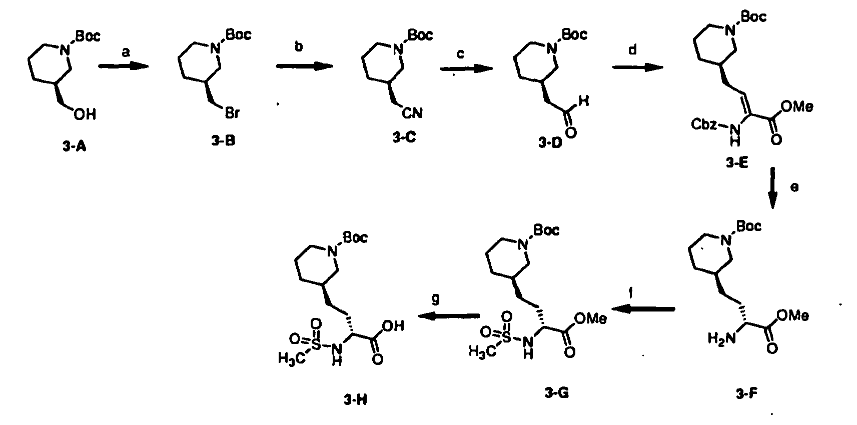

**3-B:** The compound **3-A** (2 g, 9.28 mmol) is combined with CBr₄ (4.46 g, 13.47 mmol) and triphenylphosphine (3.28 g, 12.54 mmol) in THF (0.2 M), and the solution is stirred overnight. The reaction mixture is then filtered and the solvent evaporated. A large portion of the triphenylphosphine oxide is precipitated by slow addition of the crude mixture to a large volume of ether. After filtration and concentration, the residue is purified by chromatography (EtOAc:Hexanes gradient) to afford compound 3-B. ¹H-NMR (CDCl₃, 400 MHz) δ. 4.05-3.99 (1H, m), 3.83-3.78 (1H, m), 3.27-3.34 (2H, m), 2.84-2.77 (1H, m), 2.66-2.59 (1H, m), 1.91-1.74 (2H, m), 1.67-1.56 (1H, m), 1.42 (9H, s), 1.32-1.20 (2H, m).
**3-C:** A mixture of 3-B (1 g, 3.6 mmol) and KCN (281 mg, 4.3 mmol) in anhydrous DMF (20 mL) is stirred under reflux overnight. The residue is dissolved in EtOAc (50 ml), washed successively with 1N NaHSO₄ (2×50 mL) and brine (2×50 mL), and dried over MgSO₄. The solvent is evaporated and the crude material purified by chromatography (EtOAc:Hexanes gradient) to afford compound 3-C as an oil. ¹H-NMR (CDCl₃, 400 MHz) δ. 3.83-3.78 (1H, m), 3.78-3.69 (1H, m), 2.87-2.73 (2H, m), 2.28-2.15 (2H, m), 1.84-1.72 (2H, m), 1.61-1.52 (1H, m), 1.42-1.15 (11H, m).
**3-D:** To a solution of 3-C (750 mg, 3.34 mmol) in THF (20 mL) is added DIBAL (solution 1M in THF, 5 ml) at -78°C. This mixture is allowed to reach room temperature, and stirred at room temperature for 1h. The mixture is cooled to 0°C and water (0.2 mL), and 15% NaOH aq. (0.2 mL) and water (0.5 mL) are successively added. After addition of MgSO₄, the mixture is vigorously stirred and filtered. The evaporation of solvents yields the compound 3-D as an colorless oil. This compound is used in the following step without further purification. ¹H-NMR (CDCl₃, 400 MHz) δ. 3.78-3.67 (1H, m), 3.67-3.64 (1H, m), 2.81-2.71 (1H, m), 2.71-2.50 (1H, m), 2.24-2.09 (2H, m), 1.79-1.66 (2H, m), 1.56-1.48 (1H, m), 1.39-1.13 (11H, m).
**3-E:** This compound is prepared from 3-D using methods analogous to those described for the preparation of Reference Compound 1-D.
**3-F:** This compound is prepared from 3-E using methods analogous to those described for the preparation of Reference Compound 1-F.
**3-G:** This compound is prepared from 3-F using methods analogous to those described for the preparation of Reference Compound 1-G.
**3-H:** This compound is prepared from 3-G using methods analogous to those described for the preparation of Reference Compound 1-H.

### Reference compound 4

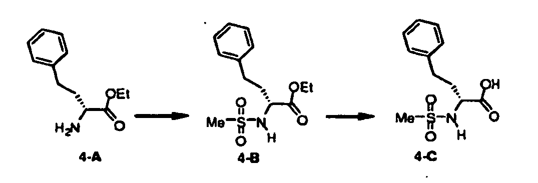

**4-B:** D-Homophenylalanine ethyl ester hydrochloride (5.00 g, 20.5 mmol) and DIEA (8.7 mL, 51.25 mmol) are dissolved in THF (100 mL) and stirred at room temperature. Mesyl chloride (1.67 mL, 21.52 mmol) is added dropwise, and the reaction stirred for 6h at room temp. The THF is evaporated; and the crude residue is dissolved in EtOAc (100 mL), washed with water (100 mL), 1N HCl (2 × 100 mL) and brine (100 mL), an dried (MgSO₄). The solvent is removed *in vacuo* and the crude material is purified with flash chromatography (Hexanes:EtOAc) to afford the ethyl ester.
**4-C:** Ethyl ester **4-B** is dissolved in dioxane (50 mL) and stirred at room temperature. LiOH·H₂O (1.00 mg, 24 mmol) dissolved in water (20 mL) is added and the reaction stirred until the ethyl ester has disappeared (by TLC and LCMS). The solvent is removed *in vacuo* and the crude material is partitioned with EtOAc (50 mL) and 1N HCl (50 mL). The aqueous layer is extracted with EtOAc (2 × 50 mL), and the combined organic phases are washed with 1M NaHSO₄ (2 × 50 mL) and brine (50 mL), and dried with MgSO₄.

The solvent is evaporated and the crude material is purified by flash chromatography (EtOAc:Hexanes gradient) to afford Reference Compound 4 as a white powder.

### Reference compound 5

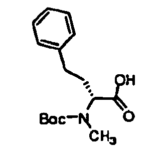

Boc-D-homophenylalanine (1.0 g, 3.58 mmol) is dissolved in THF (10 mL), and water (18 µL, 0.72 mmol) is added to a suspension of NaH (60% dispersion in mineral oil; 10.0 mmol) in tetrahydrofuran (12 mL) dropwise over a period of 20 min while maintaining an internal temperature of 20 °C. The mixture is stirred at the same temperature for 10 min, and dimethyl sulfate (1.05 mL, 6.44 mmol) is added over a period of 20 min while maintaining a temperature of 20 °C. The reaction is stirred for 2h before being quenched with 30% ammonium hydroxide (6 mL) over a period of 10 min. while maintaining an internal temperature of 30 °C. Stirring is continued for an additional 1 h (to ensure complete destruction of dimethyl sulfate). The mixture is diluted with EtOAc (20 mL) and water (20 mL). The organic layer is separated, washed with water (10 mL), dried (MgSO₄), and evaporated in vacuo to give Reference Compound 5 as a white solid.

### Reference compound 6

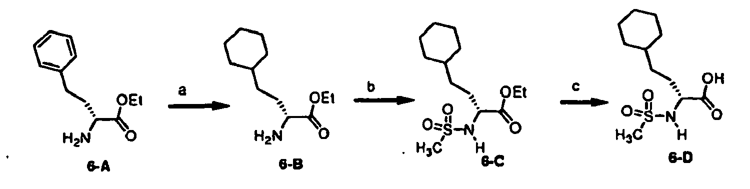

**6-B:** D-Homophenylalanine ethyl ester hydrochloride (6-A) (25.0 g, 102.5 mmol) is dissolved in 10% aqueous EtOH (500 mL). A catalytic amount of 5% Rh/C is added, and the reaction placed under an atmosphere of H₂ (1000 psi), stirred, and heated to 50 °C. After 18h, the reaction is cooled to room temperature, the H₂ gas supply is removed, and the vessel brought to atmospheric pressure. The catalyst is filtered through Celite®, and the solvent removed in vacuo, to afford D-Homocyclohexylalanine ethyl ester hydrochloride as a white powder.
**6-C:** This compound is prepared from **6-B** using methods analogous to those described for the preparation of Reference Compound **4-B.**
**6-D:** This compound is prepared from **6-C** using methods analogous to those described for the preparation of Reference Compound **4-C.**

### Reference compound 7

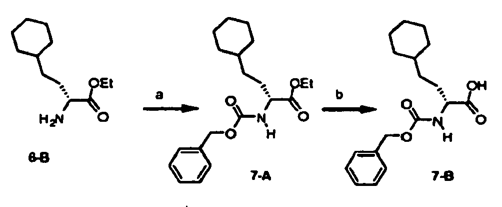

**7-A:** D-Homocyclohexylalanine ethyl ester hydrochloride (3.83 g, 18.0 mmol) and N-(Benzyloxycarbonyloxy)succinimide (Cbz-OSu) (4.49 g, 18.0 mmol) are added to a round bottomed flask containing THF (60 mL) and water (20 mL). The mixture is stirred at room temperature and Et₃N (10.1 mL, 72.0 mmol) is added, and the reaction is stirred overnight at room temperature. The clear solution is diluted with EtOAc (200 mL) and washed with 1N HCl (3 x 100 mL) and brine (1 x 100 mL) and dried with MgSO₄. Solvent is evaporated in vacuo to afford the desired product as a white solid which is used without further purification.
**7-B:** This compound is prepared from **7-A** using methods analogous to those described for the preparation of Reference Compound **4-C.**

### Reference compound 8

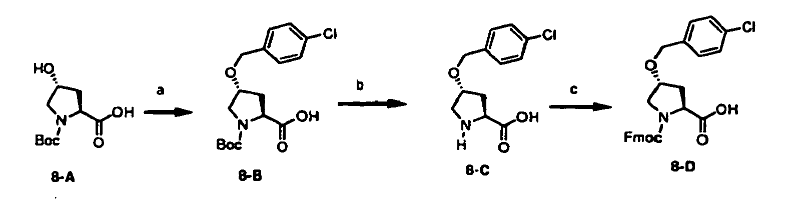

**8-B:** Finely powdered KOH (19.4 g, 0.346 mol) is dissolved in DMSO and stirred at room temperature for 20 min and then cooled to 0°C. N-Boc-*trans*-4-hydroxy-L-proline (Boc-Hyp-OH, **8-A)** (10 g, 43.3 mmol) is dissolved in DMSO (10 mL) and added, and the reaction mixture is stirred for an additional 10 min at 0 °C. Next, 4-chlorobenzyl chloride (33 g, 0.204 mol) is added, and the reaction mixture is stirred at 0° C for an additional 15 min. Thereafter, the ice bath is removed and the reaction mixture is allowed to warm to room temperature and stirred for 4 h. The reaction mixture is poured into water (300 mL) and the reaction vessel is rinsed with an additional aliquot of water (300 mL). The combined aqueous layer is extracted with ether (2 × 300 mL) and discarded. The aqueous layer is acidified with 87% H₃PO₄ to pH 2.3 and then extracted with ether (3 × 300 mL). The combined ether extracts are washed with water (2 × 400 mL) and brine (2 × 400 mL) and then dried over MgSO₄, filtered and concentrated in vacuo. The residue is purified by chromatography on silica gel with EtOAc/Hexanes (gradient 0 to 100%) to yield the compound **8-B** as a clear oil. MS m/z 256.1 (M + 1 - Boc); ¹H NMR (DMSO-D₆, 400 MHz) δ 7.39-7.31 (4H, m), 4.52-4.40 (2H, m), 4.16-4.10 (2H, m), 3.48-3.41 (2H, m), 2.40-2.30 (1H, m), 2.03-1.94 (1H, m), 1.39-1.34 (9H, m).
**8-C:** A solution of TFA in dichloromethane (50/50) is added to **8-B** and the mixture is stirred until complete removal of the Boc. The reaction mixture is then concentrated in *vacuo,* and the crude residue is used in the next step without further purification. MS m/z 256.1 (M + 1); ¹H NMR (CDCl₃, 400 MHz) δ. 8.32 (1H, broad s), 7.16-6.93 (4H, m), 4.41-4.12 (4H, m), 4.10-3.75 (2H, m), 3.70-3.53 (1H, m), 3.51-3.30 (1H, m), 2.38-2.24 (1H, m), 2.06-1.88 (1 H, m).
**8-D:** Intermediate **8-C** is dissolved in 200 ml of a solution of 1,4-dioxane/H₂O (1:1). NaHCO3 (17.9 g, 0.213 mol) is added, followed by Fmoc-Cl (12 g, 46.3 mmol). The mixture is stirred overnight. The solution is then acidified with 1N HCl, and the precipitate is filtered and dried (MgSO₄) to afford **8-D** as a white solid. ¹H-NMR (CDCl₃, 400 MHz) δ. 8.11 (1H, broad s), 7.77-7.66 (2H, m), 7.58-7.52 (2H, m), 7.42-7.21 (8H, m), 4.54-4.26 (4H, m), 4.24 (1H, t, *J* = 7.2 Hz), 4.23-4.10 (1H, m), 3.69-3.61 (2H, m), 3.50-2.38 (1H, m), 2.24-2.12 (1H, m).

### Reference compound 9

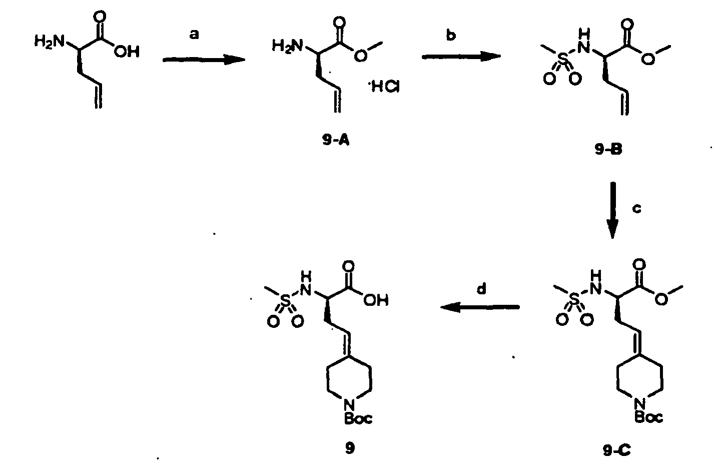

The reagents and conditions for the above reaction scheme are: (a) SOCl₂ (3.0 equiv.), MeOH, 0 °C, 100%; (b) Mesyl chloride (1.2 equiv.), Et₃N (3.0 equiv.), cat. DMAP, THF, 23 °C, 79%; (c) Hoveyda-Grubbs metathesis catalyst (8 mol%), *N-*Boc-4-methylenepiperidine (3.0 equiv.), DCM, 40 °C, 51%; (d) LiOH, dioxanes, H₂O, 23 °C, 100%.
**9-A:** D-allylglycine (5.03 g, 43.73 mmol, 1.0 equiv) is slurried in a suspension of methanol (70 mL) in an ice-water bath. Thionyl chloride (9.6 mL, 131.19 mmol, 3.0 equiv.) is added dropwise over 10 minutes. The reaction is warmed to room temperature and judged to completeness by LC/MS. The solvent is evaporated and the resulting white solid of **9-A** is directly used in the next step.
**9-B:** D-allylglycine methyl ester hydrochloride (**9-A**, 7.20 g, 43.73 mmol), Et₃N (18 mL, 131.19 mmol, 3.0 equiv.) and DMAP (10 mg, catalytic) are dissolved in THF (110 mL) and stirred at room temperature. Mesyl chloride (4.0 mL, 52.48 mmol, 1.2 equiv.) is added dropwise, and the reaction is stirred for 6h at room temp. The THF is evaporated; and the crude residue is dissolved in EtOAc (100 mL) and washed with water (100 mL), 1N HCl (2 × 100 mL) and brine (100 mL), and dried (MgSO₄). The solvent is removed *in vacuo,* and the crude material is purified with flash chromatography (Hexanes:EtOAc) to afford 7 **9-B** as a yellow oil.
**9-C:** Anhydrous dichloromethane (10 mL, 0.1 M) is added via syringe to **9-B** (2.15 g, 10.37 mmol, 1.0 equiv.) and Hoveyda-Grubbs 2nd Generation metathesis catalyst [(1,3-Bis-(2,4,6-trimethylphenyl)-2-imidazolidinylidene) dichloro (*o*-isopropoxyphenylmethylene) ruthenium II dichloride) (510 mg, 0.815 mmol, 8 mol %)] under a nitrogen atmosphere. *N-*Boc-4-methylenepiperidine (6 mL, 31.11 mmol, 3.0 eq.) is added via syringe and the reaction is fitted with a reflux condenser and heated to 40 °C for 12 hours. After the reaction is complete by LC/MS, the reaction mixture is directly purified by automated silica-gel purification (0-100% ethyl acetate in hexanes) to provide **9-C** as a dark green oil. MS *m*/*z* 277.2 (M-Boc + 1).

**Reference Compound 9**: The saponification of **9-C** is accomplished using the procedure previously described for the preparation of Reference Compound 4.

### Reference compound 10

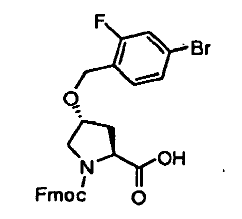

Reference compound 10 is prepared using methods analogous to those described for the preparation of Reference Compound 8.

### Example 1

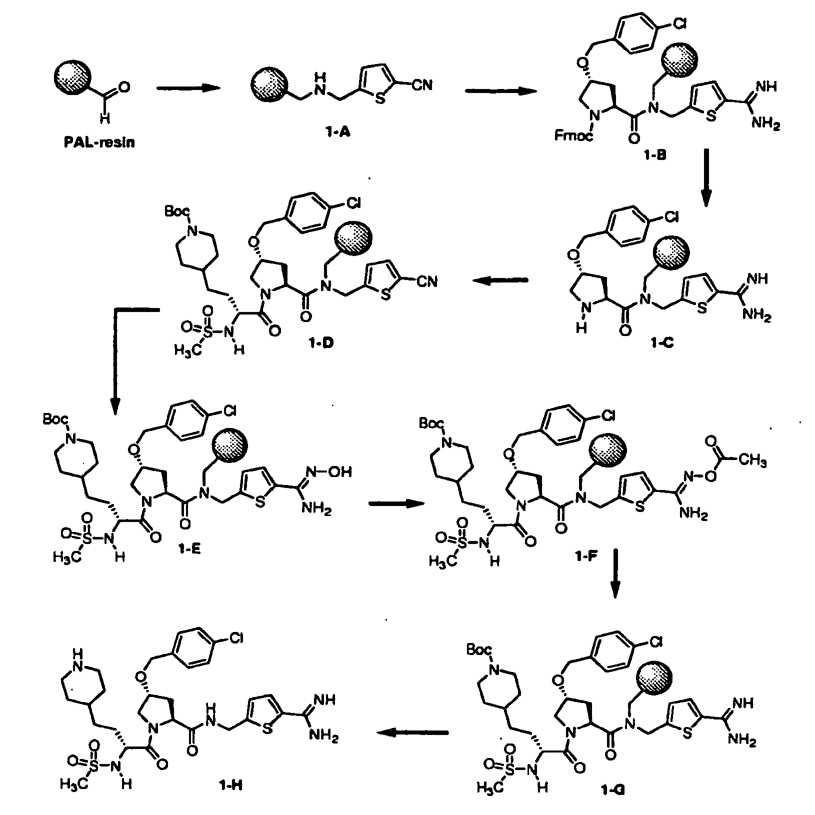

**1-A:** Loading of the PAL resin: 5-cyano-2-methylamino-thiophene (3 equiv.) is added to a solution of the resin (1 meq/g) in DMF in the presence of AcOH (8 equiv.). The mixture is shaken for 1hr before adding NaH(AcO)₃ (3 equiv.) and letting the mixture react overnight. The resin is then washed with DMF (×2), DCM (×2), MeOH (×2) and DCM (×2).
**1-B:** The Fmoc-protected amino acid **8-D** (3 equiv.) is added to 200 mg of resin **1-A** in DMF in the presence of HOBt (3.5 equiv.) and DIC (3.5 equiv.). The mixture is shaken for 3hrs. The resin is washed with DMF (×2), DCM (x2), MeOH (×2) and DCM (×2).
**1-C:** The resin is shaken in a solution of 20% piperidine in DMF for 30 min, and washed with DMF (×2) and DCM (×2).
**1-D :** The amino acid is coupled to the resin 1-C using the same procedure as **1-B.**
**1-E :** A solution of hydroxylamine hydrochloride (40 equiv.) and DIEA (40 equiv.) in DMF is added to the resin **1-D,** and the mixture is shaken overnight. The resin is washed with DMF (×2), DCM (×2), MeOH (×2) and DCM (×2).
**1-F :** To a solution of the resin **1-E** in DCM is added acetic anhydride (10 equiv.). The mixture is shaken for 2 hrs, and then washed with DCM (×2), DMF (x2) and DCM (×2).
**1-G :** The resin **1-F** is washed with anhydrous THF (×2) before a solution of SmI₂ (0.1M in THF) is added under nitrogen. The mixture is shaken for 2hrs, and the resin is washed with DMF (×2), MeOH (×2) DMF (×2) and DCM (×2).
**1-H :** The final compound **1-H** is obtained following cleavage from the resin in presence of a solution of TFA/DCM/H₂O (45:45:10). The filtrate is concentrated *in vacuo,* dissolved in acetonitrile and purified by reverse phase HPLC (H₂O-ACN gradient). After lyophilization, the TFA salt of **1-H** is obtained as a white powder. MS m/z 639.5 (M + 1); ¹H-NMR (CD₃CN, 400 MHz) δ 9.30 (1H, s), 7.89 (1H, s), 7.72 (1H, d, *J* = 4 Hz), 7.36-7.26 (4H, m), 7.09 (1H, d, *J* = 4 Hz), 6.06 (1H, d, *J* = 8 Hz), 4.60-4.41 (5H, m), 4.33-4.21 (1H, m), 4.11-4.05 (1H, m), 3.82-3.65 (2H, m), 3.29-3.27 (2H, m), 2.86 (3H, s), 2.86-2.76 (2H, m), 2.46-2.36 (1H, m), 2.15-2.07 (1H, m), 1.75-1.68 (2H, m), 1.63-1.46 (2H, m), 1.46-1.31 (2H, m), 1.31-1.37 (3H, m).

### Examples 2-31

Examples 2-31 are synthesized using methods analogous to those described for the synthesis of Example 1.

### Example 32

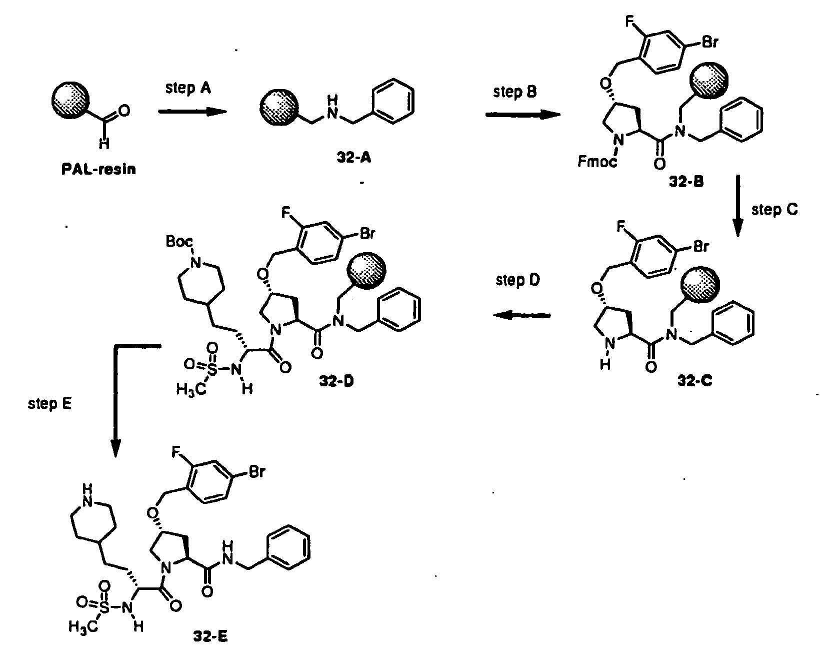

Reagent **32-A** is prepared from benzylamine and Pal-resin using methods analogous to those described for the preparation of Example **1-A.** Intermediate **32-B** is prepared from immobilized **32-A** using methods analogous to those described for the preparation of Example **1-B.** The intermediates **32-C** and **32-D** are prepared from support-bound **32-B** and **32-C** respectively, following methods analogous to those described for Example **1-C** and **1-D,** respectively. Final compound 32-E is prepared by cleaving 32-D from the resin following methods analogous to those for the preparation of Example **1-H.**

### Examples 33-54

Examples 33-54 are synthesized using methods analogous to those described for the synthesis of Example 32.

### Example 55

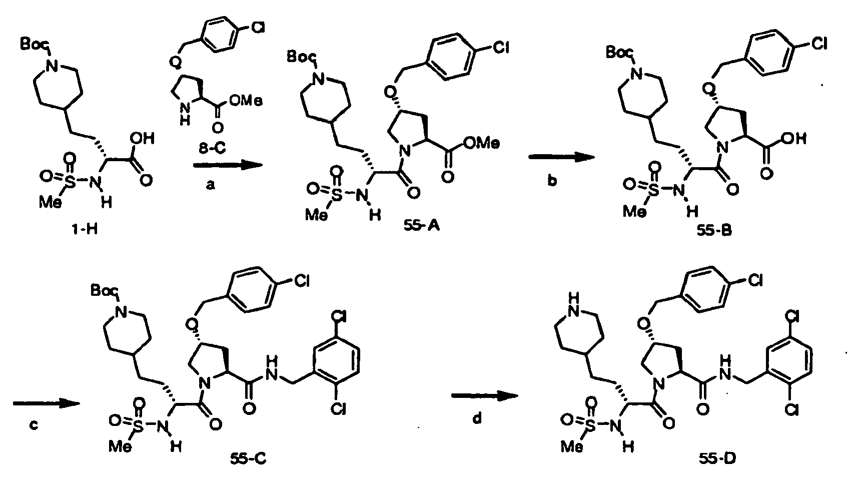

**55-A:** Compound **1-H** (1.9 g, 5.2 mmol) is added to a solution of the HCl salt of the methyl ester **8-C** (1.6 g, 5.2 mmol), PyBOP (3.79 g, 7.28 mmol) and DIEA (2.7 mL, 15.6 mmol) in DCM (50 mL). The mixture is stirred overnight, then washed with a solution of 1M NaHSO₄ (2 × 50 mL), NaHCO₃ saturated (2 × 50 mL) and brine (1 × 50 mL). The organic phase is dried on MgSO₄ and concentrated *in vacuo.* The residue is purified by flash chromatography (Hexanes:EtOAc), and the compound **55-A** is obtained as a white solid. MS m/z 616.2 (M+1).
**55-B:** Methyl ester **55-A** (2.2 g, 3.72 mmol) is dissolved in dioxane (20 mL) and stirred at room temperature. LiOH·H₂O (467 mg, 11.12 mmol) dissolved in water (50 mL) is added and the reaction is stirred until the ethyl ester has disappeared (by TLC and LCMS). The solution is acidified by addition of 1M NaHSO₄ and extracted with EtOAc (2 × 50 mL). The combined organic phases are washed with brine (50 mL), and dried with MgSO₄. The solvent is evaporated to afford the compound **55.B** as a white powder. MS m/z 602.2 (M + 1); ¹H NMR (CDCl₃, 400 MHz) δ 7.33 (2H, d, *J* = 8.4 Hz ), 7.22 (2H, d, *J* = 8.4 Hz), 5.87 (1H, d, *J* = 9.6 Hz ), 4.43-4.57 (4H, m), 4.39-4.32 (1H, m), 3.95-4.17 (4H, m), 3.87-3.93 (1H, m), 3.60-3.64 (1H, m), 2.89 (3H, s), 2.58-2.64 (2H, m), 2.45-3.51 (1H, m), 2.15-2.51 (1H, m), 1.48-1.70 (3H, m), 1.44 (9H, s), 1.22-1.35 (2H, m), 0.95-1.10 (2H, m).
**55-C:** To a solution of compound **55-B** (60 mg, 0.1 mmol) in dichloromethane (10 mL) is added HATU (55 mg, 0.14 mmol), DIEA(0.035 ml, 0.2 mmol), and 2,5-dichlorobenzylamine (23 mg, 0.13 mmol). The mixture is stirred overnight at room temperature, then washed successively with 1M NaHSO₄ (10 ml), NaHCO₃ saturated (10 ml) and brine (10 ml). The solution is dried on MgSO₄, filtered, evaporated and directly used in the next step. MS m/z 659.2 (M + 1 - Boc).
**55-D :** To the solution of **55-C** in DCM is slowly added a solution of 50% TFA in DCM. The mixture is stirred for 30 minutes, then the solvents are evaporated and the residue is dissolved in acetonitrile and purified by reverse phase HPLC. After lyophilization of the solvents, the TFA salt of the compound **55-D** is obtained as a white powder. MS m/z 659.2 (M + 1); ¹H NMR (CDCl₃, 400 MHz) δ 9.30 (1H, bs), 8.56 (1H, bs), 7.31 (1H, d, *J* = 2 Hz), 7.07-7.27 (6H, m), 5.88 (1H, d, *J* = 8.4 Hz ), 4.26-4.57 (6H, m), 3.93-4.02 (1H, m), 3.77-3.86 (1H, m), 3.47-3.86 (1H, m), 3.21-3.34 (5H, m), 2.74 (3H, s), 2.49-2.88 (4H, m), 2.17-2.36 (2H, m), 1.18-1.73 (9H, m).

### Examples 55-70

Examples 56-70 are synthesized using methods analogous to those described for the synthesis of Example 55.

Table 1 shows compounds of Formula (I), as described in Examples 1-70.

**Table 1**

| | **Structure** | **Physical Data** MS (*m*/*z*), Elemental Analysis, and ¹H NMR 400 MHz (DMSO-d₆) |
|---|---|---|
| 1 | 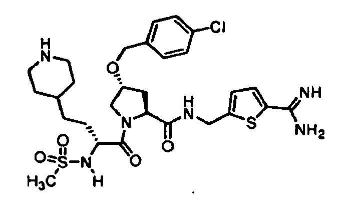 | MS m/z 639.5 (M + 1); ¹H NMR (CD₃CN, 400 MHz) δ 9.30(1H, s), 7.89 (1H, s), 7.72 (1H, d, *J* = 4 Hz), 7.36-7.26 (4 H, m), 7.09 (1H, d, *J* = 4 Hz), 6.06 (1H, d, *J* = 8 Hz), 4.60-0.41 (5H, m), 4.33-4.21 (1H, m), 4.11-4.05 (1H, m), 3.82-3.65 (2H, m), 3.29-3.27 (2H, m), 2.86 (3H, s), 2.86-2.76 (2H, m), 2.46-2.36 (1H, m), 2.15-2.07 (1H, m), 1.75-1.68 (2H, m), 1.63-1.46 (2H, m), 1.46-1.31 (2H, m), 1.31-1.27 (3H, m). |
| 2 | 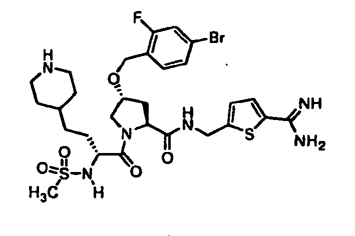 | MS m/z 701.2 (M + 1); Anal. Calcd. for C₃₄H₄₁BrF₁₀N₆O₁₁S₂ (3 TFA): C, 39.13; H, 3.96; N, 8.21; Found: C, 39.24; H, 4.25; N, 8.21;¹H NMR (CD₃CN, 400 MHz) δ9.99 (1H, s), 7.95-7.74 (2H, m), 7.74 (1H, d, *J* = 4 Hz), 7.42-7.32 (3H, m), 7.11 (1H, d, *J* = 4 Hz), 6.13 (1H,d, *J*= 8.4 Hz), 4.59-4.35 (5H, m), 4.15-4.08 (1H, m), 4.15-4.02 (1N, m), 3.84-3.68 (2H, m), 3.31-3.28 (2H, m), 2.87-2.79 (5H, m), 2.45-2.38 (1H, m), 2.18-2.10 (1H, m), 1.76-1.69 (2H, m), 1.69-1.49 (2H, m), 1.49-1.39 (2H, m), 1.39-1.29 (3H, m). |
| 3 | 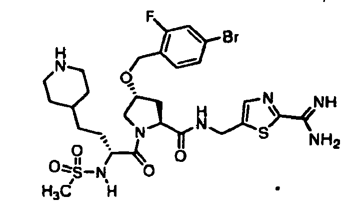 | MS m/z 352 [(M + 1)/2] |
| 4 | 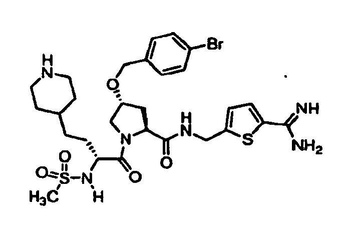 | MS *m*/*z* 683.1 (M + 1)); Anal. Calcd. for C₃₄H₄₁BrF₉N₆O₁₁S₂ (3 TFA): C, 39.81; H, 4.13; N, 8.19; Found: C, 40.48; H, 4.34; N, 8.46; ¹H NMR (CD₃CN, 400 MHz) δ9.81 (1H, s), 7.92-7.77 (2H, m), 7.71 (1H, d, *J*= 4 Hz), 7.49 (2H, d, *J*= 8.4 Hz), 7.23 (2H, d, J = 8 Hz), 7.08 (1H, d, *J* = 4 Hz), 6.16-6.02 (1H, m), 4.55-4.43 (5H, m), 4.28 (1H, s), 4.12-4.05 (1H, m), 3.80-3.69 (2H, m), 3.27-3.24 (2H, m), 2.84-2.70 (5H, m), 2.42-2.33 (1H, m), 2.11-2.03 (1H, m), 1.76-1.62 (2H, m), 1.62-1.46 (2H, m), 1.46-1.39 (2H, m), 1.38-1.18 (3H, m). |
| 5 | 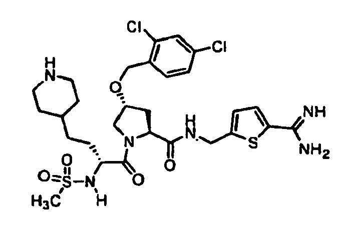 | MS *m*/*z* 673.2 (M + 1); ¹H NMR (CD₃CN, 400 MHz) δ 10.07 (1H, s), 7.72 (1H, d, *J*= 4 Hz), 7.63-7.55 (2H, m), 7.52 (1H, d, *J* = 2 Hz), 7.46 (2H, d, *J* = 8 Hz), 7.37 (1H, dd, *J* = 8 Hz, 2 Hz), 7.14 (1H, d, *J* = 4Hz), 5.88 (1H, d, *J* = 9.2 Hz), 4.68-4.50 (4H, m), 4.46 (1 H, t, *J* = 7.6 Hz), 4.38 (1H, s), 4.18-4.05 (1H, m), 3.86-3.73 (2H, m), 3.31-3.28 (2H, m), 2.85-2.76 (5H, m), 2.50-2.43 (1H, m), 2.22-2.12 (1H, m), 1.81-1.66 (2H, m), 1.65-1.48 (2H, m), 1.48-1.39 (2H, m), 1.39-1.23 (3H, m). |
| 6 | 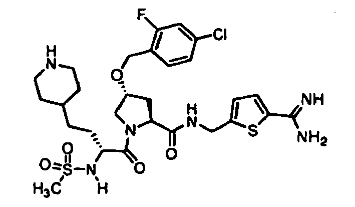 | MS *m*/*z* 657.2 (M + 1) |
| 7 | 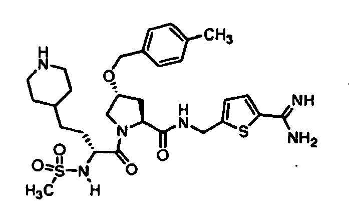 | MS *m*/*z* 619.3 (M + 1); ¹H NMR (CD₃CN, 400 MHz) δ 10.01 (1H, s), 7.73 (1H, d, *J* = 4 Hz), 7.69-7.55 (2H, m), 7.17-7.24 (4H, m), 7.12 (1H, d, *J* = 4 Hz), 5.97 (1H, d, *J* = 9.2 Hz), 4.56-4.40 (5H, m), 4.30 (1H, s), 4.14-4.09 (1H, m), 3.82-3:70 (2H, m), 3.30-3.24 (2H, m), 2.85-2.77 (5H, m), 2.43-2.34 (1H, m), 2.34 (3H, s), 2.17-2.08 (1H, m), 1.79-1.71 (2H, m), 1.68-1.50 (2H, m), 1.50-1.39 (2H, m), 1.35-1.26 (3H, m). |
| 8 | 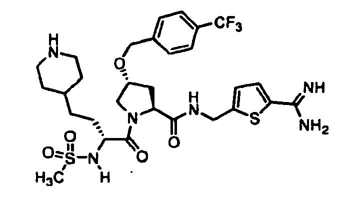 | MS *m*/*z* 672.7 (M + 1) |
| 9 | 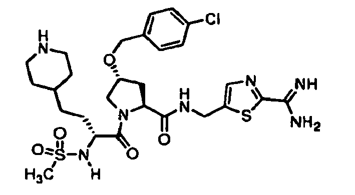 | MS *m*/*z* 640.2 (M + 1) |
| 10 | 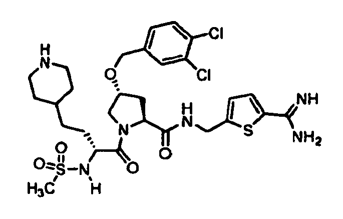 | MS *m*/*z* 673.1 (M + 1); Anal. Calcd. for C₃₄H₄₃Cl₂F₉N₆O₁₂S2 (3 TFA, 1 H₂O): C, 39.50; H, 4.19; N, 8.13; Found: C, 39.54; H, 4.30; N, 7.89; ¹H NMR (CD₃CN, 400 MHz) δ 10.02 (1H, s), 7.70 (1H, d, *J* = 4 Hz), 7.51 (2H, d, *J* = 8 Hz), 7.23 (1 H, dd, *J* = 8 Hz, 2 Hz), 7.12 (1H, d, *J* = 4 Hz), 5.86-5.78 (1 H, m), 4.59-4.39 (5H, m), 4.34-4.29 (1 H, m), 4.13-4.02 (1 H, m), 3.84-3.65 (2H, m), 3.34-3.23 (2H, m), 2.88-2.76 (5 H, m), 2.45-2.36 (1H, m), 2.7-2.07 (1H, m), 1.81-1.67 (2H, m), 1.67-1.46 (2H, m), 1.46-1.36 (2H, m), 1.36-1.24 (3H, m). |
| 11 | 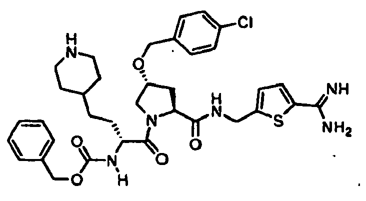 | MS *m*/*z* 695.3 (M + 1);¹H NMR (CD₃CN, 400 MHz) δ9.82 (1H, s), 7.69 (1H, d, *J* = 4 Hz), 7.53-7.45 (2H, m), 7.43-7.49 (9H, m), 7.10 (1H, d, *J* = 4 Hz), 6.22-6.14 (1H, m); 5.06-4.87 (2H, m), 4.60-4.44 (5H, m), 4.32-4.18 (2H, m), 3.88-3.72 (2H, m), 3.35-3.24 (2H, m), 2.92-2.77 (2H, m), 2.45-2.34 (1 H, m), 2.24-2.11 (1H, m), 1.86-1.51 (4H, m), 1-50-1.21 (5H, m). |
| 12 | 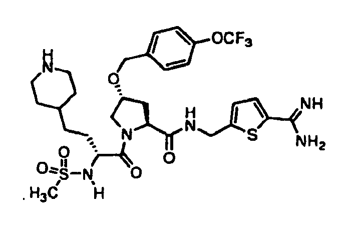 | MS *m*/*z* 689.2 (M + 1); ); ¹H NMR (CD₃CN, 400 MHz) δ 10.05 (1H, s), 7.86-7.81 (3H, m), 7.43 (1H, d, *J* = 4 Hz), 7.43 (2H, d, *J* = 8 Hz), 7.29 (2H, d, *J* = 8 Hz), 7.11 (1H, d, *J* = 4 Hz), 6.13 (1 H, d, *J* = 9.2 Hz), 4.61-4.43 (5H, m), 4.33 (1 H, s), 4.19-4.1 (1H, m), 3.86-3.70 (2H, m), 3.29-3.27)2H, m), 2.85 (3H, s), 2.89-2.80 (2H, m), 2.45-2.36 (1H, m), 2.19-2.09 (1H, m), 1.81-1.64 (2H, m), 1.64-1.50 (2H, m), 1.50-1.43 (2H, m), 1.43-1.30 (3H). |
| 13 | 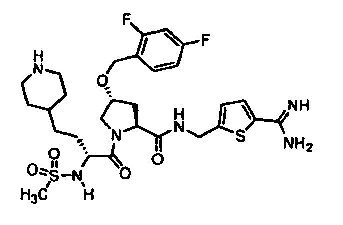 | MS *m*/*z* 641.2 (M + 1); ¹H NMR (CD₃CN, 400 MHz) δ 9.79 (1 H, s), 7.80 ( 1 H, s), 7.73 (1 H, d, *J* = 4 Hz), 7.67-7.52 (2H, m), 7.51-7.38 (1H, m), 7.12 (1H, d, *J* = 4 Hz), 7.02-6.93 (2H, m), 6.03 (1H, d, *J* = 9.2 Hz), 4.62-4.48 (4H, m), 4.43 (1H, t, *J* = 7.6 Hz), 4.34 (1H, s), 4.15-4.09 (1H, m), 3.86-3.82 (2H, m), 3.32-3.29 (2H, m), 2.86-2.80 (5H, m), 2.44-2.34 (1H, m), 2.19-2.10 (1H, m), 1.83-1.70 )2H, m), 1.70-1.50 (2H, m), 1.50-1.34 (2H, m), 1.34-1.25 (3H, m). |
| 14 | 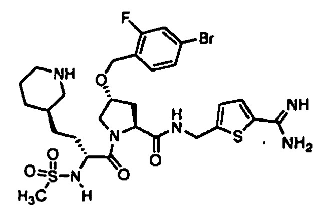 | MS *m*/*z* 703.1 (M + 1) |
| 15 | 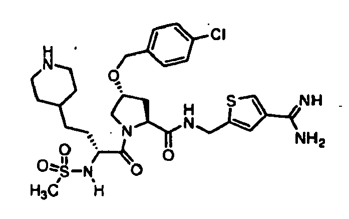 | MS *m*/*z* 639.2 (M + 1); ¹H NMR (CD₃CN, 400 MHz) δ 9.48 (1H, s), 8.15 (1H, s), 7.84 (1H, s), 7.62-7.59 (1 H, m), 7.46-7.25 (6H, m), 6.05 (1H, d, *J* = 8.8 Hz), 4.74-4.09 (5 H, m), 4.32 (1H, m), 4.14-4.09 (1H, m), 3.87-3.70 (2H, m), 3.32-3.24 (2H, m), 2.91-2.78 (5H, m), 2.49-2.36 (1 H, m), 2.16-2.08 (1H, m), 1.83-1.68 (2H, m), 1.67-1.51 (2H, m), 1.49-1.41 (2H, m), 1.38-1.29 (3H, m). |
| 16 | 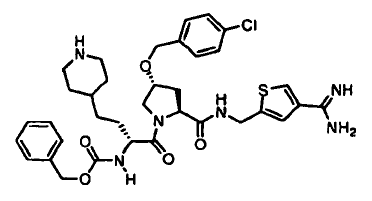 | MS *m*/*z* 695.3 (M + 1); ¹H NMR (CD₃CN, 400 MHz) δ 9.47 (1 H, s), 8.12 (1H, s), 7.83 (1H, s), 7.70-7.67 (1H, m),7.47 (1H, s), 7.40-7.25 (9H, m), 6.40 (1H, d, *J* = 6.8 Hz), 5.03-4.76 (2H, m), 4.60-0.39 (5H, m), 4.32-4.18 (2H, m), 3.35-3.24 (2H, m), 2.91-2.75 (2H, m), 2.48-2.38 (1H, m), 2.17-2.05 (1H, m), 1.81-1.52 (4H, m), 1.52-1.97 (5H, m). |
| 17 | 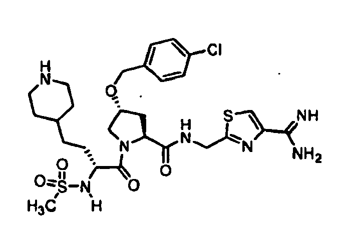 | MS *m*/*z* 640.2 (M + 1); Anal. Calcd. for C₃₃H₄₁ClF₉N₇O₁₁S₂ (3 TFA): C, 40.35; H, 4.21; N, 9.98; Found: C, 40.39; H, 4.10; N, 10.00; ¹H NMR (CD₃CN, 400 MHz) δ 9.80 (1H, s), 8.43 (1H, s), 8.17-7.80 (2H, m), 7.72-7.61 1H, m), 7.40-7.12 (4H, m), 5.87 (1H, d, *J* = 9.2 Hz), 4.64-4.43 (5H, m), 4.34-4.25 (1H, m), 4.14-4.05 (1H, m), 3.86-3.70 (2H, m), 3.37-3.23 (2H, m), 2.90-2.77 (5H, m), 2.49-2.38 (1H, m), 2.18-2.05 (1H, m), 1.81-1.67 (2H, m), 1.66-1.49 (2H, m), 1.49-1.37 (2H, m), 1.35-1.19 (3H, m). |
| 18 | 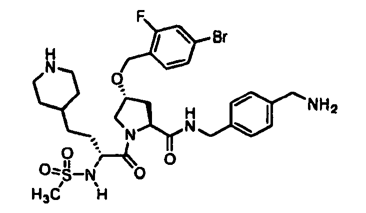 | MS *m*/*z* 682.4 (M + 1) |
| 19 | 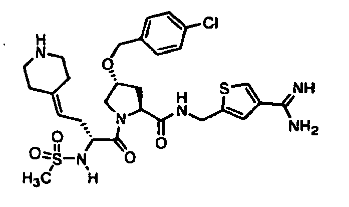 | MS *m*/*z* 637.2 (M + 1) |
| 20 | 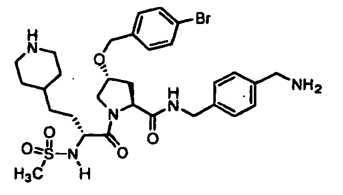 | MS *m*/*z* 664.2 (M + 1); ¹H NMR (CD₃CN, 400 MHz) δ 7.55-7.26 (8H, m), 6.03-5.91 (1H, m), 4.58-4.40 (3H, m), 4.39-4.23 (3H, m), 4.09-4.04 (3H, m), 3.84-3.70 (2H, m), 3.34-3.27 (2H, m), 2.86-2.79 (5H, m), 2.46-2.38 (1H, m), 2.14-2.06 (1H, m), 1.83-1.67 (2H, m), 1.67-1.48 (2H, m), 10.48-1.42 (2H, m), 1.42-1.25 (3H, m). |
| 21 | 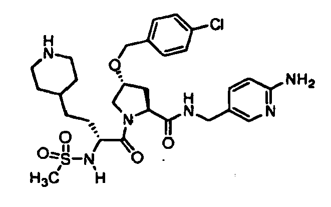 | MS *m*/*z* (M + 1) |
| 22 | 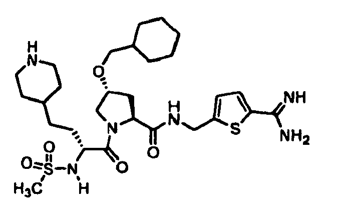 | MS *m*/*z* 611.3 (M + 1); Anal. Calcd. for C₃₂H₅₂F₆N₆O₁₁S₂ (2 TFA, 2 H₂O): C, 43.93; H, 5.99; N, 9.61; Found: C, 43.80; H, 5.60; N, 9.17;¹H NMR (CD₃CN, 400 MHz) 8 9.34(1 H, s), 7.87-7.81 (2H, m), 7.72 (1H, d, *J* = 3.6 Hz), 7.60-7.34 (2H, m), 7.10 (1H, s, *J* = 3.2 Hz), 6.07-5.98 (1H, m), 4.56-4.47 (2H, m), 4.37 (1H, t, *J* = 7.6 Hz), 4.12-4.02 (2H, m), 3.79-3.61 (2H, m), 3.35-3.32 (2H, m), 3.27-3.18 (2H, m), 2.91-2.82 (5H, m), 2.36-2.26 (1H, m), 2.09-1.98 (1H, m), 1.84-1.81 (2H, m), 1.75-1.64 (6H, m), 1.58-1.10 (10H, m), 0.95-0.83 (2H, m). |
| 23 | 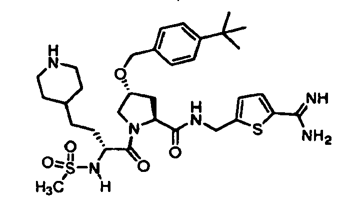 | MS *m*/*z* 661.3 (M+ 1) |
| 24 | 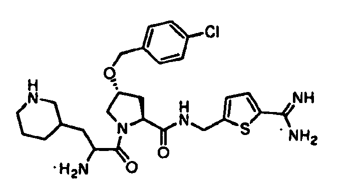 | MS *m*/*z* (M + 1) |
| 25 | 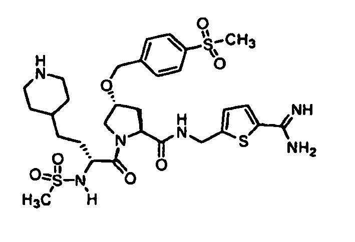 | MS *m*/*z* 683.2 (M + 1); ¹H NMR (CD₃CN, 400 MHz) 8 10.19(1 H, s), 7.93 (2H, d, *J* = 8.4 Hz), 7.73 (1H, d, *J* = 4 Hz), 7.58 (2H, d, *J* = 8.4 Hz), 7.52-7.40 (2H, m), 7.15 (1H, d, *J* = 4 Hz), 5.81-5.73 (1H, m), 4.74-4.61 (2H, m), 4.57 (2H, d, *J* = 5.2 Hz), 4.46 (1 H, t, *J* = 7.6 Hz), 4.39-4.34 (1H, m), 4.15-4.06 (1H, m), 3.86-3.26 (2H, m), 3.35-3.26 (2H, m), 3.09 (3H, s), 2.89-2.77 (5H, m), 2.49-2.39 (1H, m), 2.22-2.11 (1 H, m), 1.82-1.70 (2H, m), 1.77-1.50 (2H, m), 1.50 (2H, m), 1.38-1.21 (3H, m). |
| 26 | 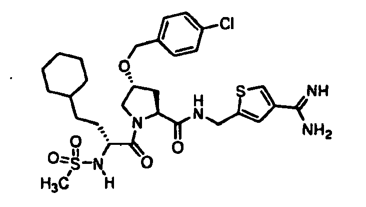 | MS *m*/*z* 638.2 (M + 1) |
| 27 | 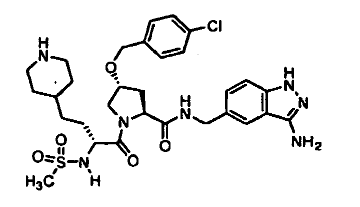 | MS *m*/*z* (M + 1) |
| 28 | 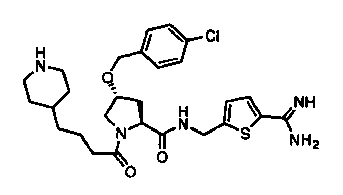 | MS *m*/*z* (M + 1) |
| 29 | 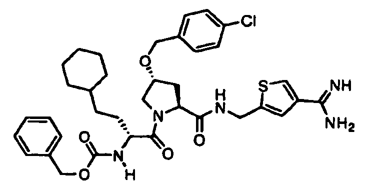 | MS *m*/*z* (m + 1) |
| 30 | 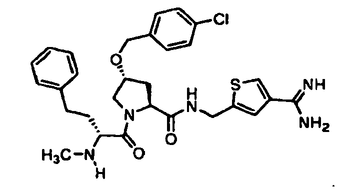 | MS *m*/*z* 568.2 (M + 1) |
| 31 | 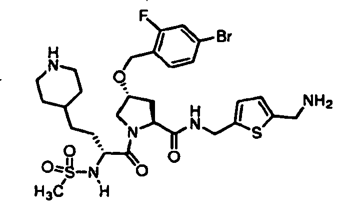 | MS *m*/*z* 688.5 (M + 1) |
| 32 | 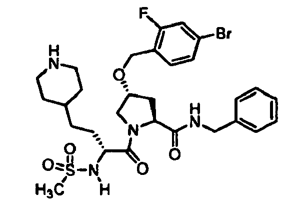 | MS *m*/*z* 653.2 (M + 1) |
| 33 | 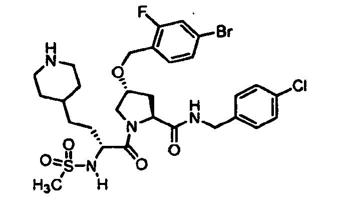 | MS *m*/*z* 687.3 (M+1) |
| 34 | 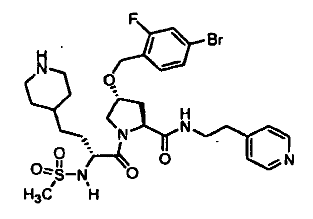 | MS *m*/*z* 668.3 (M + 1) |
| 35 | 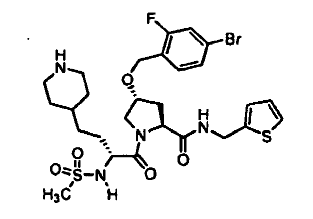 | MS *m*/*z* 659.3 (M + 1) |
| 36 | 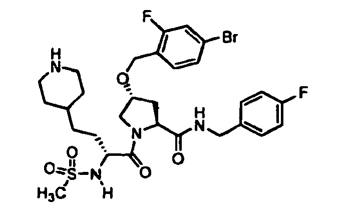 | MS *m*/*z* 671.2 (M + 1) |
| 37 | 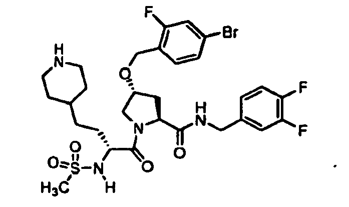 | MS *m*/*z* 689.2 (M + 1) |
| 38 | 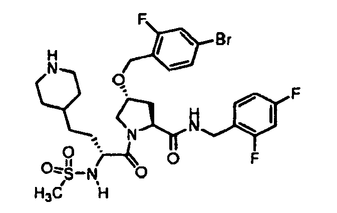 | MS *m*/*z* 689.2 (M + 1) |
| 39 | 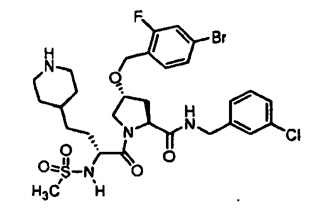 | MS *m*/*z* 687.2 (M + 1) |
| 40 | 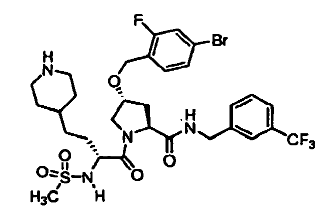 | MS *m*/*z* 721.3 (M + 1) |
| 41 | 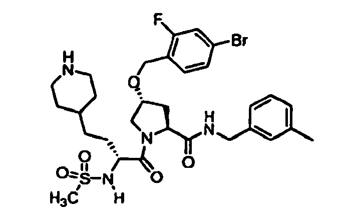 | MS *m*/*z* 667.3 (M + 1) |
| 42 | 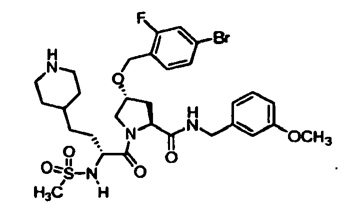 | MS *m*/*z* 683.3 (M + 1) |
| 43 | 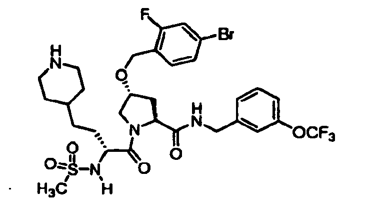 | MS *m*/*z* 737.3 (M + 1) |
| 44 | 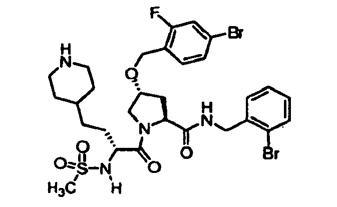 | MS *m*/*z* 731.2 (M + 1) |
| 45 | 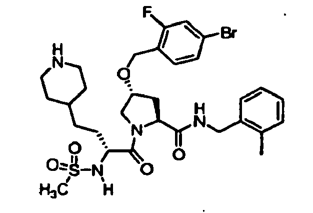 | MS *m*/*z* 667.3 (M + 1) |
| 46 | 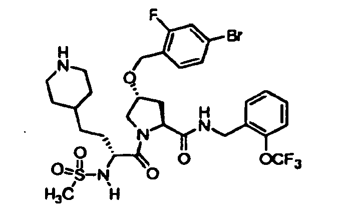 | MS *m*/*z* 737.3 (M + 1) |
| 47 | 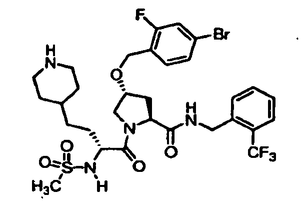 | MS *m*/*z* 721.3 (M + 1) |
| 48 | 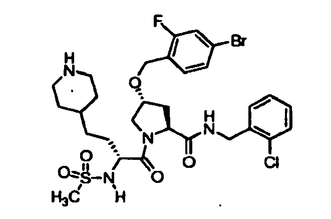 | MS *m*/*z* 687.3 (M + 1) |
| 49 | 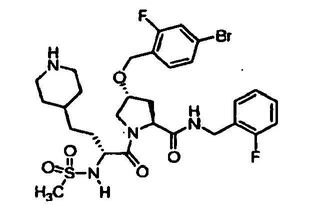 | MS *m*/*z* 671.3 (M + 1) |
| 50 | 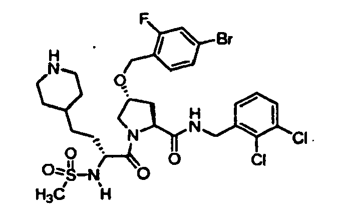 | MS *m*/*z* 721.2 (M + 1) |
| 51 | 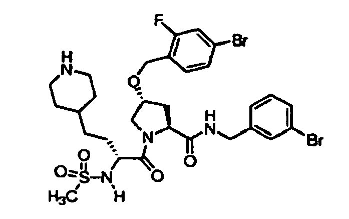 | MS *m*/*z* 731.2 (M + 1) |
| 52 | 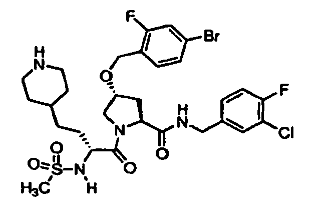 | MS *m*/*z* 705.3 (M + 1) |
| 53 | 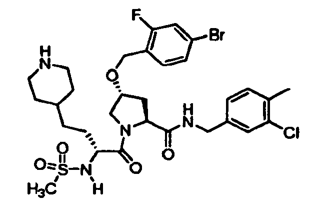 | MS *m*/*z* 701.3(M + 1) |
| 54 | 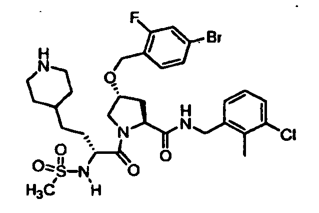 | MS *m*/*z* 701.3 (M + 1) |
| 55 | 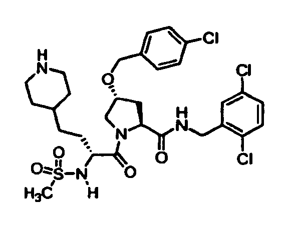 | MS m/z 659.2 (M + 1), ¹H NMR (CDCl₃, 400 MHz) δ 9.30 (1H, bs), 8.56 (1H, bs), 7.31 (1H, d, *J* = 2 Hz ), 7.07-7.27 (6H, m), 5.88 (1H, d, *J* = 8.4 Hz), 4.26-4.57 (6H, m), 3.93-4.02 (1H, m), 3.77-3.86 (1 H, m), 3.47-3.86 (1H, m), 3.21-3.34 (5 H, m), 2.74 (3H, s), 2.49-2.88 (4H, m), 2.17-2.36 (2H, m), 1.18-1.73 (9H, m). |
| 56 | 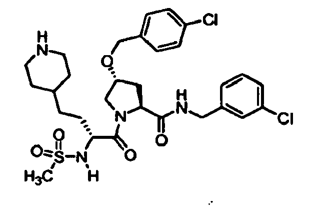 | MS *m*/*z* 625.2 (M + 1) |
| 57 | 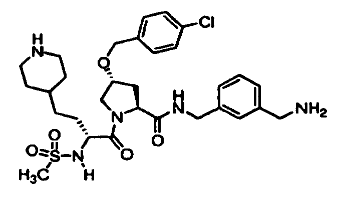 | MS *m*/*z* 620.2 (M + 1) |
| 58 | 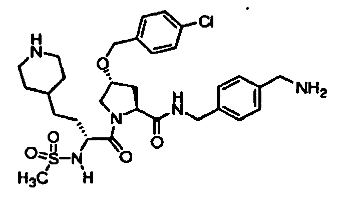 | MS *m*/*z* 620.2 (M + 1) |
| 59 | 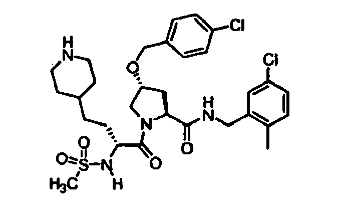 | MS *m*/*z* 639.2 (M + 1) |
| 60 | 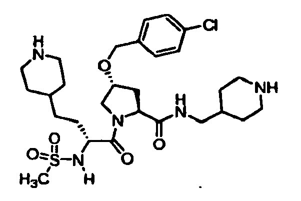 | MS *m*/*z* 598.2 (M + 1) |
| 61 | 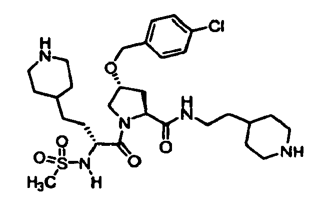 | MS *m*/*z* 612.2 (M + 1) |
| 62 | 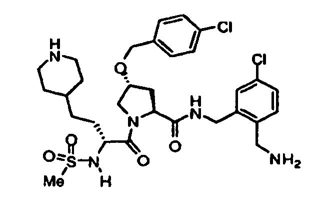 | MS *m*/*z* 654.3 (M + 1) |
| 63 | 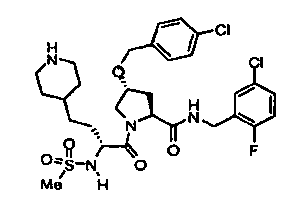 | MS *m*/*z* 643.2 (M + 1) |
| 64 | 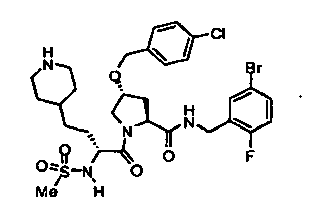 | MS *m*/*z* 687.2 (M + 1) |
| 65 | 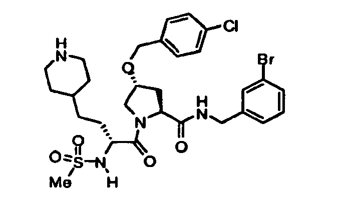 | MS *m*/*z* 669.2 (M + 1) |
| 66 | 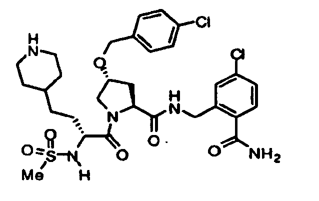 | MS *m*/*z* 668.3 (M + 1) |
| 67 | 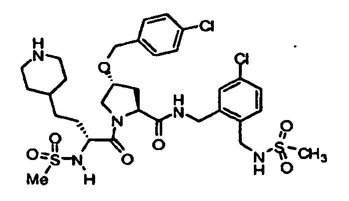 | MS *m*/*z* 732.3 (M + 1) |
| 68 | 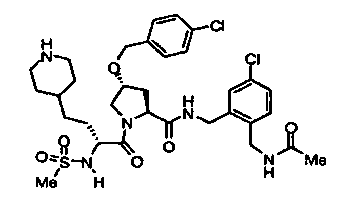 | MS *m*/*z* 696.3 (M + 1) |
| 69 | 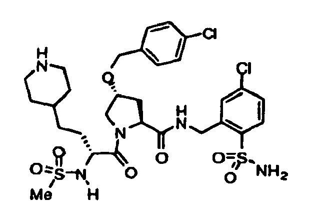 | MS *m*/*z* 704.2 (M + 1) |
| 70 | 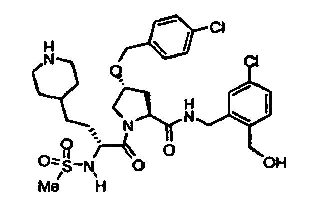 | MS *m*/*z* 655.3 (M + 1) |

### Assays

The suitability of a channel activating protease inhibitor such as a prostasin inhibitor for the treatment of a disease mediated by inhibition of a channel activating protease may be tested by determining the inhibitory effect of the channel activating protease inhibitor on: (1) the native, isolated, purified or recombinant channel activating protease, using a suitable biochemical assay format, using the method described in Shipway et al.; Biochemical and Biophysical Research Communications 2004; 324(2):953-63; and/or (2) the ion channel/ion transport function in suitable isolated cells or confluent epithelia, using the methods described in Bridges et al.; American Journal of Physiology Lung Cell Molecular Physiology 2001; 281(1):L16-23; and Donaldson et al.; Journal of Biological Chemistry 2002; 277(10):8338-45.

### Biochemical assays

Recombinant human prostasin and matriptase and guinea pig prostasin are generated according to methods described in Shipway et al., Biochem, and Biophys. Res. Commun. 2004; 324(2):953-63. The recombinant enzymes are incubated in an electrolyte buffer containing the test compounds or vehicle in a suitable multiple well assay plate such as a 96 or 384 well plate. At a defined time after the mixing of enzyme with compound or vehicle, a suitable fluorescent peptide substrate is added to the assay mixture. As substrate becomes cleaved by the active enzyme, fluorescence (measured, using a suitable fluorescence plate reader) increases and the rate of turnover of substrate (i.e. enzyme activity) may be quantified and thus the inhibitory effect of any test compound. The efficacy of test compounds is expressed as the concentration that induces 50% attenuation in the enzyme activity (Kᵢ).

In general, compounds of the invention may have Kᵢ values from 0.1 nM to 5 µM. In some examples, compounds of the invention may have Kᵢ values from 0.1 nM to 500 nM; from 0.1 nM to 50 nM; from 0.1 nM to 5 nM; or from 0.1 nM to 0.5 nM. In particular examples, compounds of the invention may have Kᵢ values from 0.1 nM to 0.5 nM; from 0.5 nM to 5 nM; from 5 nM to 50 nM; from 50 nM to 500 nM; or from 500 nM to 5 µM. In yet other examples, compounds may have Kᵢ values less than 0.1 nM or more than 5 µM.

### Epithelial ion transport

Human bronchial epithelial cells are cultured according to methods described in Danahay et al., Am. J. Physiol. Lung Cell Mol. Physiol. 2002; 282(2):L226-36. When suitably differentiated (days 14-21 after establishing an apical-air interface), epithelial cells are treated with either vehicle, aprotinin (200 µg/ml) or test compound for 90 minutes. Epithelia are then placed into chambers as described in Danahay et al., *supra,* maintaining the concentration of vehicle, aprotinin or test compound on the apical side of the epithelia. Short circuit current (ISC) is then measured by voltage clamping the epithelia to zero millivolts. The amiloride-sensitive ISC is then measured by the addition of amiloride (10 µM) to the apical surface of the epithelia. The potency of the test compound is expressed as the concentration inducing a 50% inhibition of the total aprotinin-sensitive component of the amiloride-sensitive ISC.

In general, compounds of the invention may have IC₅₀ values from 1 nM to 10 µM. In some examples, compounds of the invention may have IC₅₀ values from 1 nM to 1 µM; or more particularly from 1 nM to 100 nM. In yet other examples, compounds of the invention may have IC₅₀ values from 100 nM to 1 µM, or from 1 µM to 10 µM. In yet other examples, compounds may have IC₅₀ values less than 1 nM or more than 10 µM.

### Tracheal potential difference (in vivo)

Guinea pigs are anaesthetized, using a short acting inhalation anaesthesia such as halothane and N₂0. While under short acting anaesthesia, an oral gavage needle is inserted into the trachea via the oropharangeal route. Once inside the trachea, a small volume (50-200 µl) of vehicle or test compound, in a suitable aqueous-based diluent, is instilled into the airways. Animals then recover and become fully ambulatory. Alternatively, test compounds may be administered to animals, using aerosol or dry powder dosing. At a defined time after dosing, the animals are surgically anaesthetized, using a suitable anaesthesia such as ketamine and xylazine. The trachea is then exposed and a plastic agar bridge electrode is inserted into the tracheal lumen. A reference electrode is also inserted into the layers of muscle in the animal's neck. The tracheal potential difference is then measured, using a suitable high impedance voltmeter as described in Takahashi et al., Toxicol Appl Pharmacol. 1995; 131(1):31-6. The potency of the test compound is expressed as the dose inducing a 50% reduction in the sensitive-component of the tracheal potential difference.

## Claims

1. A compound of Formula (1): 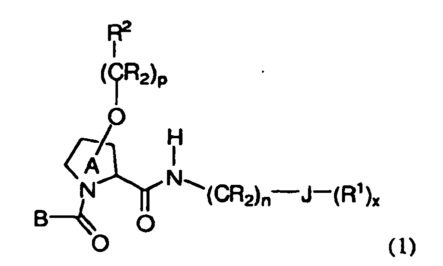 or pharmaceutically acceptable salts thereof, wherein
O-(CR₂)ₚ-R² is a substituent at any position on ring A;
J is a 5-12 membered monocyclic or fused carbocyclic ring, heterocyclic ring comprising N, O and/or S; aryl or heteroaryl ring, provided J is not triazolyl;
B is 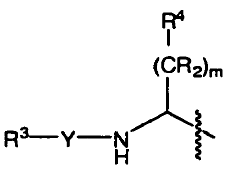 or (CR₂)ₖ-R⁵;
Y is a bond, -SO₂-, -NHCO- or -O-(CO)-;
R¹ is halo, -(CR₂)ₜ-NR⁶R⁷, -(CR₂)ₜ-NRC(=NR)-NR⁶R⁷, -(CR₂)ₜ-C(=NR)-NR⁶R⁷, -C(O)-(CR₂)ₜ-NR⁶R⁷, -(CR₂)ₜ-NR-SO₂R⁶, -(CR₂)ₜ-NR-C(O)-R⁶, -(CR₂)ₜ-SO₂NR⁶R⁷, or -(CR₂)ₜ-OR⁶, or an optionally substituted C₁₋₆ alkoxy, C₁₋₆ alkyl, C₂₋₆ alkenyl or C₂₋₆ alkynyl; or an optionally substituted carbocyclic ring, heterocyclic ring, aryl or heteroaryl;
R³ is C₁₋₆ alkyl, C₂₋₆ alkenyl, C₂₋₆ alkynyl or -(CR₂)ₜ-R⁵;
alternatively, NH-Y-R³ together fonn NH₂;
R², R⁴ and R⁵ are independently an optionally substituted 5-12 membered carbocyclic ring, heterocyclic ring, aryl or heteroaryl; or R⁴ is H, C₁₋₆ alkyl, C₂₋₆ alkenyl, C₂₋₆ alkynyl, or 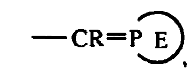 wherein P is C or N and ring E together with P form an optionally substituted 5-12 membered monocyclic or fused ring;
R⁶ and R⁷ are independently H, C₁₋₆ alkyl, C₂₋₆ alkenyl, C₂₋₆ alkynyl or -(CR₂)ᵣ-R⁵;
each R is H, or C₁₋₆ alkyl, C₂₋₆ alkenyl, or C₂₋₆ alkynyl;
1 is 0-6;
k, m, n and p are independently 1-6;
x is 0-4;
provided R⁴ is piperidinyl when NH-Y-R³ together form NH₂; and
further provided that R⁵ is piperidinyl when B is (CR₂)ₖ-R⁵.

2. The compound of claim 1, wherein J is thiophenyl, thiazolyl, phenyl, pyridyl, indazolyl, piperidinyl or pyrrolidinyl.

3. The compound of claim 1, wherein R¹ is halo, C₁₋₆alkyl, CF₃, OCF₃, phenyl, -(CR₂)ₜ-NR⁶R⁷, -(CR₂)ₜ-C(=NR)-NR⁶R⁷, -C(O)-(CR₂)ₜ-NR⁶R⁷, -(CR₂)ₜ-NR-SO₂R⁶, -(CR₂)ₜ-NR-C(O)-R⁶, -(CR₂)ₜ-SO₂NR⁶R⁷, or-(CR₂)ₜ-OR⁶; wherein each 1 is 0-1; and
R, R⁶ and R⁷ are independently H or C₁₋₆ alkyl.

4. The compound of claim 1, wherein R⁷ is phenyl or cyclohexyl, each of which optionally substituted with halo, SO₂(C₁₋₆ alkyl), or an optionally halogenated C₁₋₆ alkyl or C₁₋₆ alkoxy.

5. The compound of claim 1, wherein R⁴ is an optionally substituted piperidinyl, cyclohexyl, phenyl,

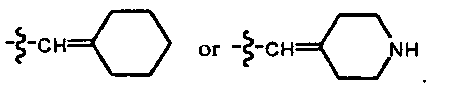

6. The compound of claim 1, wherein Y is a bond, SO₂ or -O-(CO)-.

7. The compound of claim 1, wherein said compound is of Formula (2): 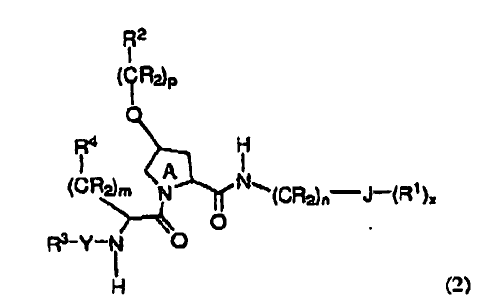 wherein R² and J are independently an optionally substituted 6-membered aryl; R³ is C₁₋₆ alkyl, C₂₋₆alkenyl, C₂₋₆ alkynyl or -(CR₂)ₜ-R⁵; or NH-Y-R³ together form NH₂; each R in (CR₂) is H or C₁₋₆ alkyl; and
m, n and p are independently 1-2,
or pharmaceutically acceptable salts thereof.

8. The compound of claim 7, wherein R² and J are independendy an optionally substituted phenyl.

9. The compound of claim 7, wherein x is 1-3.

10. The compound of claim 7, wherein Y is SO₂.

11. The compound of claim 7, wherein R³ is C₁₋₆ alkyl or an optionally substituted benzyl.

12. The compound of claim 7, wherein R⁴ is an optionally substituted piperidinyl.

13. The compound of claim 1, wherein said compound is selected from thte group consisting of: 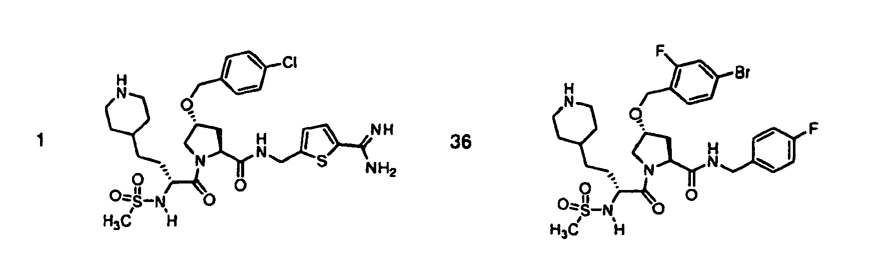 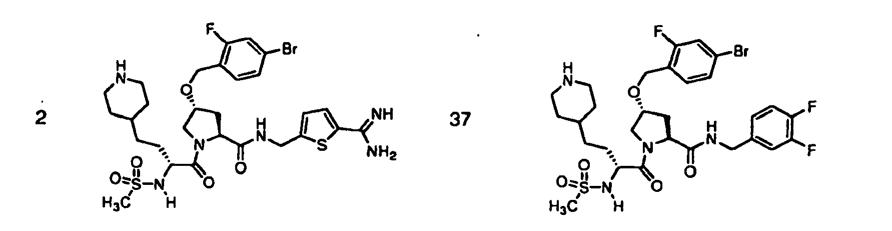 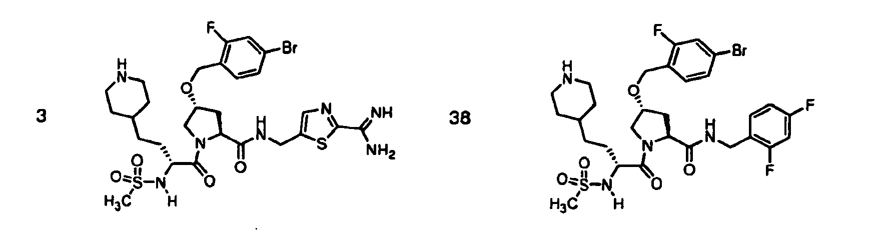 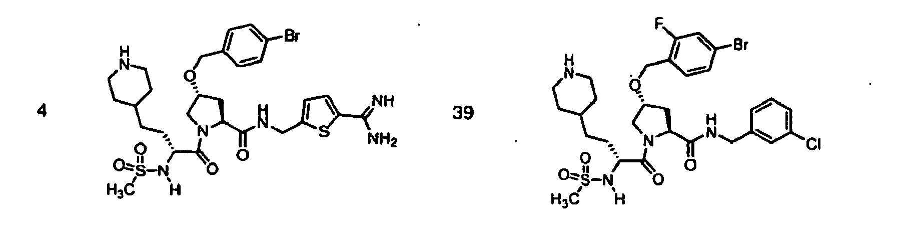 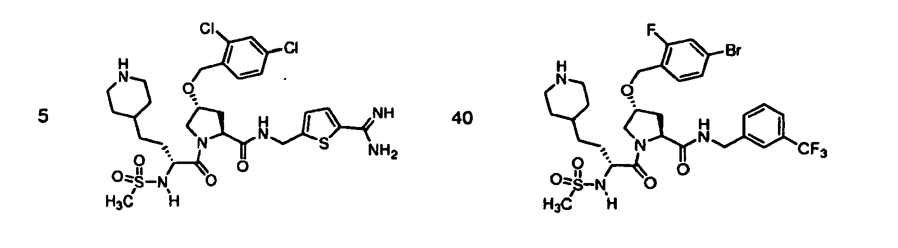 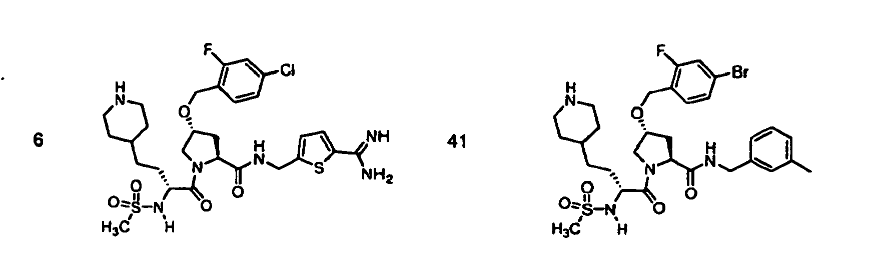 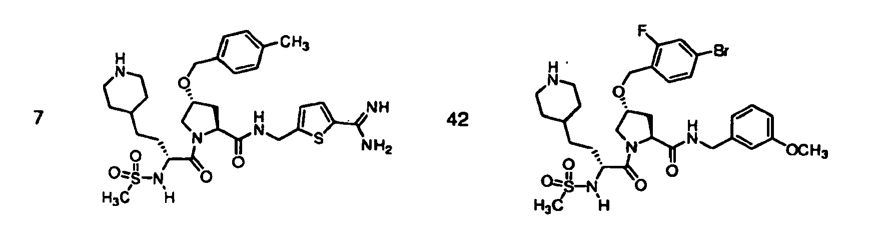 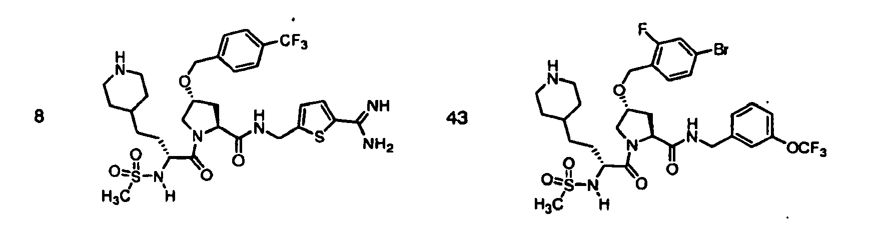 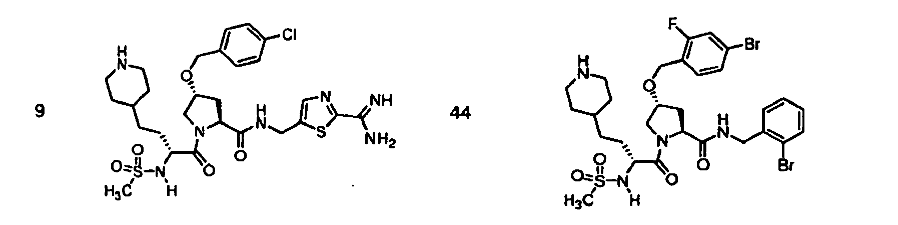 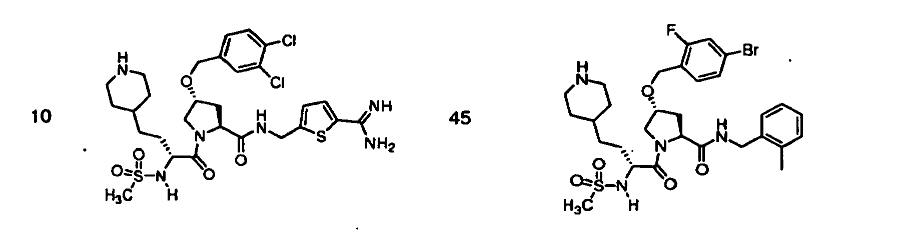 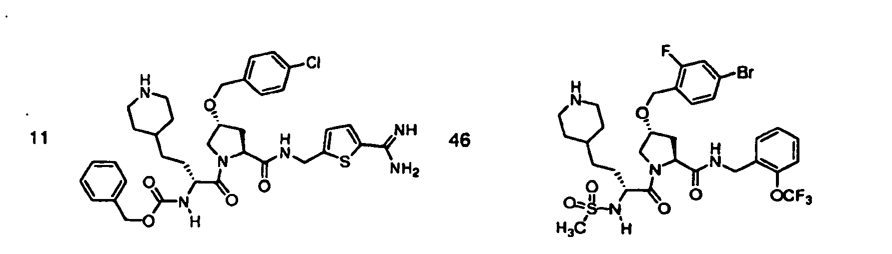 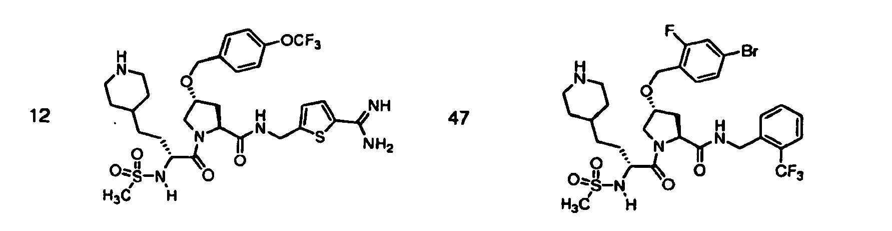 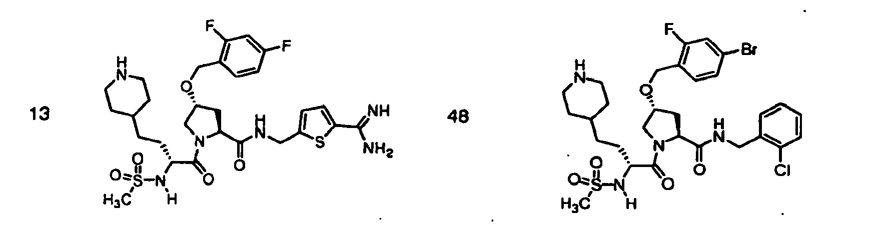 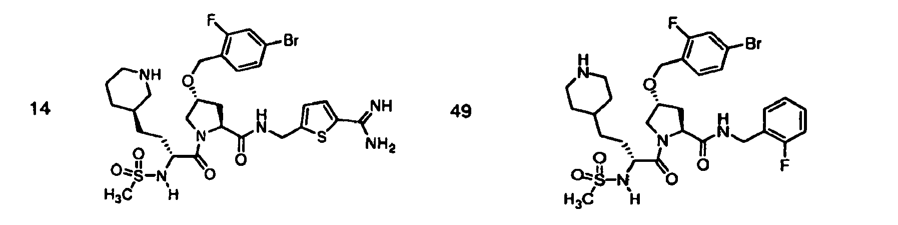 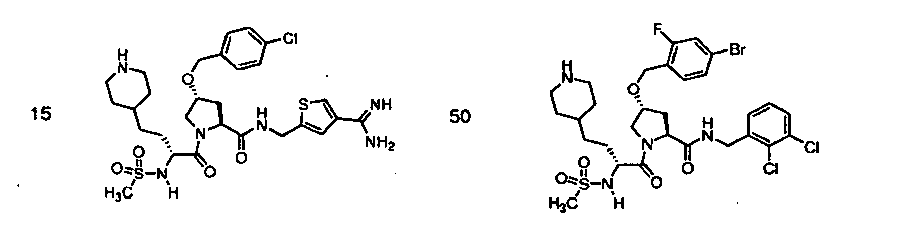 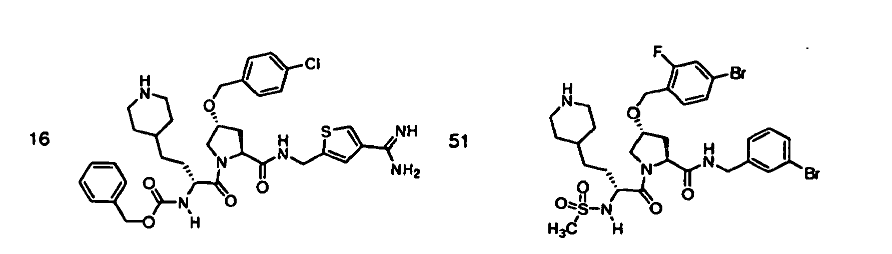 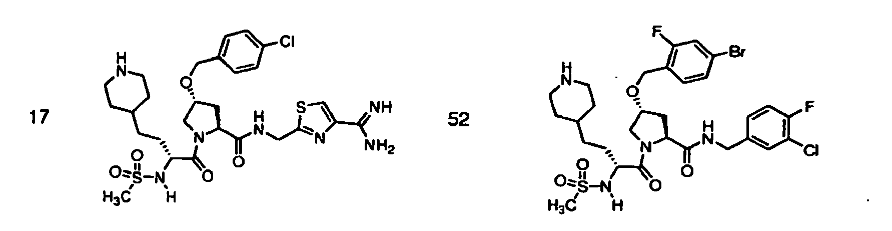 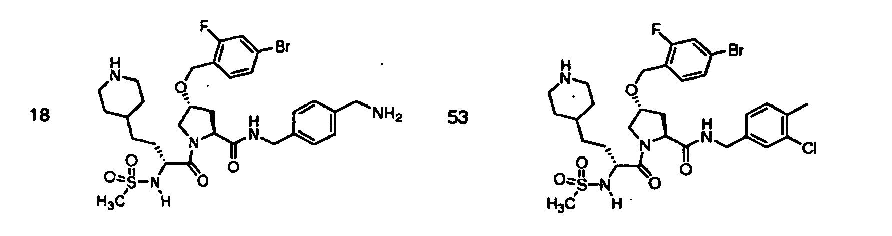 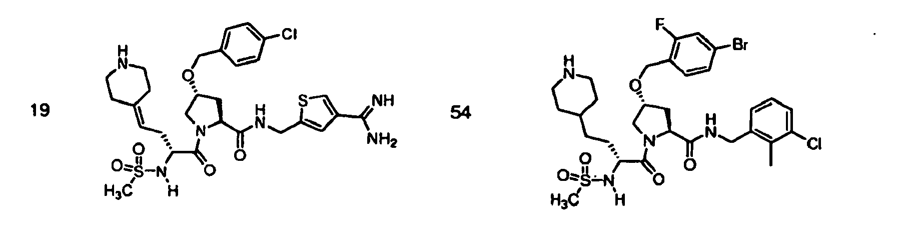 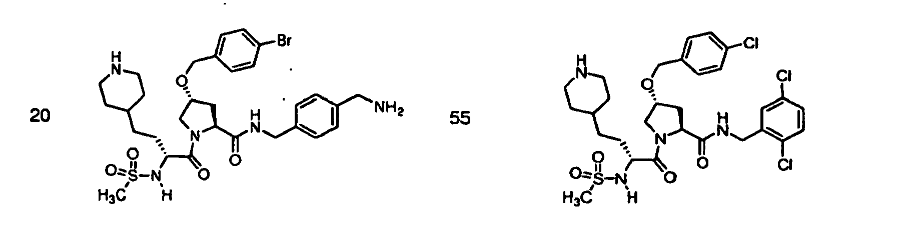 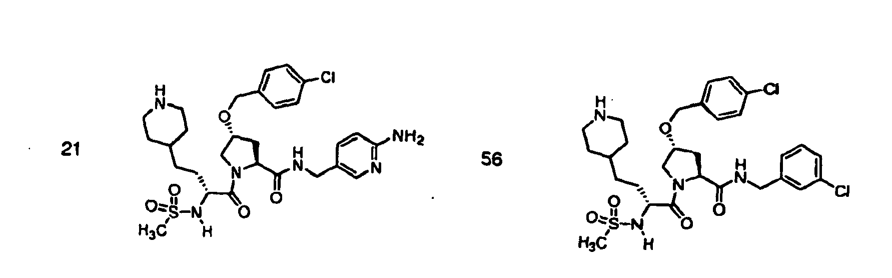 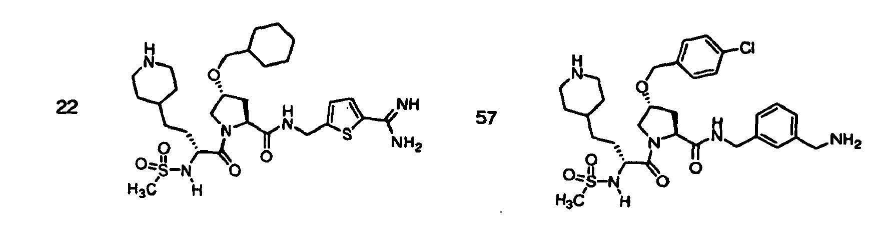 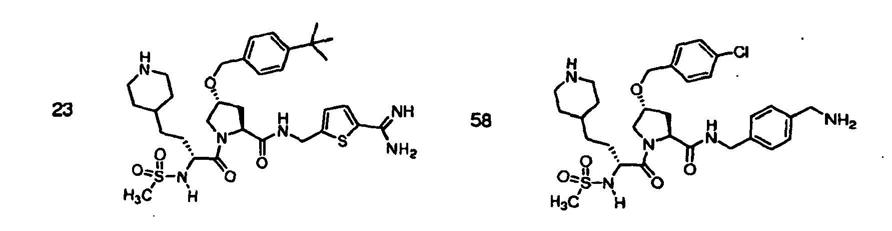 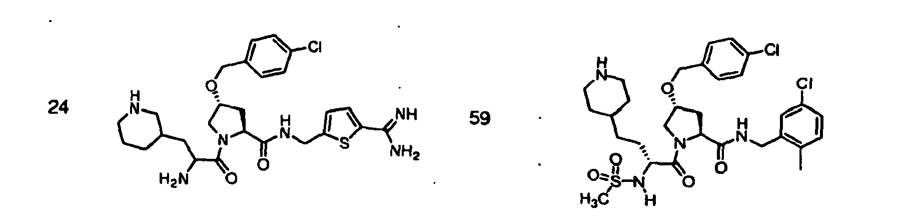 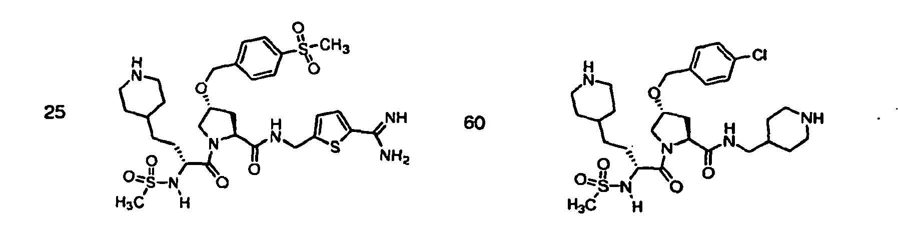 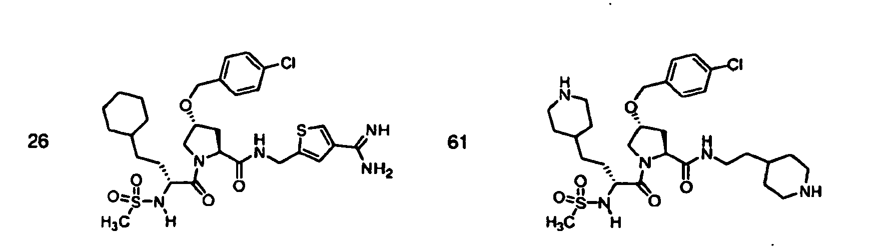 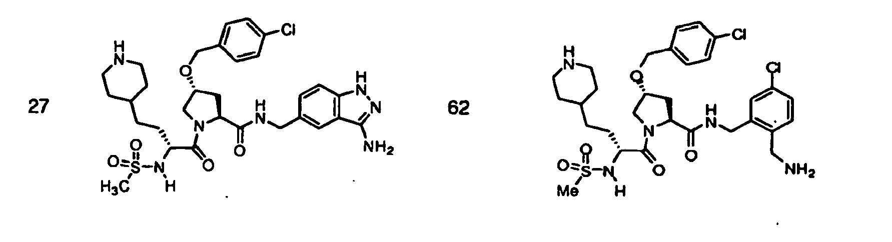 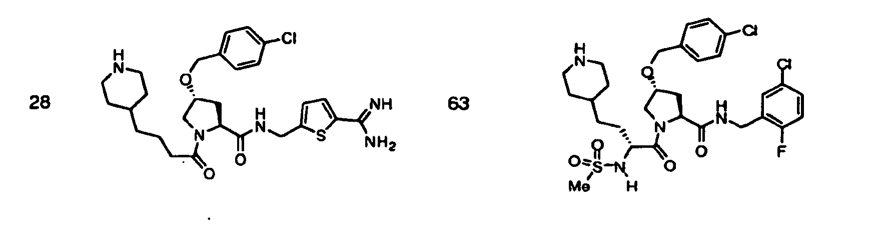 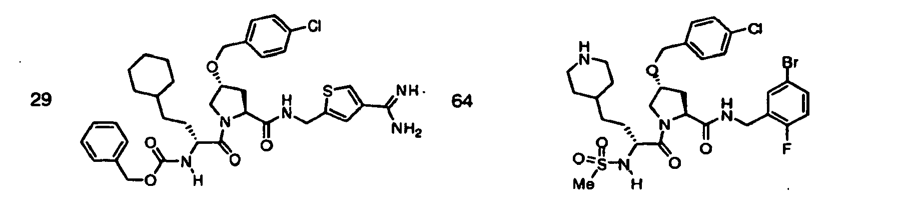 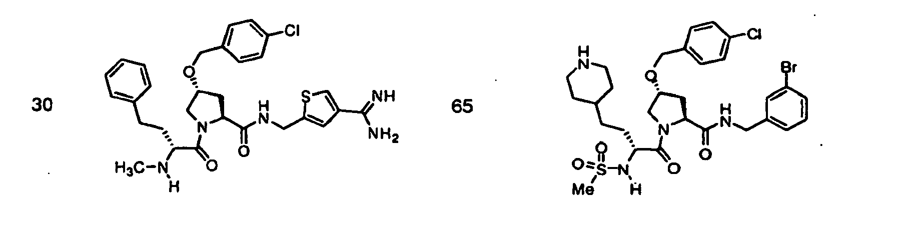 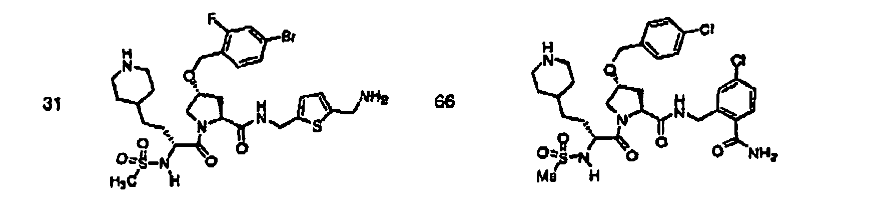 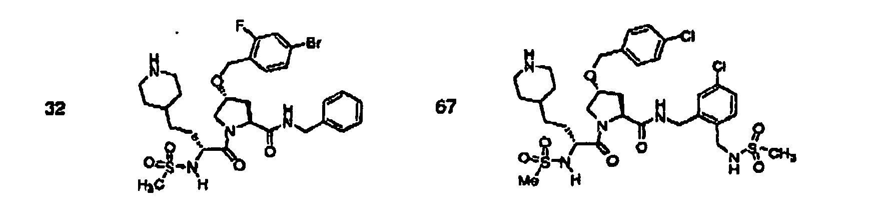 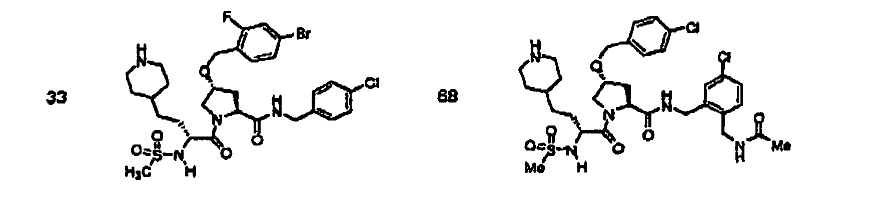 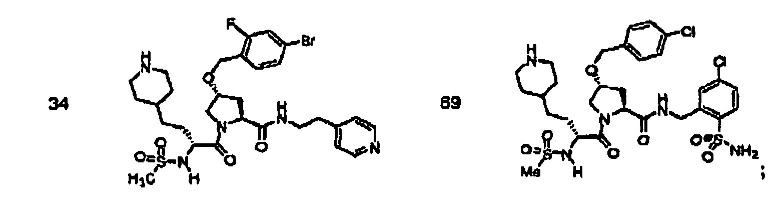 and 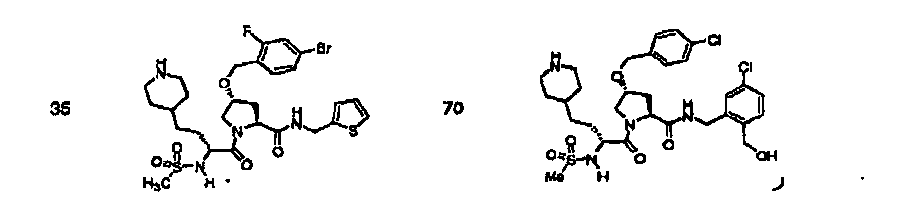 or pharmaceutically acceptable salts thereof.

14. The compound of claim 1, wherein said compound is selected from the group consisting of: 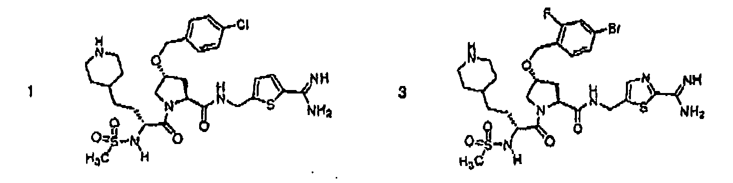 or pharmaceutically acceptable salts thereof.

15. The compound of claim 1, wherein said compound is selected from the group consisting of: 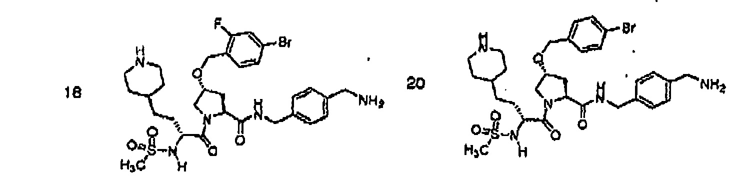 or pharmaceutically acceptable salts thereof.

16. The compound of claim 1, wherein said compound is selected from the group consisting of: 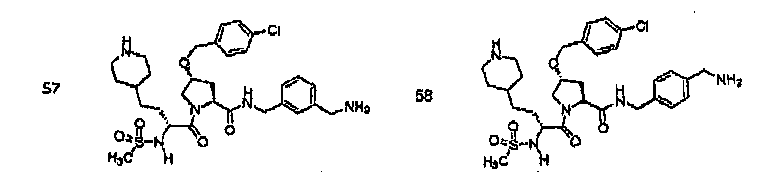 or pharmaceutically acceptable salts thereof.

17. The compound of claim 1, wherein said compound is 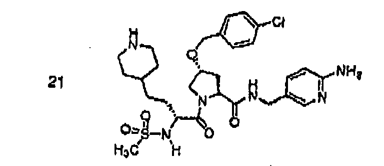 or pharmaceutically acceptable salts thereof.

18. The compound of claim 1, wherein said compound is 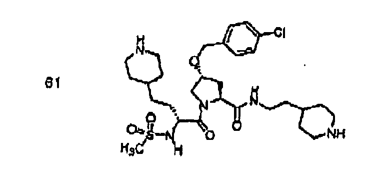 or pharmaceutically acceptable salts thereof.

19. The compound of claim 1, wherein said compound is 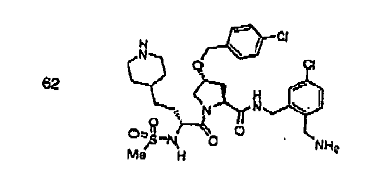 or pharmaceutically acceptable salts thereof.

20. A pharmaceutical composition comprising a therapeutically effective amount of a compound according to any of claims 1-19.

21. A compound of any one of claims 1-19 for inhibiting a channel activating protease in a cell or tissue system or in a mammal, wherein said channel activating procease is prostasin, PRSS22, TMPRSS11 (*e.g*.. TMPRSS11B, TMPRSS11E), TMPRSS2, TMPRSS3, TMPRSS4 (MTSP-2), matriptase (MTSP-1), CAP2, CAP3, trypsin, cathepsin A, or neutrophil elastase.

22. Use of a compound of any one of claim 1-19 for the manufacture of a medicament for treating a condition mediated by a channel activating protease in a cell or tissue system or in a mammal, and optionally in combination with a second therapeutic agent; wherein said channel activating protease is prostasin, PRSS22, TMPRSS11 (*e.g*., TMPRSS 11B, TMPRSS11E), TMPRSS2. TMPRSS3, TMPRSS4 (MTSP-2), matriptase (MTSP-1), CAP2, CAF3, trypsin. cathepsin A, or neutrophil elastase.

23. The use of claim 22 wherein said condition is associated with the movement of fluid across ion transporting epithelia or the accumulation of mucus and sputum in respiratory tissues, or a combination thereof.

24. The use of claim 22, wherein said condition is cystic fibrosis, primary ciliary dyskinesia, lung carcinoma, chronic bronchitis, chronic obstructive pulmonary disease, asthma or a respiratory tract infection.

25. The use of claim 22, wherein said second therapeutic agent is an anti-inflammatory, bronchodilatory, andhistamine, anti-cussive, antibiotic or DNase, and is administered prior to, simultaneously with, or after the compound according to any of claims 1.

26. The compound of claim 21 or use of 22, wherein said channel activating protease is prostasin.

27. The compound of claim 21 or use of 22, wherein said cell or tissue system comprises bronchial epithelial cells.

## Patentansprüche

1. Verbindung der Formel (1): 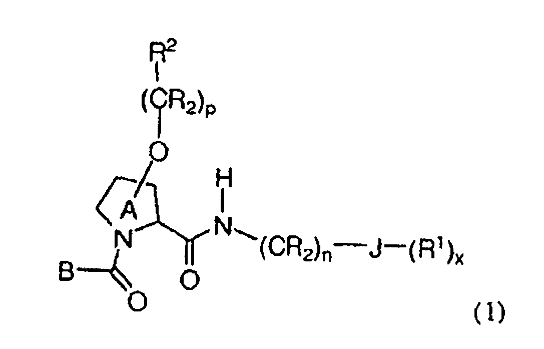 oder ein pharmazeutisch annehmbares Salz davon, worin
O-(CR₂)ₚ-R² ein Substituent an einer beliebigen Position an dem Ring A ist;
J ein 5- bis 12-gliedriger monozyklischer oder kondensierter Ring, ein heterozyklischer Ring, der N, O und/oder S umfasst, oder ein Aryl- oder Heteroarylring ist, mit der Maßgabe, dass J nicht Triazolyl ist;
B= 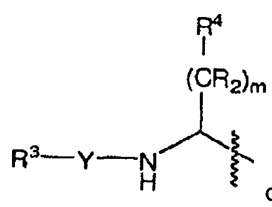 oder (CR₂)ₖ-R⁵ ist;
Y eine Bindung, -SO₂-, -NHCO- oder -O-(CO)- ist;
R¹ Halogen, -(CR₂)ₗ-NR⁶R⁷, -(CR₂)ₗ-NRC(=NR)-NR⁶R⁷, -(CR₂)ₗ-C(=NR)-NR⁶R⁷, -C(O)-(CR₂)ₗ-NR⁶R⁷, -(CR₂)ₗ-NR-SO₂R⁶, -(CR₂)ₗ-NR-C(O)-R⁶, -(CR₂)ₗ-SO₂NR⁶R⁷ oder -(CR₂)ₗ-OR oder ein gegebenenfalls substituiertes C₁₋₆-Alkoxy, C₁₋₆-Alkyl, C₂₋₆-Alkenyl oder C₂₋₆-Alkinyl oder ein gegebenenfalls substituierter carbozyklischer Ring, heterozyklischer Ring, ein Aryl oder Heteroaryl ist;
R³ C₁₋₆-Alkyl, C₂₋₆-Alkenyl, C₂₋₆-Alkinyl oder -(CR₂)ₗ-R⁵ ist;
wobei NH-Y-R³ alternativ dazu NH₂ bilden;
R², R⁵ und R⁵ unabhängig voneinander ein gegebenenfalls substituierter 5- bis 12-gliedriger carbozyklischer Ring, heterozyklischer Ring, Aryl oder Heteroaryl sind oder
R⁴ H, C₁₋₆-Alkyl, C₂₋₆-Alkenyl, C₂₋₆-Alkinyl oder 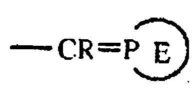 ist, worin P = C oder N ist und der Ring E gemeinsam mit P einen gegebenenfalls substituierten, 5- bis 12-gliedrigen monozyklischen oder kondensierten Ring bildet;
R⁶ und R⁷ unabhängig von H, C₁₋₆-Alkyl, C₂₋₆-Alkenyl, C₂₋₆-Alkinyl oder -(CR₂),-R⁵ ist; die R jeweils H oder C₁₋₆-Alkyl, C₂₋₆-Alkenyl oder C₂₋₆-Alkinyl sind;
I = 0 bis 6 ist;
k, m, n und p unabhängig voneinander 1 bis 6 sind;
x = 0 bis 4 ist,
mit der Maßgabe, dass R⁴ Piperidinyl ist, wenn NH-Y-R³ gemeinsam NH₂ bilden, und mit der Maßgabe, dass R⁵ Piperidinyl ist, wenn B = (CR₂)ₖ-R⁵ ist.

2. Verbindung nach Anspruch 1, worin J Thiophenyl, Thiazolyl, Phenyl, Pyridyl, Indazolyl, Piperidinyl oder Pyrrolidinyl ist.

3. Verbindung nach Anspruch 1, worin R¹ Halogen, C₁₋₆-Alkyl, CF₃, OCF₃, Phenyl, -(CR₂)ₗ-NR⁶R⁷, -(CR₂)ₗ-C(=NR)-NR⁶R⁷, -C(O)-(CR₂)ₗ-NR⁶R⁷, -(CR₂)ₗ-NR-SO₂R⁶, -(CR₂)ₗ-NR-C(O)-R⁶, -(CR₂)ₗ-SO₂NR⁶R⁷ oder -(CR₂)ₗ-OR⁶ ist, worin I = 0 bis 1 ist und R, R⁶ und R⁷ unabhängig voneinander H oder ein C₁₋₆-Alkyl sind.

4. Verbindung nach Anspruch 1, worin R² Phenyl oder Cyclohexyl ist, die jeweils gegebenenfalls mit Halogen, SO₂(C₁₋₆-Alkyl) oder gegebenenfalls halogeniertem C₁₋₆-Alkyl oder C₁₋₆-Alkoxy substituiert sind.

5. Verbindung nach Anspruch 1, worin R⁴ ein gegebenenfalls substituiertes Piperidinyl, Cyclohexyl, Phenyl, 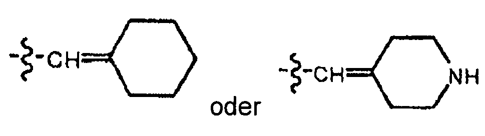 ist.

6. Verbindung nach Anspruch 1, worin Y eine Bindung, SO₂ oder -O-(CO)- ist.

7. Verbindung nach Anspruch 1, worin die Verbindung eine Verbindung der Formel (2) ist: 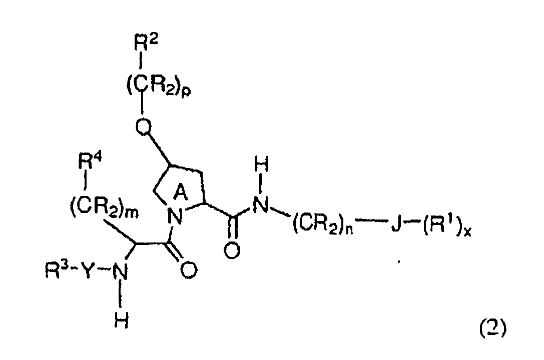 worin R² und J unabhängig voneinander ein gegebenenfalls substituiertes 6-gliedriges Aryl sind;
R³ C₁₋₆-Alkyl, C₂₋₆-Alkenyl, C₂₋₆-Alkinyl oder -(CR₂)ₗ-R⁵ ist; oder NH-Y-R³ gemeinsam NH₂ bilden;
die R in (CR₂) jeweils H oder ein C₁₋₆-Alkyl sind; und
m, n und p unabhängig voneinander 1 bis 2 sind;
oder pharmazeutisch annehmbare Salze davon.

8. Verbindung nach Anspruch 7, worin R² und J unabhängig voneinander ein gegebenenfalls substituiertes Phenyl sind.

9. Verbindung nach Anspruch 7, worin x = 1 bis 3 ist.

10. Verbindung nach Anspruch 7, worin Y = SO₂ ist.

11. Verbindung nach Anspruch 7, worin R³ ein C₁₋₆-Alkyl oder ein gegebenenfalls substituiertes Benzyl ist.

12. Verbindung nach Anspruch 7, worin R⁴ ein gegebenenfalls substituiertes Piperidinyl ist.

13. Verbindung nach Anspruch 1, worin die Verbindung aus der aus Folgendem: 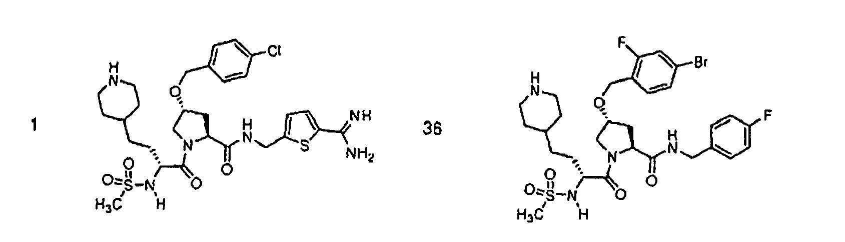 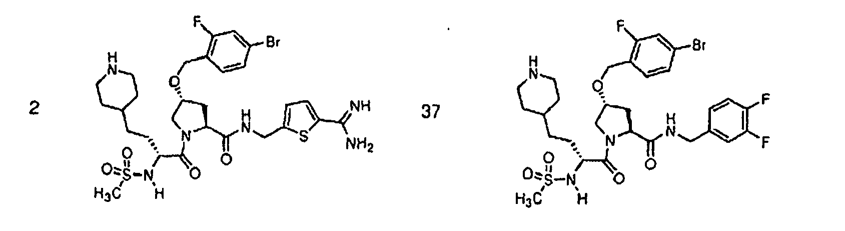 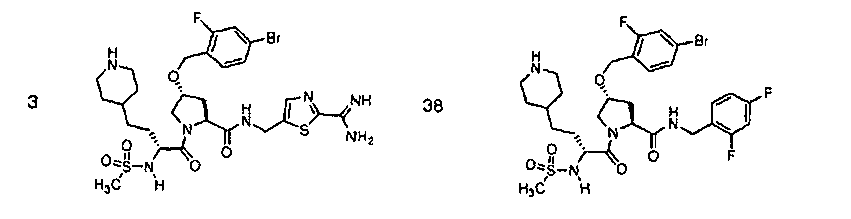 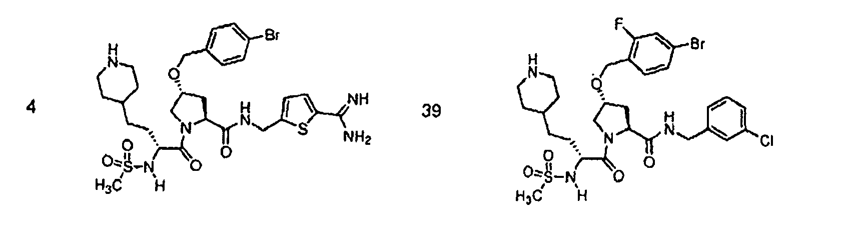 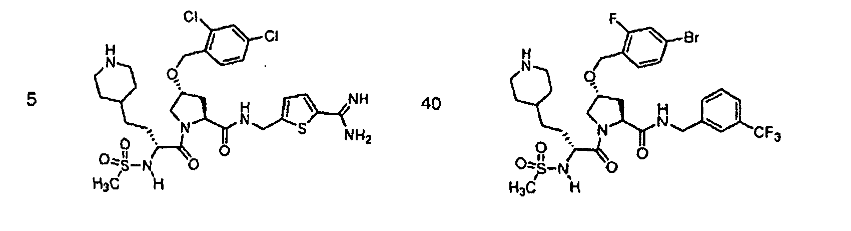 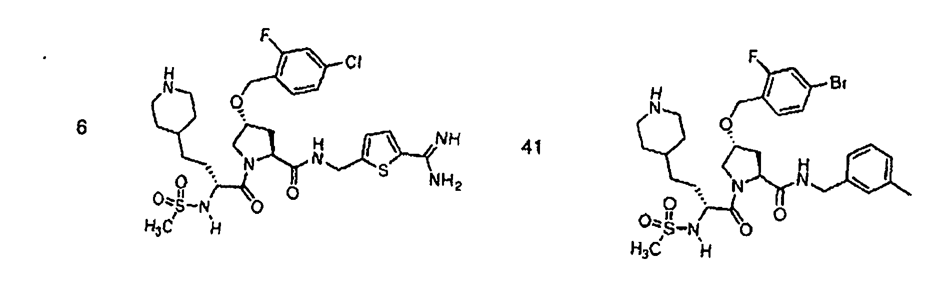 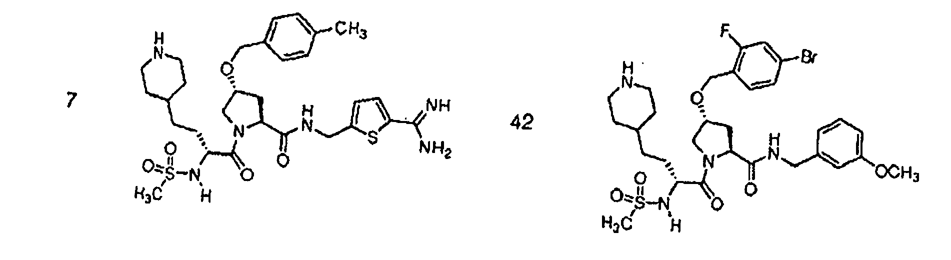 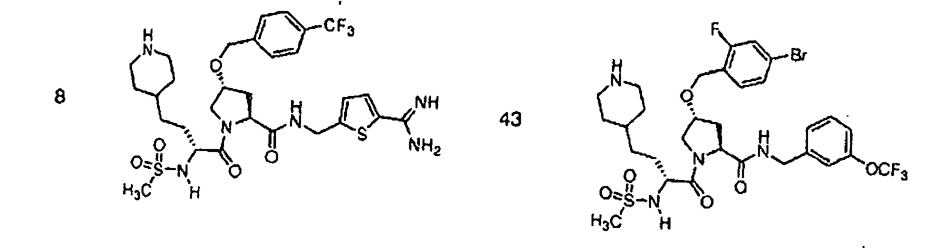 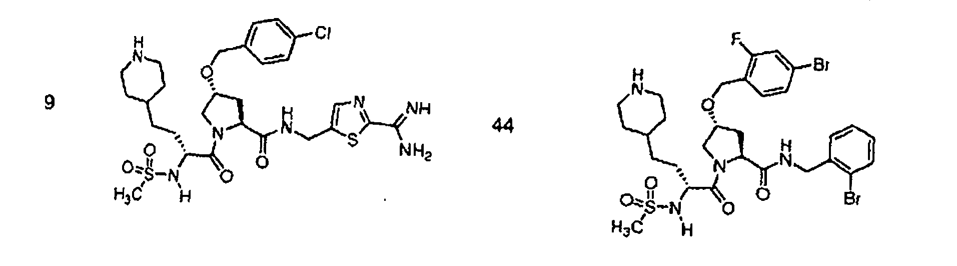 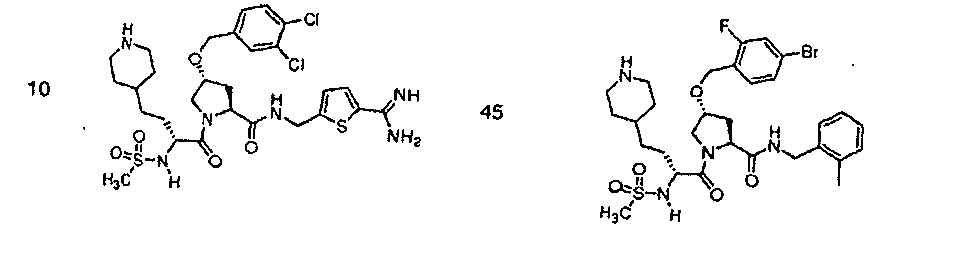 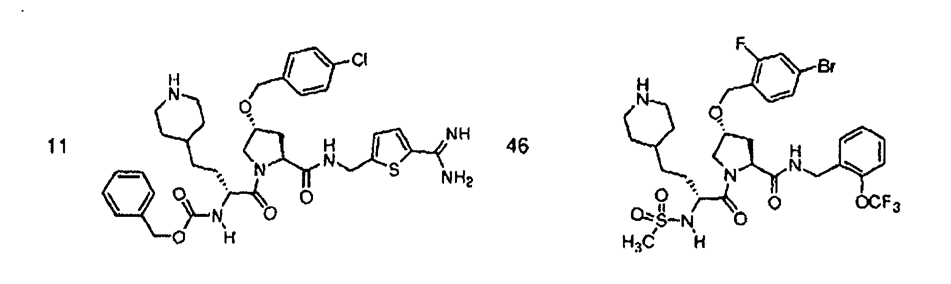 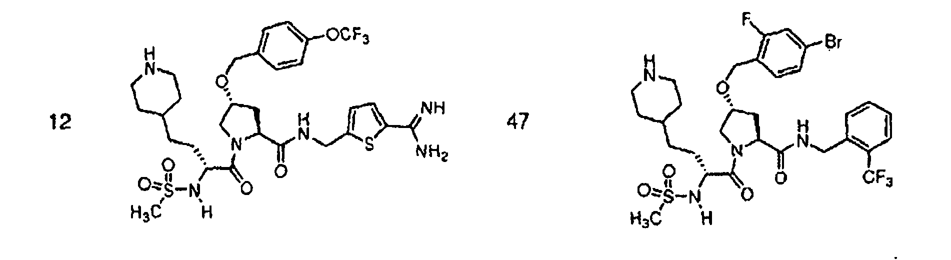 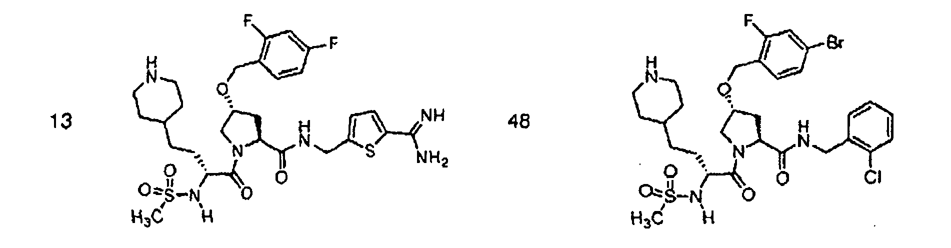 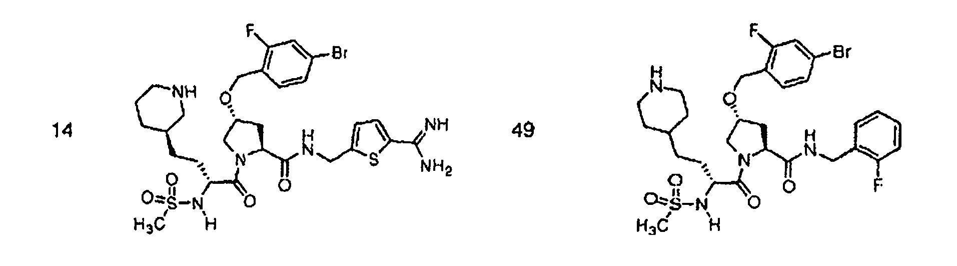 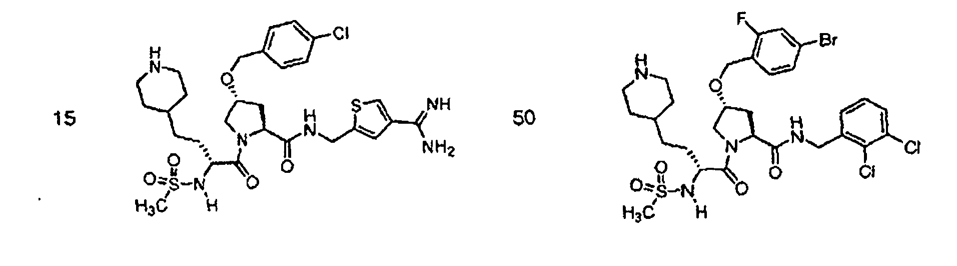 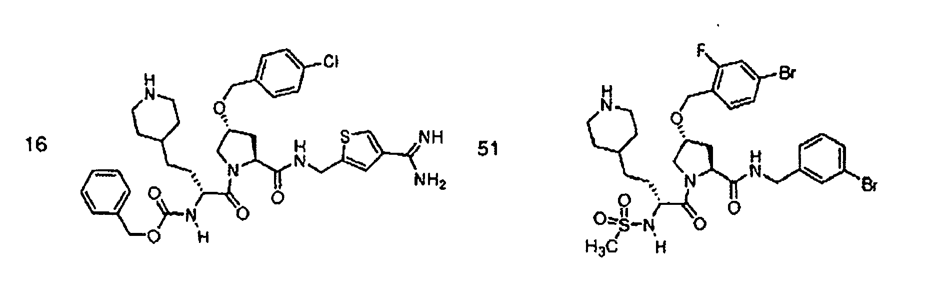 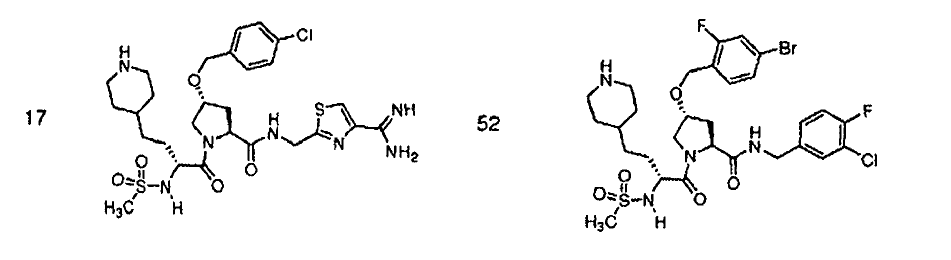 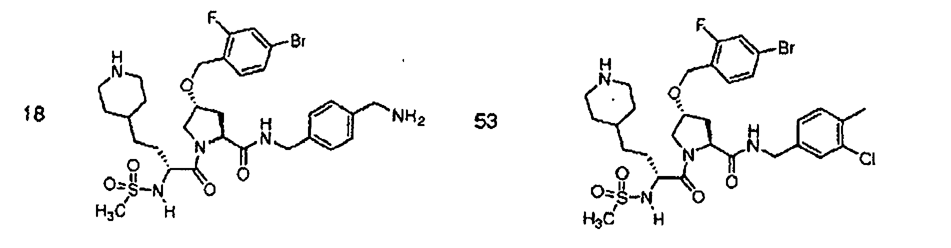 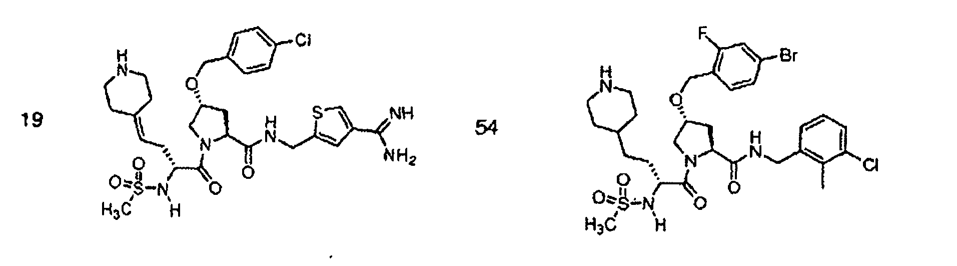 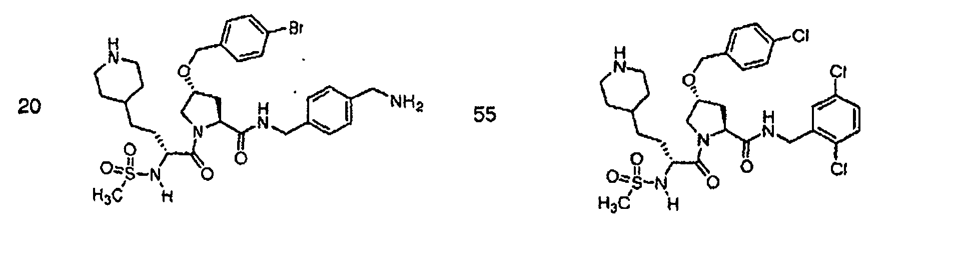 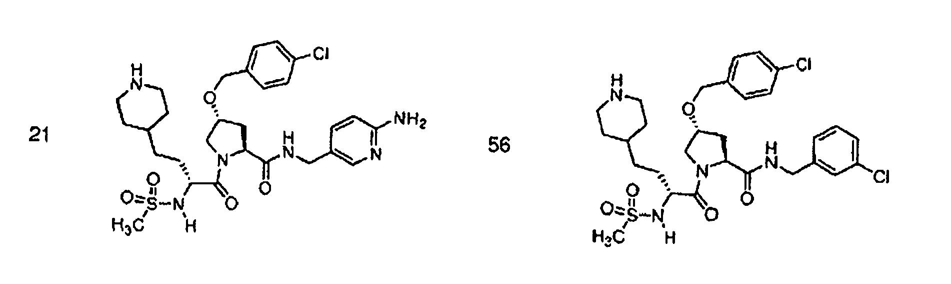 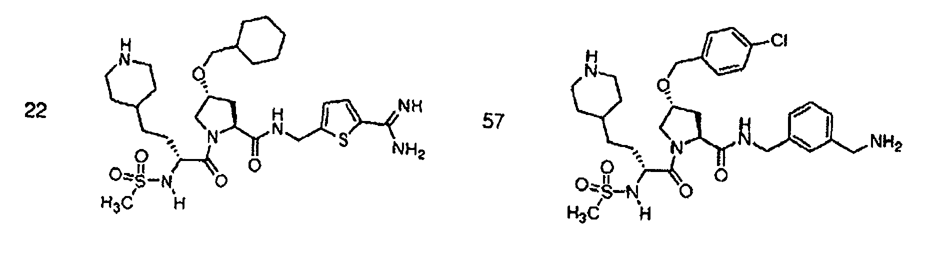 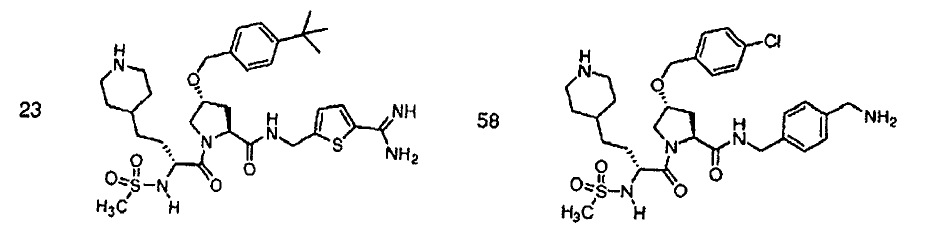 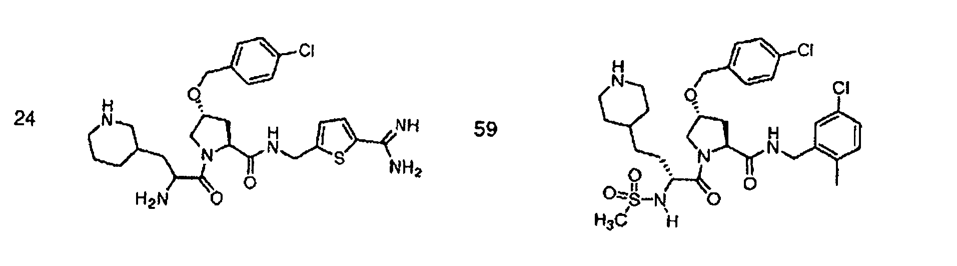 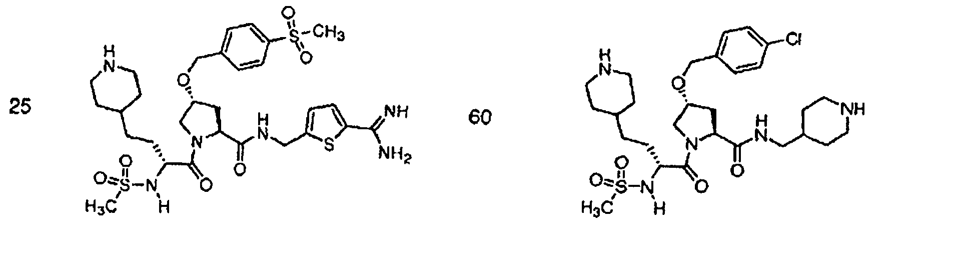 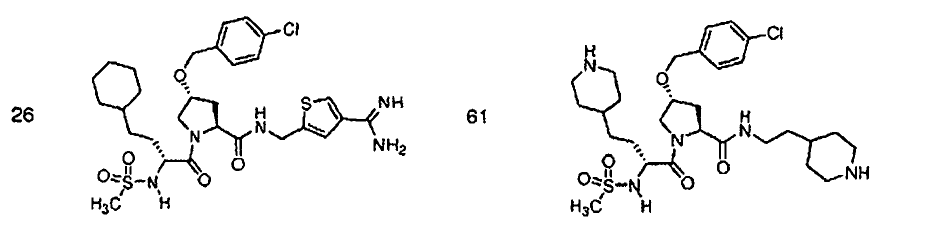 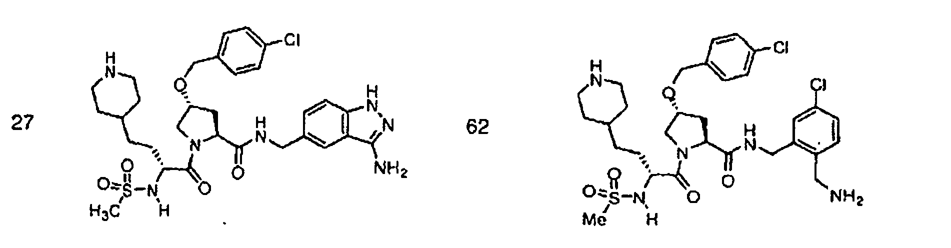 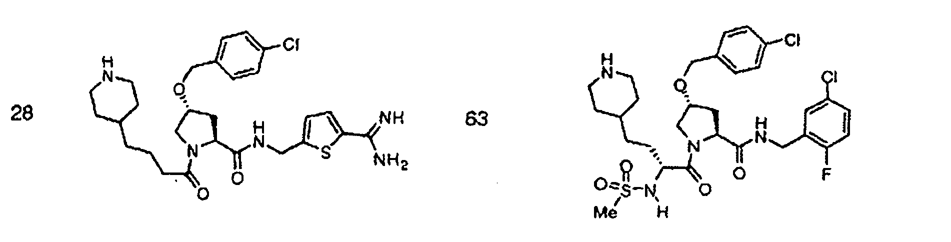 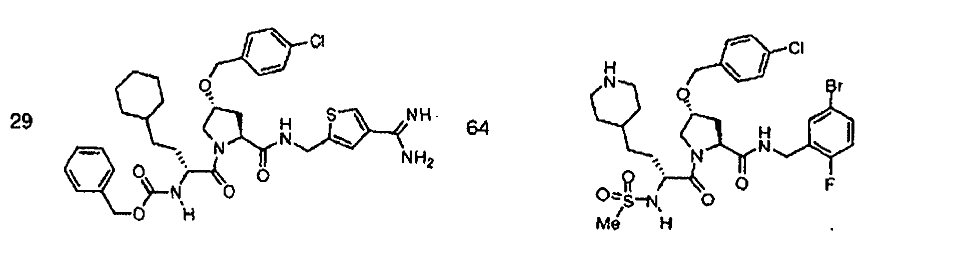 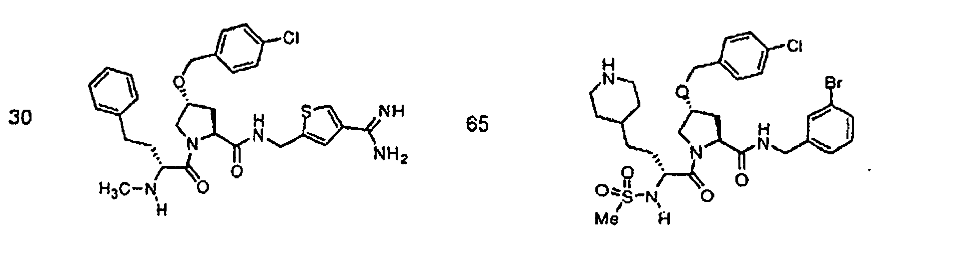 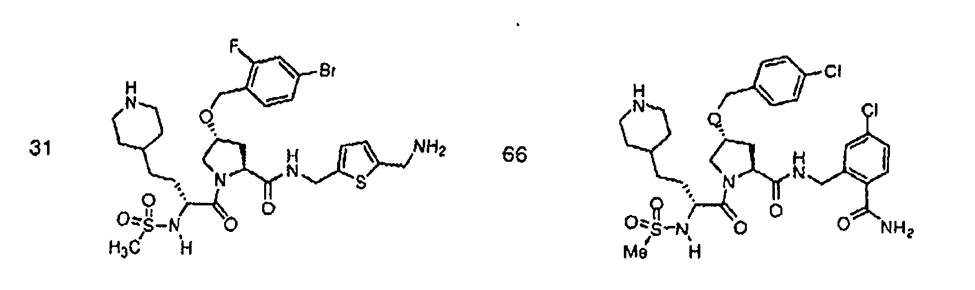 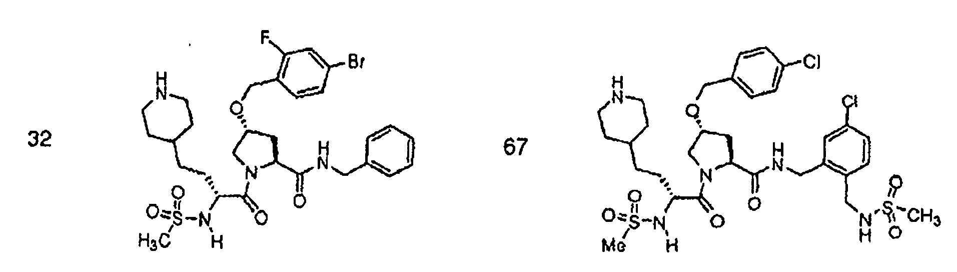 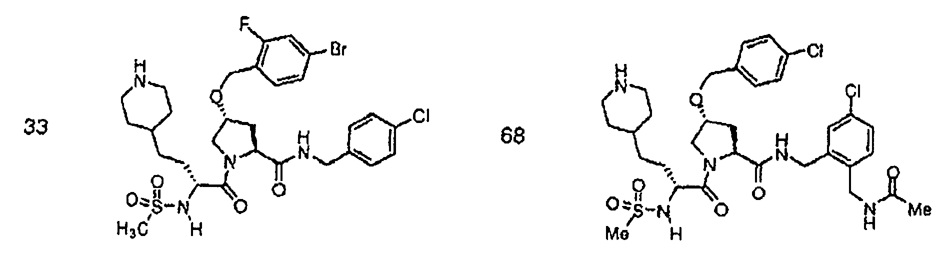 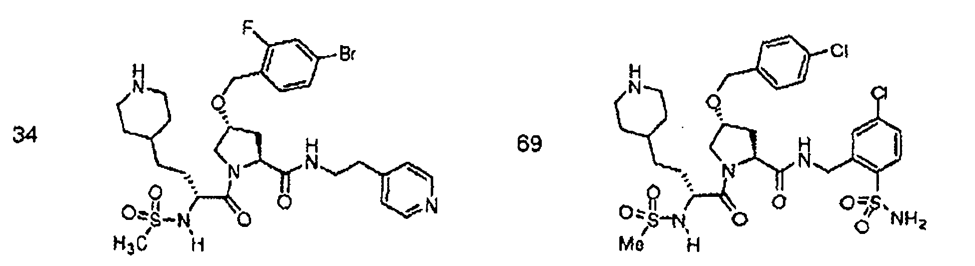 und 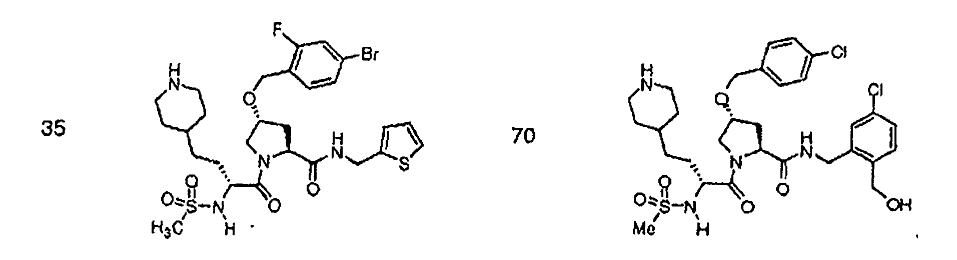 oder pharmazeutisch annehmbaren Salze davon bestehenden Gruppe ausgewählt ist.

14. Verbindung nach Anspruch 1, worin die Verbindung aus der aus Folgendem: 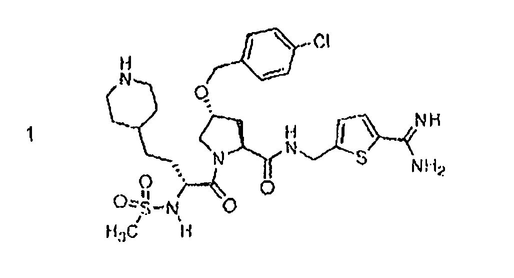 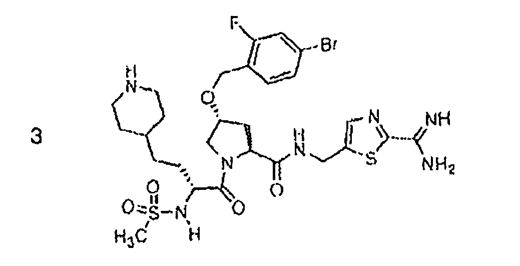 oder pharmazeutisch annehmbaren Salzen davon bestehenden Gruppe ausgewählt ist.

15. Verbindung nach Anspruch 1, worin die Verbindung aus der aus Folgendem: 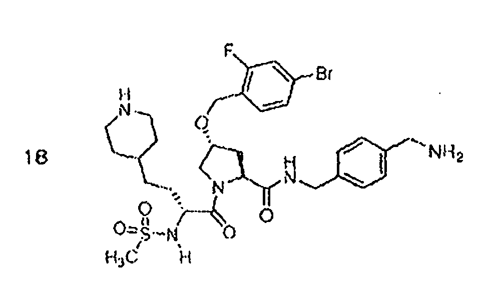 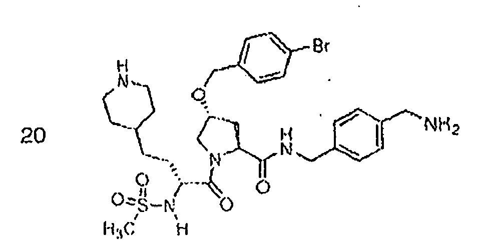 oder pharmazeutisch annehmbaren Salzen davon bestehenden Gruppe ausgewählt ist.

16. Verbindung nach Anspruch 1, worin die Verbindung aus der aus Folgendem: 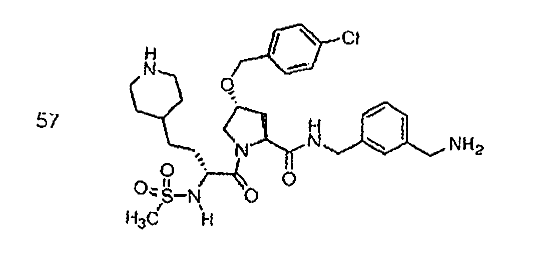 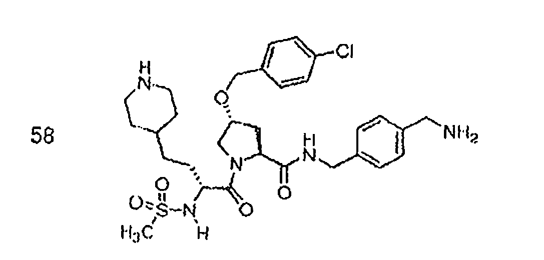 oder pharmazeutisch annehmbaren Salzen davon bestehenden Gruppe ausgewählt ist.

17. Verbindung nach Anspruch 1, worin die Verbindung aus der aus Folgendem: 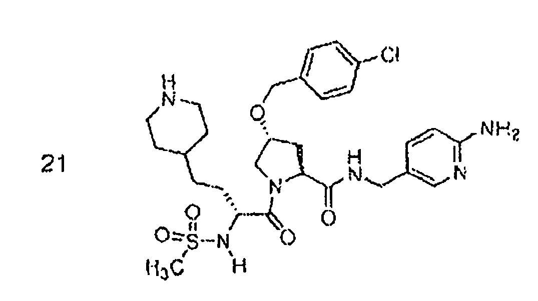 oder pharmazeutisch annehmbaren Salzen davon bestehenden Gruppe ausgewählt ist.

18. Verbindung nach Anspruch 1, worin die Verbindung aus der aus Folgendem: 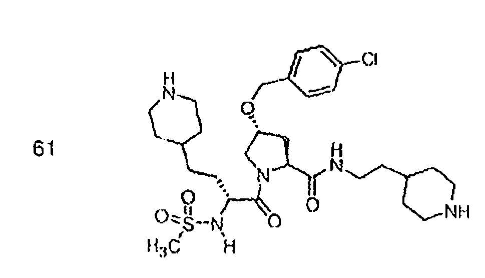 oder pharmazeutisch annehmbaren Salzen davon bestehenden Gruppe ausgewählt ist.

19. Verbindung nach Anspruch 1, worin die Verbindung aus der aus Folgendem: 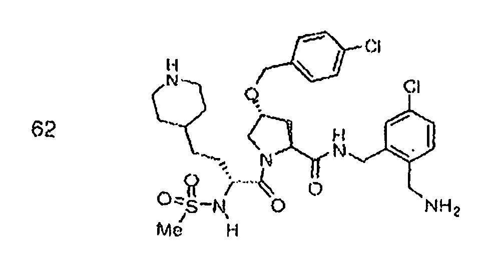 oder pharmazeutisch annehmbaren Salzen davon bestehenden Gruppe ausgewählt ist.

20. Pharmazeutische Zusammensetzung, die eine therapeutisch wirksame Menge einer Verbindung nach einem der Ansprüche 1 bis 19 umfasst.

21. Verbindung nach einem der Ansprüche 1 bis 19 zum Hemmen einer kanalaktivierenden Protease in einem Zell- oder Gewebesystem oder in einem Säugetier, worin die kanalaktivierende Protease Prostasin, PRSS22, TMPRSS11 (z.B. TMPRSS11B, TMPRSS11E), TMPRSS2, TMPRSS3, TMPRSS4 (MTSP-2), Matripase (MTSP-1), CAP2, CAP3, Trypsin, Cathepsin A oder neutrophile Elastase ist.

22. Verwendung einer Verbindung nach einem der Ansprüche 1 bis 19 zur Herstellung eines Medikaments zur Behandlung einer Erkrankung, die durch eine kanalaktivierende Protease vermittelt wird, in einem Zell- oder Gewebesystem oder in einem Säugetier, gegebenenfalls in Kombination mit einem zweiten Therapeutikum, worin die kanalaktivierende Protease Prostasin, PRSS22, TMPRSS11 (z.B. TMPRSS11B, TMPRSS11E), TMPRSS2, TMPRSS3, TMPRSS4 (MTSP-2), Matripase (MTSP-1), CAP2, CAP3, Trypsin, Cathepsin A oder neutrophile Elastase ist.

23. Verwendung nach Anspruch 22, worin die Erkrankung mit der Bewegung von Fluid über ionentransportierende Epithelien oder mit der Ansammlung von Mukus und Sputum in Geweben der Atemwege oder einer Kombination davon in Zusammenhang steht.

24. Verwendung nach Anspruch 22, worin es sich bei Erkrankung um Mukoviszidose, primäre Ziliardyskinesie, ein Lungenkarzinom, chronische Bronchitis, chronisch obstruktive Lungenkrankheit, Asthma oder eine Infektion der Atemwege handelt.

25. Verwendung nach Anspruch 22, worin das zweite Therapeutikum ein entzündungshemmendes Mittel, ein Bronchiodilator, ein Antihistamin, ein Hustenmittel, ein Antibiotikum oder DNase ist und vor, gleichzeitig mit oder nach der Verbindung nach einem der Ansprüche 1 bis 19 verabreicht wird.

26. Verbindung nach Anspruch 21 oder Verwendung nach Anspruch 22, worin die kanalaktivierende Protease Prostasin ist.

27. Verbindung nach Anspruch 21 oder Verwendung nach Anspruch 22, worin das Zell- oder Gewebesystem bronchiale Epithelzellen umfasst.

## Revendications

1. Composé de la Formule (1): 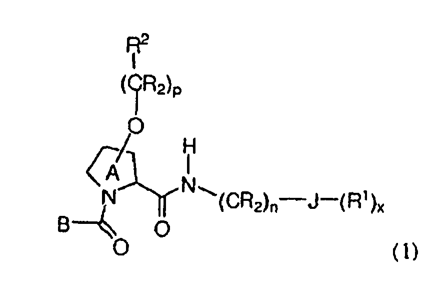 ou des sels pharmaceutiquement acceptables de celui-ci, où
O-(CR₂)ₚ-R² est un substituant à n'importe quelle position sur le cycle A;
J est un cycle carbocyclique monocyclique ou fusionné de 5 à 12 membres, le cycle hétérocyclique comprenant N, O et/ou S; le cycle aryle ou hétéroaryle, à condition que J ne soit pas triazolyle;
B est 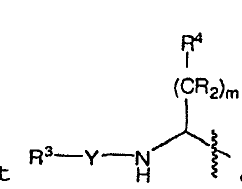 or (CR₂)ₖ-R⁵ ou (CR₂) ₖ-R⁵;
Y est une liaison, -SO₂- -NHCO- ou -O-(CO)-;
R¹ est halo, - (CR₂) ₁-NR⁶R⁷, - -(CR₂)₁-NRC(=NR)-NR⁶R⁷ - (CR₂)₁-C(=NR)- -NR⁶R⁷, -C(O)- (CR₂)₁-NR⁶R⁷, - (CR₂)₁-NR-SO₂R⁶, - (CR₂) ₁-NR-C (O) -R ⁶, - (CR₂) ₁-SO₂NR⁶R⁷ ou -(CR₂) ₁-OR⁶, ou un alcoxy C₁₋₆, alkyleC₁₋₆, alkényleC₂₋₆ ou alkynyleC₂₋₆ optionnellement substitué; ou un cycle carbocyclique, cycle hétérocyclique, aryle ou hétéroaryle optionnellement substitué;
R³ est alkyleC₁₋₆, alkényleC₂₋₆, alkynyleC₂₋₆ ou -(CR₂)₁-R⁵;
alternativement, NH-Y-R³ ensemble forment NH₂;
R², R⁴ et R⁵ sont indépendamment un cycle carbocyclique de 5 à 12 membres, cycle hétérocyclique, aryle ou hétéroaryle optionnellement substitué; ou bien R⁴ est H,
alkyleC₁₋₆, alkényleC₂₋₆, alkynyleC₂₋₆ ou 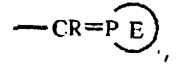 où P est C ou N et le cycle E ensemble avec P forment un cycle monocyclique ou fusionné de 5 à 12 membres optionnellement substitué;
R⁶ et R⁷ sont indépendamment H, alkyleC₁₋₆, alkényle C₂₋₆, alkynyleC₂₋₆ ou -(CR₂)₁-R⁵;
chaque R est H ou alkyleC₁₋₆, alkényleC₂₋₆ ou alkynyle C₂₋₆ ;
1 est 0-6;
k, m, n et p sont indépendamment 1-6;
x est 0-4;
à condition que R⁴ soit pipéridinyle lorsque NH-Y-R³ forment ensemble NH₂; et
à condition en outre que R⁵ soit pipéridinyle lorsque B est (CR₂) ₖ-R⁵.

2. Composé selon la revendication 1, où J est thiophényle, thiazolyle, phényle, pyridyle, indazolyle; pipéridinyle ou pyrrolidinyle.

3. Composé selon la revendication 1, où R¹ est halo, alkyleC₁₋₆, CF₃, OCF₃, phényle, -(CR₂)₁-NR⁶R⁷, (CR₂)₁-C(=NR)-NR⁶R⁷, -C(O)-(CR₂)₁-NR⁶R⁷, -(CR₂)₁-NR-SO₂R⁶, -(CR₂)₁-NR-C(O)-R⁶, - (CR₂) ₁-SO₂NR⁶R⁷ ou - (CR₂) ₁-OR⁶; où chaque 1 est 0-1; et R, R⁶ et R⁷ sont indépendamment H ou alkyle C₁₋₆.

4. Composé selon la revendication 1, où R² est phényle ou cyclohexyle, chacun étant optionnellement substitué par halo, SO2(alkyleC₁₋₆), ou un alkyleC₁₋₆ ou alcoxyC₁₋₆ optionnellement halogéné.

5. Composé selon la revendication 1, dans lequel R⁴ est pipéridinyl, cyclohexyle, phényle optionnellement
substitué,

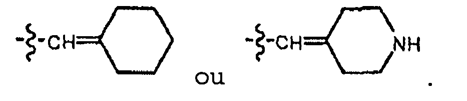

6. Composé selon la revendication 1, où Y est une liaison, SO₂ ou -O-(CO)-.

7. Composé selon la revendication 1, où ledit composé est de la Formule (2): 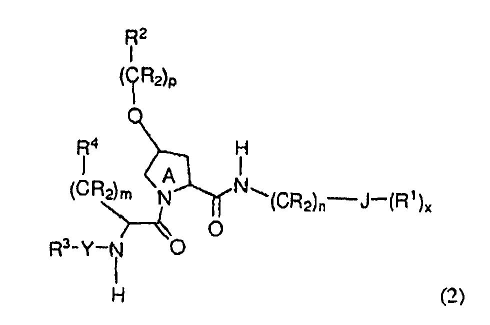 où R² et J sont indépendamment un aryle à 6 membres optionnellement substitué;
R³ est alkyleC₁₋₆, alkényleC₂₋₆, alkynyle C₂₋₆ ou (CR₂)₁ R⁵; ou bien NH-Y-R³ ensemble forment NH₂;
chaque R dans (CR₂) est H ou alkyleC₁₋₆; et
m, n et p sont indépendamment 1-2,
ou des sels pharmaceutiquement acceptables de celui ci.

8. Composé selon la revendication 7, où R² et J sont indépendamment un phényle optionnellement substitué.

9. Composé selon la revendication 7, où x est 1-3.

10. Composé selon la revendication 7, où Y est SO₂.

11. Composé selon la revendication 7, où R³ est alkyleC₁₋₆ ou un benzyle optionnellement substitué.

12. Composé selon la revendication 7, où R⁴ est un pipéridinyle optionnellement substitué.

13. Composé selon la revendication 1, où ledit composé est sélectionné dans le groupe consistant en: 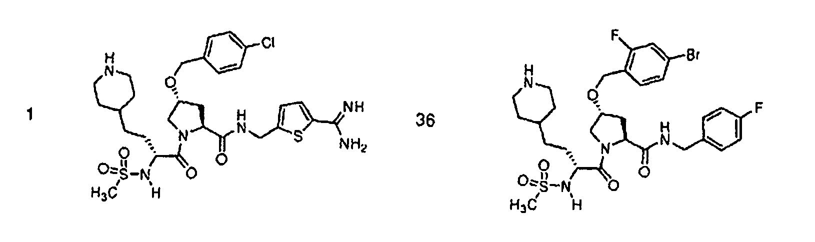 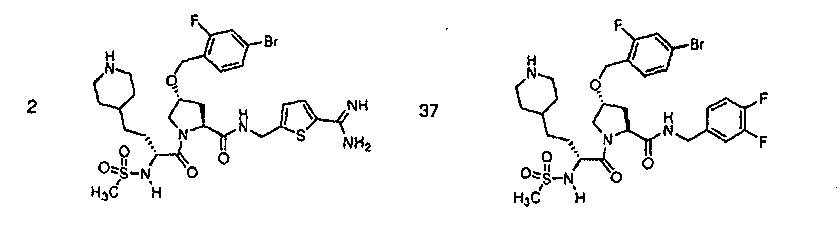 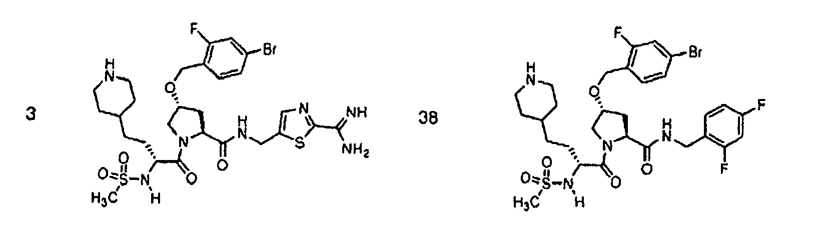 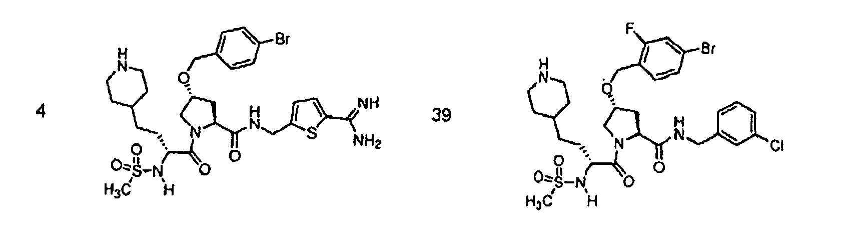 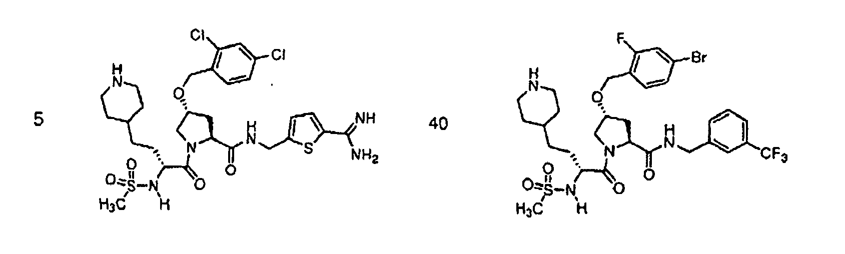 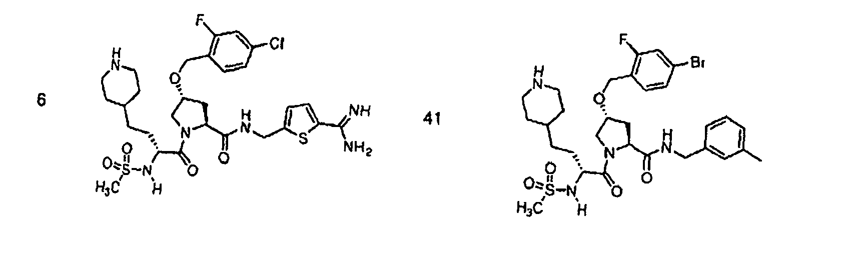 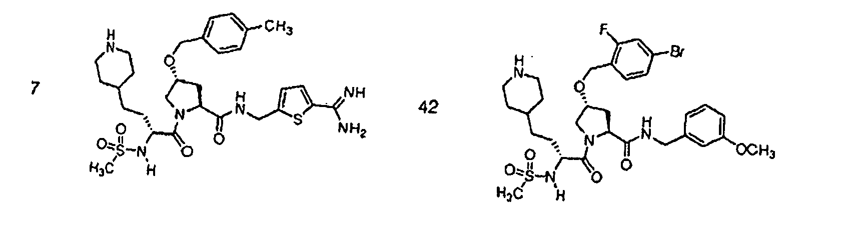 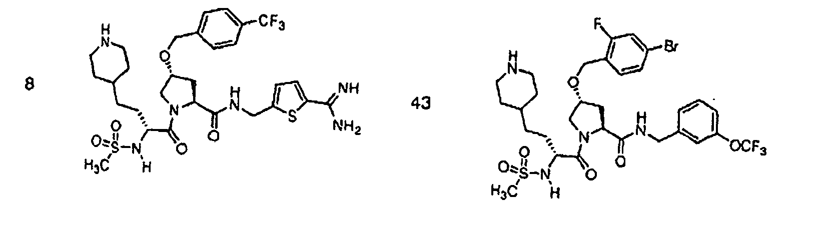 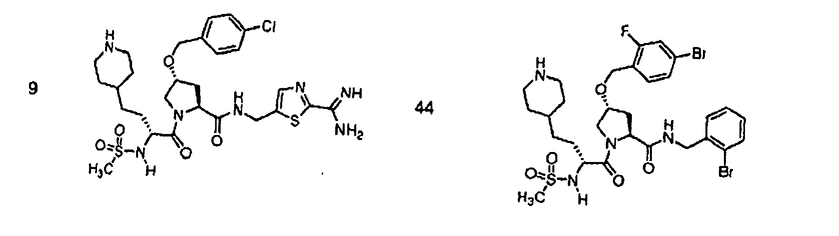 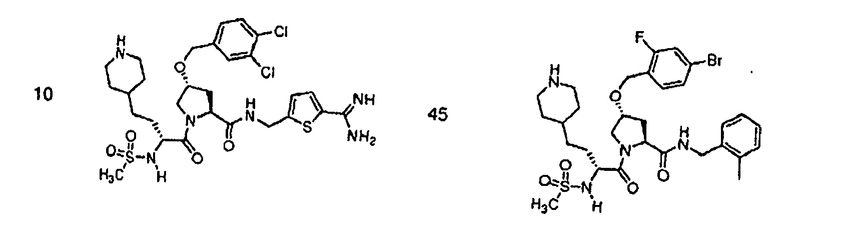 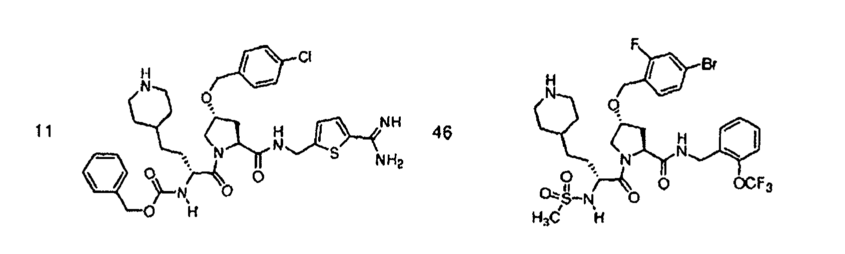 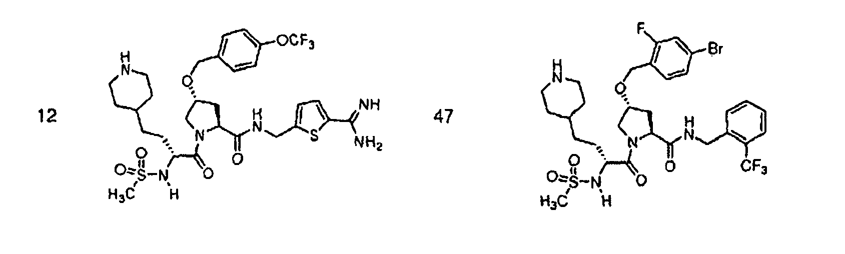 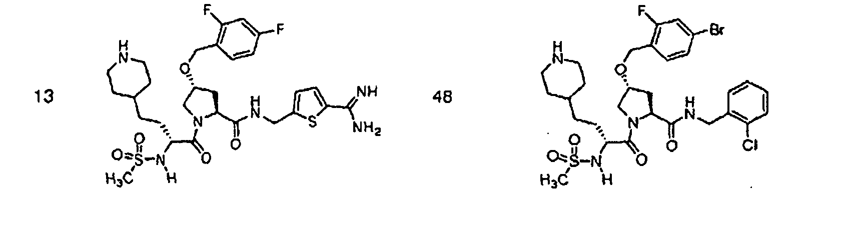 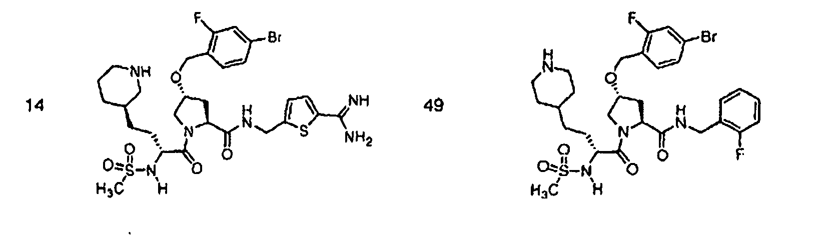 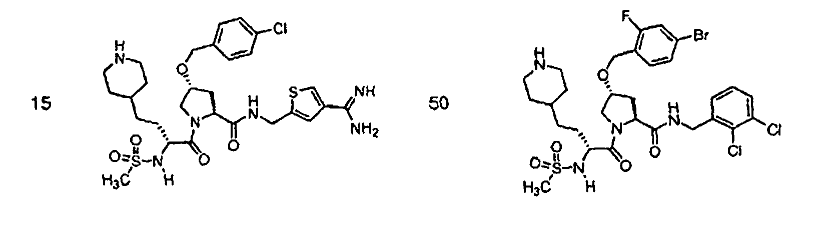 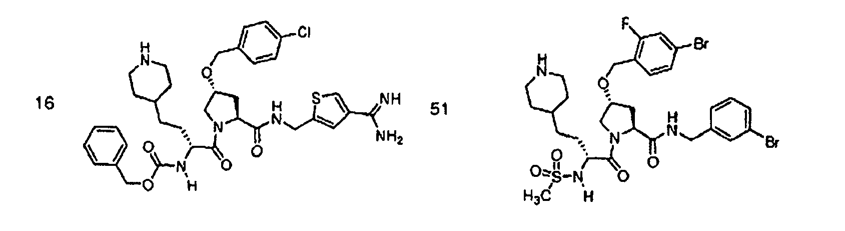 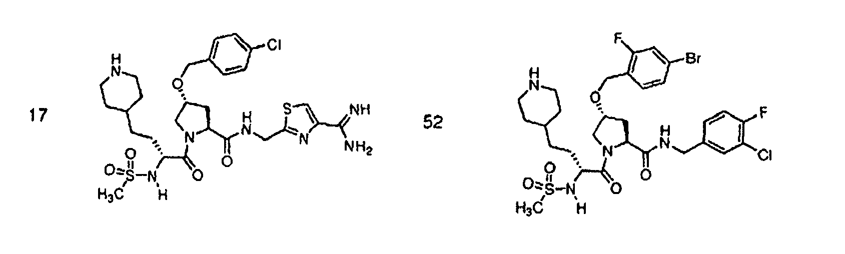 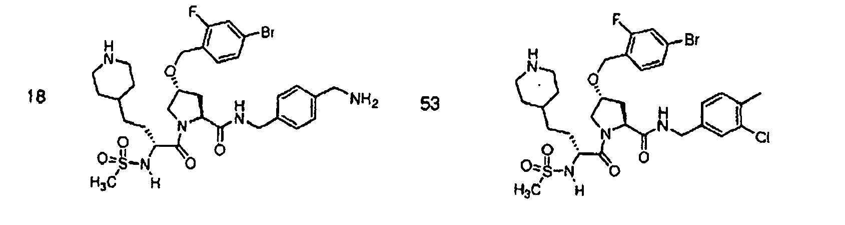 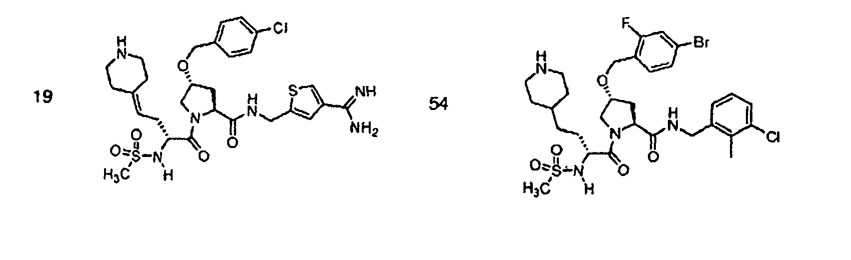 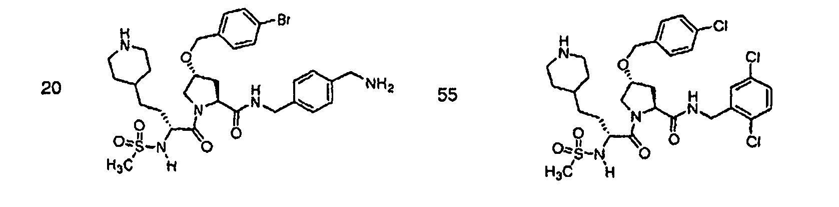 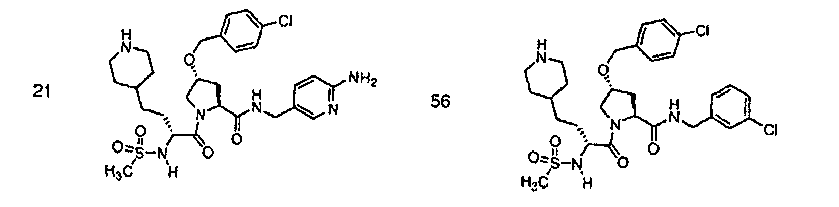 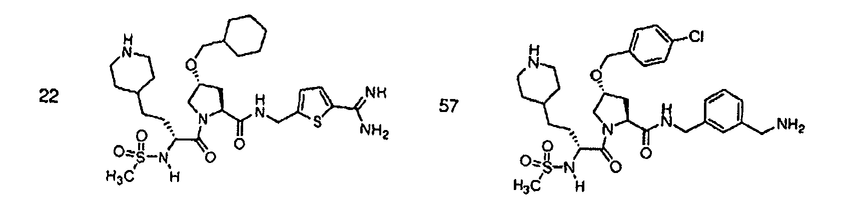 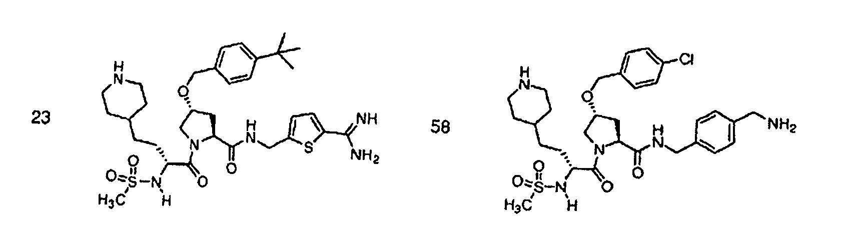 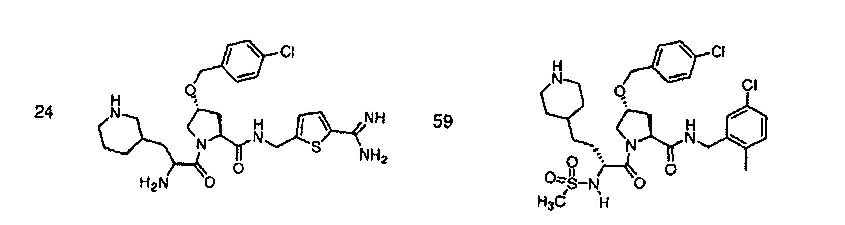 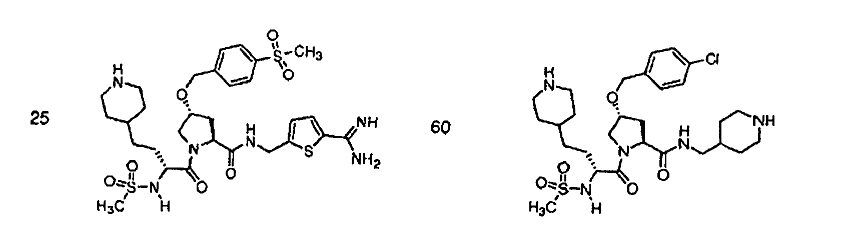 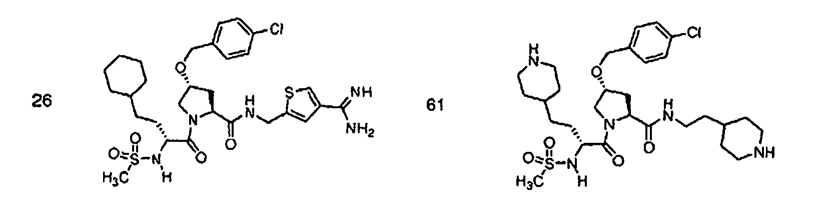 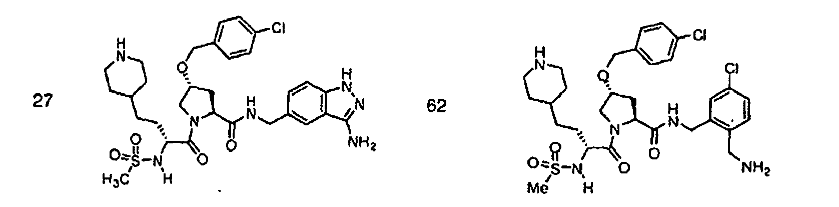 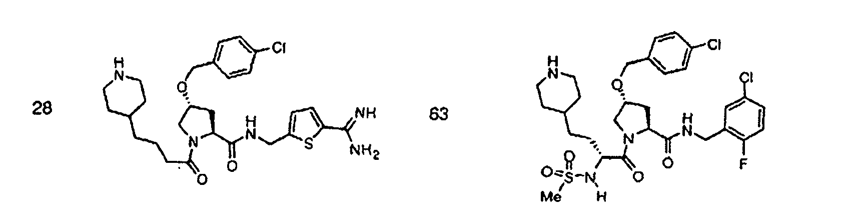 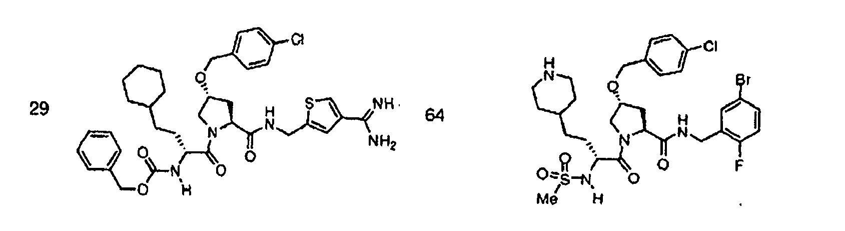 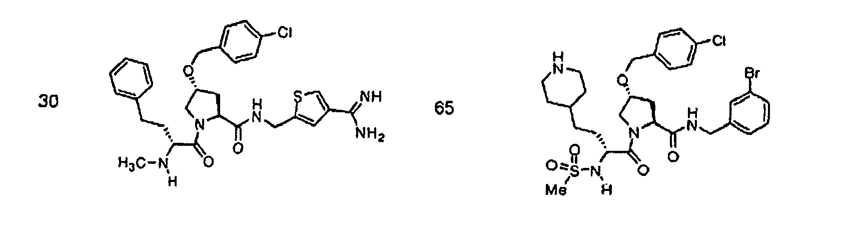 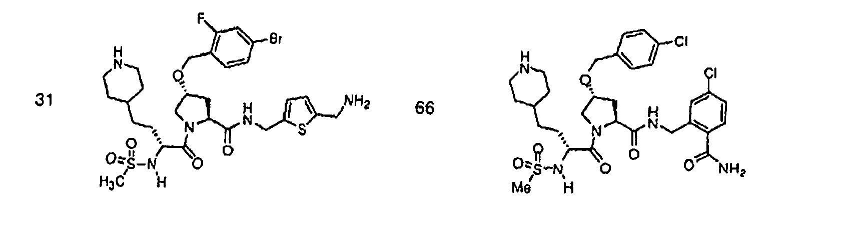 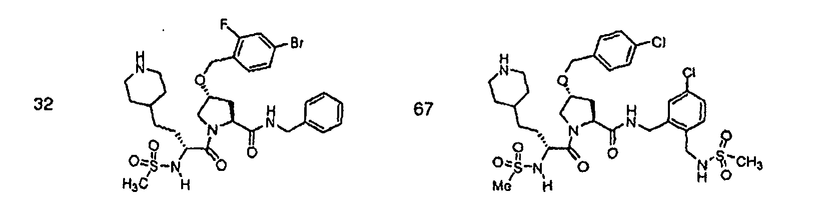 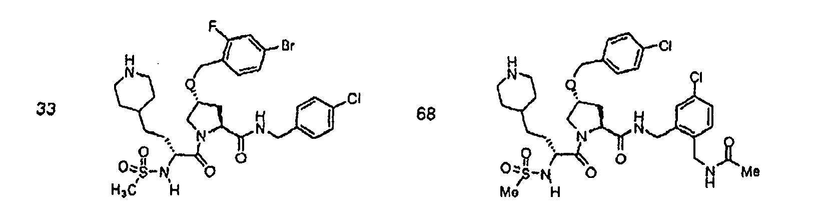 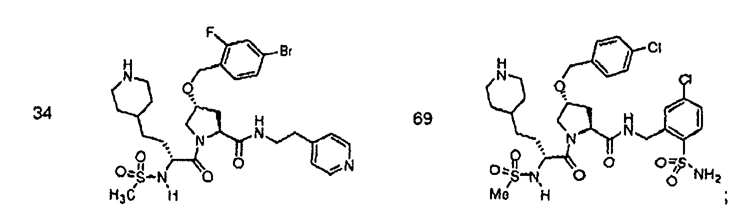 et 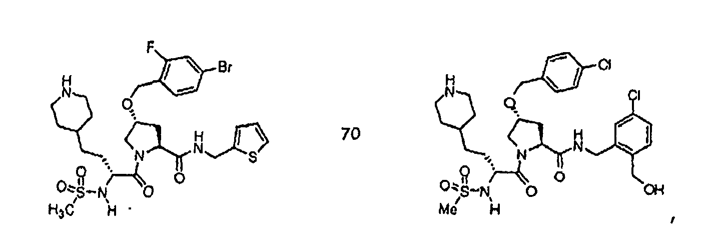 ou des sels pharmaceutiquement acceptables de celui-ci.

14. Composé selon la revendication 1, où ledit composé est sélectionné dans le groupe consistant en 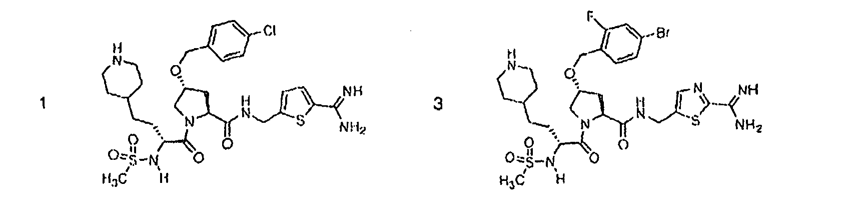 ou des sels pharmaceutiquement acceptables de celui-ci.

15. Composé selon la revendication 1, où ledit composé
est sélectionné dans le groupe consistant en 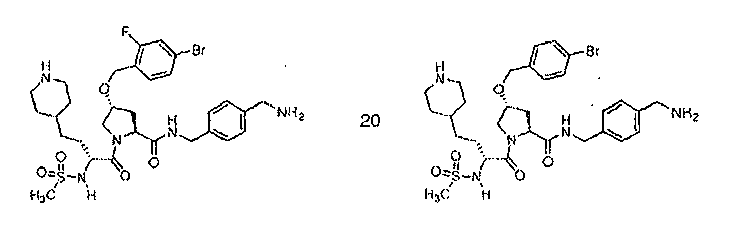 ou des sels pharmaceutiquement acceptables de celui-ci.

16. Composé selon la revendication 1, où ledit composé
est sélectionné dans le groupe consistant en 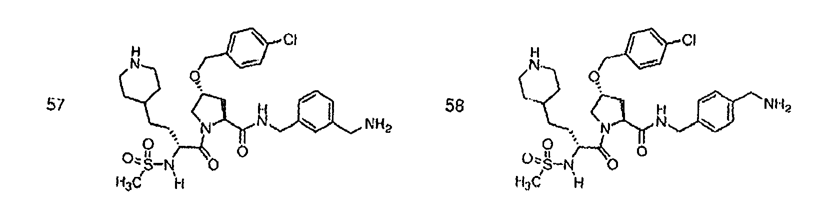 ou des sels pharmaceutiquement acceptables de celui-ci.

17. Composé selon la revendication 1, où ledit composé est 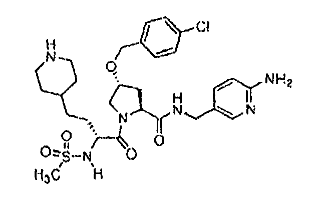 ou des sels pharmaceutiquement acceptables de celui-ci.

18. Composé selon la revendication 1, où ledit composé est 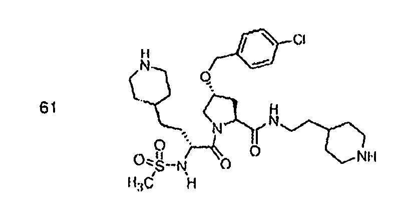 ou des sels pharmaceutiquement acceptables de celui-ci.

19. Composé selon la revendication 1, où ledit composé est 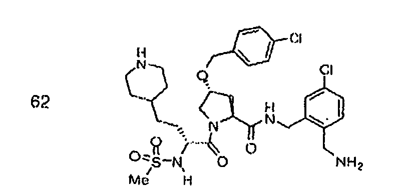 ou des sels pharmaceutiquement acceptables de celui-ci.

20. Composition pharmaceutique comprenant une quantité thérapeutiquement efficace d'un composé selon l'une quelconque des revendications 1 à 19.

21. Composé selon l'une quelconque des revendications 1 à 19 pour inhiber une protéase d'activation de canal dans un système de cellules ou de tissu ou dans un mammifère, où ladite protéase activant le canal est prostasine, PRSS22, TMPRSSII (par exemple, TMPRSSIIB, TMPRSSIIE), TMPRSS2, TMPRSS3, TMPRSS4 (MTSP-2), matriptase (MTSP-1), CAP2, CAP3, trypsine, cathepsine A ou élastase neutrophile.

22. Utilisation d'un composé selon l'une quelconque des revendications 1 à 19 pour la fabrication d'un médicament pour traiter une pathologie provoquée par une protéase activant le canal dans un système de cellules ou de tissu ou dans un mammifère, et optionnellement en combinaison avec un deuxième agent thérapeutique; où ladite protéase activant le canal est prostatine, PRSS22, TMPRSSII (*par exemple,* TMPRSSIIB, TMPRSSIIE), TMPRSS2, TMPRSS3, TMPRSS4 (MTSP-2), matriptase (MTSP-1), CAP2, CAP3, trypsine, cathepsine A ou élastase neutrophile.

23. Utilisation selon la revendication 22, où ladite pathologie est associée au mouvement du fluide sur l'épithélium transportant des ions ou l'accumulation de mucus et de crachat dans des tissus respiratoires ou une combinaison de ceux-ci.

24. Utilisation selon la revendication 22, où ladite pathologie est la fibrose cystique, la dyskinésie ciliaire primaire, le carcinome des poumons, la bronchite chronique, la bronchopneumopathie chronique obstructive, l'asthme ou une infection des voies respiratoires.

25. Utilisation selon la revendication 22, où ledit deuxième agent thérapeutique est anti-inflammatoire, bronchodilatateur, anti-histaminique, anti-tussif, antibiotique ou DNase, et est administré avant, simultanément ou après le composé selon l'une quelconque des revendications 1 à 19.

26. Composé selon la revendication 21 ou utilisation selon la revendication 22, où ladite protéase activant le canal est la prostasine.

27. Composé selon la revendication 21 ou l'utilisation selon la revendication 22, où ledit système de cellules ou de tissu comprend des cellules épithéliales bronchiques.
